# EUROPEAN PATENT SPECIFICATION

(11) **EP 3 631 445 B1**
(45) Date of publication and mention of the grant of the patent: **25.09.2024**
(21) Application number: 18810303.0
(22) Date of filing: 01.06.2018
(51) Int. Cl.: G01N 33/50, G01N 33/574, G01N 33/52, C12N 5/09, C12N 5/095, C12N 5/22

(54) **METHODS OF DETERMINING THERAPIES BASED ON SINGLE CELL CHARACTERIZATION OF CIRCULATING TUMOR CELLS (CTCS) IN METASTATIC DISEASE**
VERFAHREN ZUR BESTIMMUNG VON THERAPIEN AUF DER BASIS VON EINZELZELLCHARAKTERISIERUNG VON ZIRKULIERENDEN TUMORZELLEN (CTCS) BEI METASTASIERENDEN ERKRANKUNGEN
MÉTHODES DE DÉTERMINATION DE THÉRAPIES SUR LA BASE D'UNE CARACTÉRISATION CELLULAIRE UNIQUE DE CELLULES TUMORALES CIRCULANTES (CTC) DANS UNE MALADIE MÉTASTATIQUE

(30) Priority: 02.06.2017 US 201762514642 P; 12.07.2017 US 201762531725 P
(43) Date of publication of application: 08.04.2020
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2018/035581
(87) International publication number: WO 2018/222979

(56) References cited:
- WO-A1-2015/112955
- WO-A1-2016/196284
- US-A1- 2015 212 089
- US-A1- 2016 266 127
- H. BELTRAN ET AL: "The Initial Detection and Partial Characterization of Circulating Tumor Cells in Neuroendocrine Prostate Cancer", CLINICAL CANCER RESEARCH, vol. 22, no. 6, 15 March 2016 (2016-03-15), US, pages 1510 - 1519, XP055382275, ISSN: 1078-0432, DOI: 10.1158/1078-0432.CCR-15-0137
- SCHER ET AL.: "Baseline CTC subtype to predict outcomes on mCRPC patients (pts) receiving enzalutamide (E) compared to abiraterone (A", J CLIN ONCOL, vol. 35, no. 15, 20 May 2017 (2017-05-20), pages 5070, XP009517907, Retrieved from the Internet <URL:http://ascopubs.org/doi/abs/10.1200/JCO.2017.35.15_suppl.5070> DOI: 10.1200/JCO.2017.35.15_suppl.5070

## Description

The invention relates generally to the field of cancer diagnostics and, more specifically to methods for single cell phenotypical and morphological analysis of circulating tumor cells (CTCs) to predict drug resonsiveness of hormon-refractory prostate cancer patients.

### BACKGROUND

After successive cancer therapies, multiple subpopulations of cancer cells arise, each with divergent genetic aberrations that may confer drug resistance or susceptibility. Tissue biopsies may not detect these subpopulations, but a liquid biopsy of blood can help identify these important tumor cells and characterize how a patient's tumors have evolved over time. Single cell genomic profiling is a powerful new tool for investigating evolution and diversity in cancer and understanding the role of rare cells in tumor progression. Clonal diversity is destined to play an important role in invasion, metastasis, and the evolution of resistance to therapy.

Prostate cancer is the most commonly diagnosed solid organ malignancy in the United States (US) and remains the second leading cause of cancer deaths among American men. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416700 new cases in 2012, representing 22.8% of cancer diagnoses in men. In total, 92200 PC-specific deaths are expected, making it one of the three cancers men are most likely to die from, with a mortality rate of 9.5%

Despite the proven success of hormonal therapy for prostate cancer using chemical or surgical castration, most patients eventually will progress to a phase of the disease that is metastatic and shows resistance to further hormonal manipulation. This has been termed metastatic castration-resistant prostate cancer (mCRPC). Despite this designation, however, there is evidence that androgen receptor (AR)-mediated signaling and gene expression can persist in mCRPC, even in the face of castrate levels of androgen. This may be due in part to the upregulation of enzymes involved in androgen synthesis, the overexpression of AR, or the emergence of mutant ARs with promiscuous recognition of various steroidal ligands. Androgen receptor (AR)-gene amplification, found in 20-30% of mCRPC is proposed to develop as a consequence of hormone-deprivation therapy and be a prime cause of treatment failure. Treatment of patients with mCRPC remains a significant clinical challenge. Studies have further elucidated a direct connection between the PI3K-AKT-mTOR and androgen receptor (AR) signaling axes, revealing a dynamic interplay between these pathways during the development of hormone resistance. PTEN is one of the most commonly deleted/mutated tumor suppressor genes in human prostate cancer. As a lipid phosphatase and negative regulator of the PI3K/AKT/mTOR pathway, PTEN controls a number of cellular processes, including survival, growth, proliferation, metabolism, migration, and cellular architecture. PTEN loss can be used as a diagnostic and prognostic biomarker for prostate cancer, as well as predict patient responses to emerging PI3K/AKT/mTOR inhibitors.

Prior to 2004, there was no treatment proven to improve survival for men with mCRPC. The treatment of patients with mitoxantrone with prednisone or hydrocortisone was aimed only at alleviating pain and improving quality of life, but there was no benefit in terms of overall survival (OS). In 2004, the results of two major phase 3 clinical trials, TAX 327 and SWOG (Southwest Oncology Group) 9916, established Taxotere^{®} (docetaxel) as a primary chemotherapeutic option for patients with mCRPC. Additional hormonal treatment with androgen receptor (AR) targeted therapies, chemotherapy, combination therapies, and immunotherapy, has been investigated for mCRPC, and recent results have offered additional options in this difficult-to-treat patient group. With the advent of exponential growth of novel agents tested and approved for the treatment of patients with metastatic castration-resistant prostate cancer (mCRPC) in the last 5 years alone, issues regarding the optimal sequencing or combination of these agents have arisen. Several guidelines exist that help direct clinicians as to the best sequencing approach and most would evaluate presence or lack of symptoms, performance status, as well as burden of disease to help determine the best sequencing for these agents. Mohler et al., 2014, J Natl Compr Canc Netw. 2013;1 1(12): 1471-1479; Cookson et al., 2013, J Urol. 2013;190(2):429-438. Currently, approved treatments consist of taxane-class cytotoxic agents such as Taxotere^{®} (docetaxel) and Jevtana^{®} (cabazitaxel), and anti-androgen hormonal therapy drugs such as Zytiga^{®} (arbiterone, blocks androgen production) or Xtandi^{®} (enzalutamide, an androgen receptor (AR) inhibitor).

The challenge for clinicians is to decide the best sequence for administering these therapies to provide the greatest benefit to patients. Used sequentially, the response to enzalutamide after abiraterone acetate, or abiraterone acetate after enzalutamide is less frequent and of shorter duration. Whether taxane based chemotherapy would be more beneficial than a second anti-androgen hormonal therapy is a key question. However, therapy failure remains a significant challenge based on heterogeneous responses to therapies across patients and in light of cross-resistance from each agent. Mezynski et al., Ann Oncol. 2012;23(11):2943-2947;. Noonan et al., Ann Oncol. 2013;24(7):1802-1807; Pezaro et al., Eur Urol. 2014, 66( 3): 459-465. In addition, patients may lose the therapeutic window to gain substantial benefit from each drug that has been proven to provide overall survival gains. Hence, better methods of identifying the target populations who have the most potential to benefit from targeted therapies remain an important goal.

Poly ADP-ribose Polymerase (PARP) inhibitors (PARPi) have demonstrated efficacy in mCRPC, breast, ovarian and other cancer patients with germline BRCA mutations and more recently in patients with somatic inactivating homologous recombination (HR) DNA repair pathway mutations (Mateo et al., NEJM, 2015;373(18):1697-708; Robinson et al., Cell, 2015;161(5):1215-28; Balmana et al., Ann Oncol. 2014, 25:1656-63; Del Conte et al., Br J Cancer, 2014,111:651-9). Current methods to detect HR deficiency (HRD) require genomic analysis from fresh or archival tumor biopsy to detect inactivating mutations or genomic scars (LSTs, NtAI or LOH) indicative of HRD (Abkevich et al., Br J Cancer, 2012 Nov 6, 107(10):1776-82). HRD genomic biomarkers are prevalent in 10-20% of the patient population (Marquard et al., Biomark Res. 2015 May 1, 3:9).

Significant strides have also been made recently to elucidate the relationship between HRD genotypes and sensitivity to platinum agents. One retrospective analysis pooled samples from the PrECOG 0105, Cisplatin-1 and Cisplatin-2 trials revealed that the Myriad HRD score was highly associated with complete pathological response to neoadjuvant platinum agents in triple negative breast cancer (TNBC) (Telli et al. Clinical cancer research: An Official Journal of the American Association for Cancer Research. 2016). In the adjuvant (Vollebergh et al. Breast Cancer Res. 2014, 16(3):R47) and metastastic (Isakoff et al. J. Clinical Oncol., 2015, 33(17):1902-9) settings, HRD was revealed to highly associate with favorable outcome on platinum agents, compared to the rest of the cohort in TNBC and hormone receptor positive breast cancer.

Measuring HRD in from solid tumor biopsies may be problematic due to the inaccessibility/unavailability of biopsy material (i.e. bone metastasis) and poor correlation of archival primary tumor samples to fresh biopsy (Punnoose et al., Br J Cancer. 2015 Oct 20;113(8):1225-33). Low concordance between archival and fresh biopsy is largely attributed to high degrees of intra-tumor and inter-cellular heterogeneity from temporal clonal evolution in response to prior therapeutic interventions resulting in spatial heterogeneity and ultimately under sampling of a polyclonal disease.

Circulating tumor cells (CTCs) represent a significant advance in cancer diagnosis made even more attractive by their non-invasive measurement. Cristofanilli et al., N Engl J Med 2004, 351:781-91. US 2016/266127 A1 discloses a method of predicting response to a hormone-directed therapy in prostate cancer patients comprising characterization of genotypic, morphometric and protein expression parameters of CTCs in a blood sample from the patient by direct analysis, to generate a profile for each of the CTCs.CTCs released from either a primary tumor or its metastatic sites hold important information about the biology of the tumor. Historically, the extremely low levels of CTCs in the bloodstream combined with their unknown phenotype has significantly impeded their detection and limited their clinical utility. A variety of technologies have recently emerged for detection, isolation and characterization of CTCs in order to utilize their information. CTCs have the potential to provide a non-invasive means of assessing progressive cancers in real time during therapy, and further, to help direct therapy by monitoring phenotypic physiological and genetic changes that occur in response to therapy. In most advanced prostate cancer patients, the primary tumor has been removed, and CTCs are expected to consist of cells shed from metastases, providing a "liquid biopsy." While CTCs are traditionally defined as EpCAM/cytokeratin positive (CK+) cells, CD45-, and morphologically distinct, recent evidence suggests that other populations of CTC candidates exist including cells that are EpCAM/cytokeratin negative (CK-) or cells smaller in size than traditional CTCs. These findings regarding the heterogeneity of the CTC population, suggest that enrichment-free CTC platforms are favorable over positive selection techniques that isolate CTCs based on size, density, or EpCAM positivity that are prone to miss important CTC subpopulations.

CRPC presents serious challenges to both the patients suffering from this advanced form of prostate cancer and the clinicians managing these patients. Clinicians are often faced with providing comprehensive diagnoses and assessments of the mechanisms that cause disease progression in an effort to guide appropriate and individualized treatments. By identifying appropriate therapeutic and prognostic markers, the potential clinical benefit of targeted therapy is increased, and clinicians are enabled to better managed CRPC, improve the quality of life for patients, and enhance clinical outcomes. A need exists to understand the frequency of subclonal CNV driver alterations and genomic instability in individual CTCs in combination with cell phenotype to enable a more accurate view of heterogeneous disease, predict therapeutic response, and identify novel mechanisms of resistance. Predictive biomarkers of sensitivity to anti-androgen hormonal therapy and taxane based chemotherapy are needed that can be assessed in individual patients each time a decision to select therapy is needed. The present invention addresses this need and provides related advantages are provided.

### SUMMARY OF THE INVENTION

The invention provides a method of predicting responsiveness to treatment with abiraterone and enzalutamide in a hormone-refractory prostate cancer patient, comprising:
(a) performing a direct analysis comprising immunofluorescent staining and morphological characterization of nucleated cells in a blood sample obtained from the patient to identify and enumerate circulating tumor cells (CTC);
(b) individually characterizing digital pathology parameters to generate a profile for each of the CTCs, wherein the parameters comprise:
   (i) size of the nucleus,
   (ii) nuclear entropy, and
   (iii) number of nucleoli, or size of the cytoplasm;
(c) classifying individual cells into CTC subtypes; and
(d) determining the absence or presence of a biomarker CTC in the CTC subtypes, wherein the biomarker CTC has the characteristics of a large nucleus, high nuclear entropy, and frequent nucleoli or large cytoplasm, wherein the presence of the biomarker CTC indicates responsiveness of the patient to abiraterone, and wherein the absence of the biomarker CTC indicates responsiveness of the patient to enzalutamide.

In one embodiment, the cells are classified with a CTC subtype classifier developed from unsupervised classification.

In another embodiment, the method further comprises the step of isolating the CTCs from said sample.

The invention further provides abiraterone for use in a method of treatment of a hormone-refractory prostate cancer patient, wherein the method comprises the diagnostic method of the invention.

The invention further provides enzalutamide for use in a method of treatment of a hormone-refractory prostate cancer patient, wherein the method comprises the diagnostic method of the invention.

References in the description to methods of treatment are to be understood in the above sense as references to the hormone-based therapies for use in the treatment of a hormone-refractory prostate cancer.

Other features and advantages of the invention will be apparent from the detailed description.

### BRIEF DESCRIPTION OF THE DRAWINGS

Figure 1A shows a description of standard Epic CTC analysis process. Images are analyzed using a multi-parametric digital pathology algorithm to detect CTC candidates and quantitate protein biomarker expression levels. CTC classifications are displayed in a web-based report and are confirmed by trained technicians. Figure 1B shows a description of the CTC recovery and genomic profiling workflow. Individual cells are isolated, subjected to Whole Genome Amplification, and NGS library preparation. Sequencing is performed on an Illumina NextSeq 500.

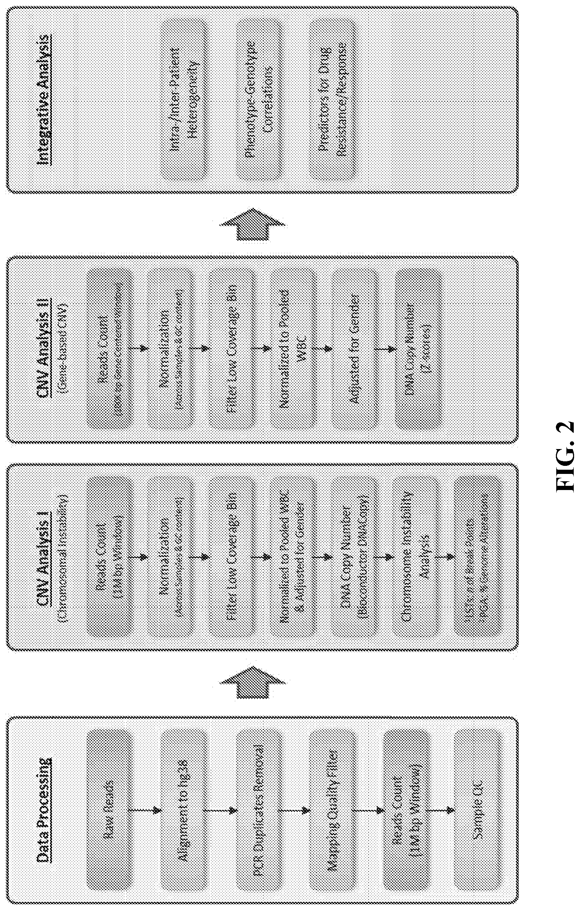

Figure 2 provides a diagram of the bioinformatic analysis performed. Raw FASTQ files are assessed and filtered for quality. Reads are aligned to the hg 38 reference genome (UCSC), PCR duplicates removed, and filtered by the MAPQ score 30. Samples with >250K reads post filtering are analyzed for copy number alterations. The filtered alignment files are further analyzed with Epic's Copy Number Pipelines. One pipeline was for estimating genomic instability using 1M bp window, and the other was for gene specific copy number measurement. ¹ LSTs: n of chromosomal breaks between adjacent regions of at least 10 Mb. ² PGAs: percentage of a patient's genome harboring copy number alterations (amplification or deletions).

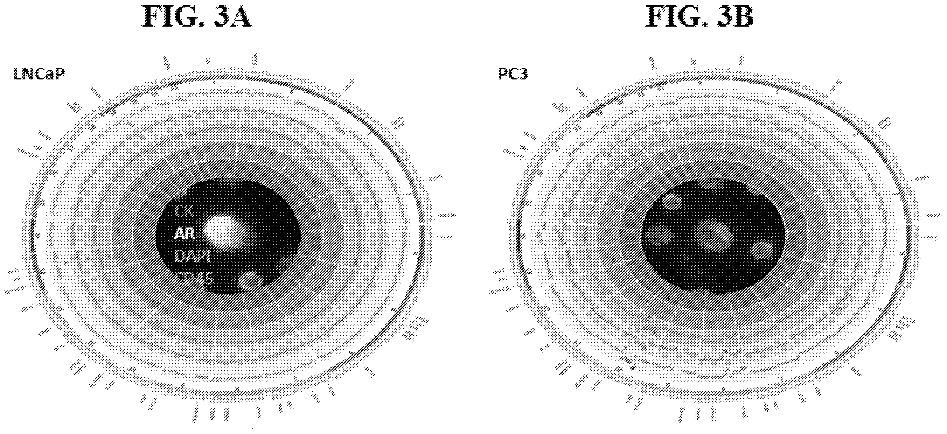

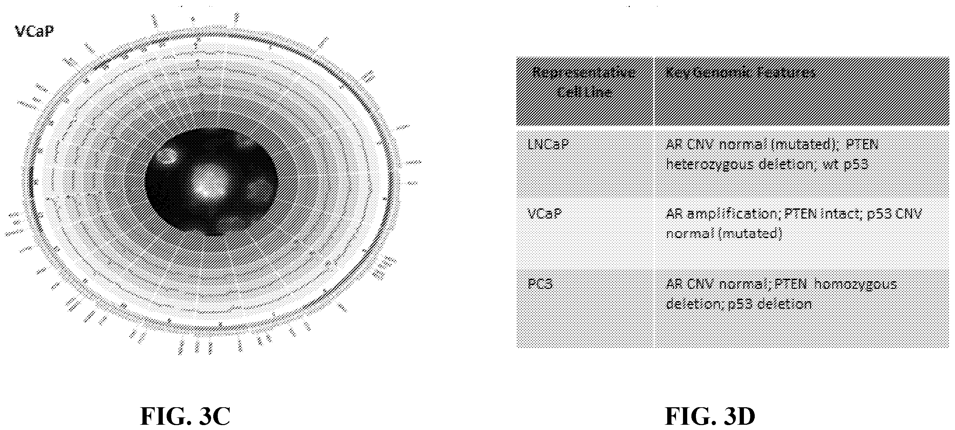

Figures 3A-3D show copy number variations (CNVs) in single cells. Single cells each from LNCaP, PC3, and VCaP (Figures 3A-3C) were isolated and analyzed by whole genome sequencing for copy number variations. Amplifications and deletions can be observed reproducibly across replicates. Representative images of each cell line are also shown. Cells are stained with a CK cocktail, AR, CD45, and DAPI. Replicates of 5 from each cell line are shown here to demonstrate reproducibility. Known genomic alterations from each cell line are described in Figure 3D. Plots were generated with Circos: Krzywinski, M. et al. Circos: an Information Aesthetic for Comparative Genomics. Genome Res (2009) 19:1639-1645

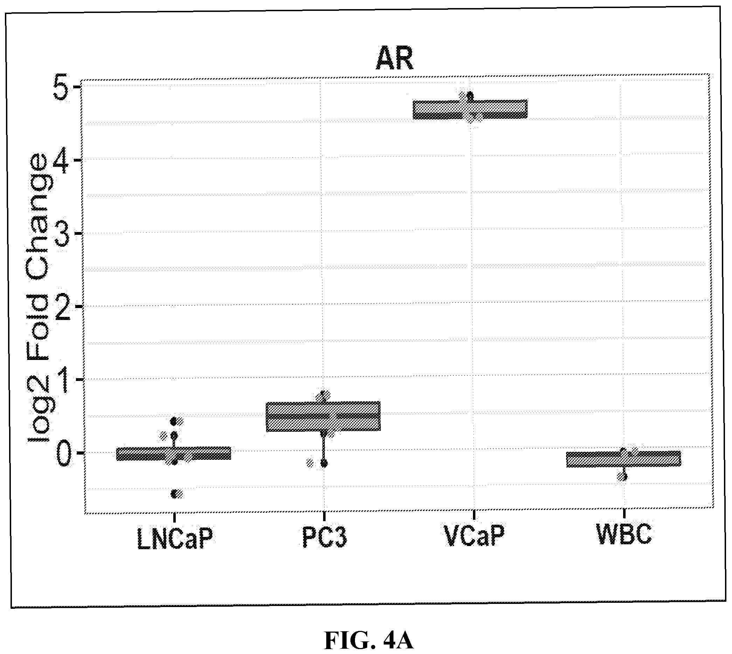

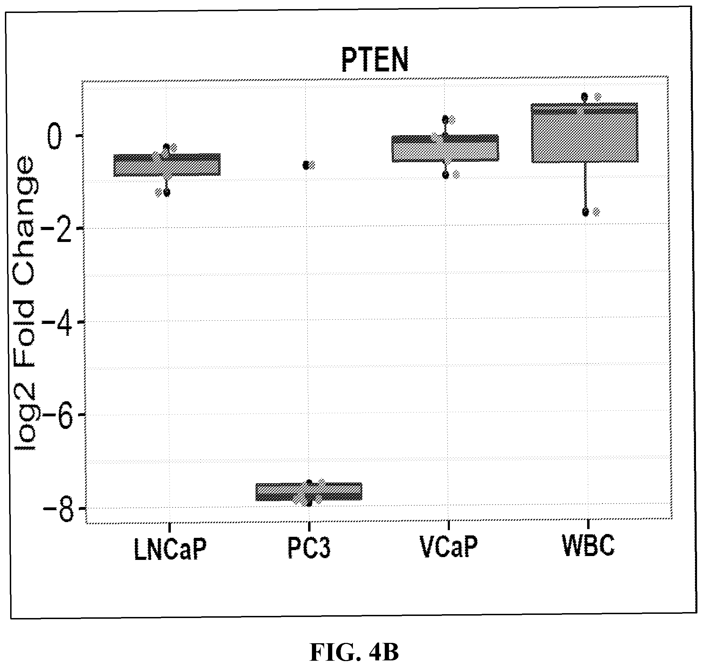

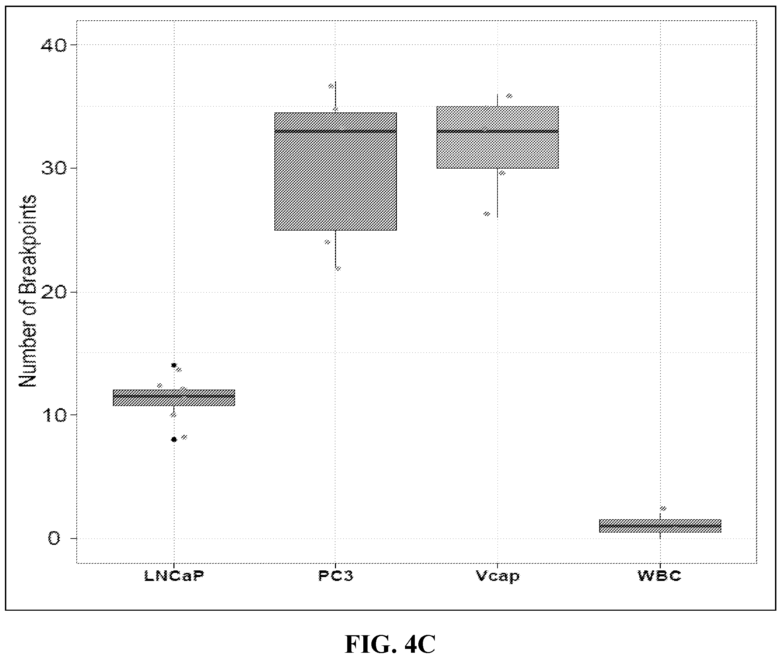

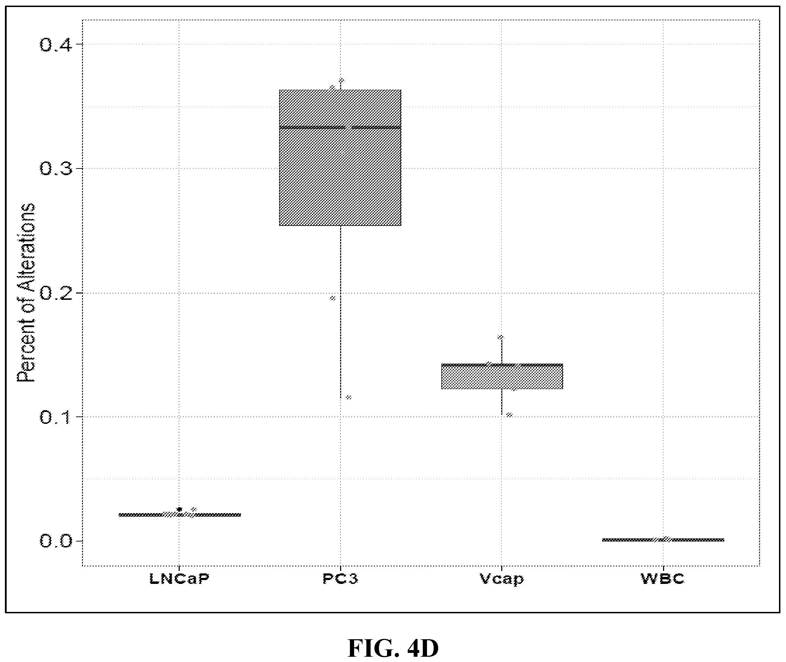

Figures 4A-4B show CNV and Figures 4C-4D show Genomic Instability Measurements. Figure 4A shows comparison of log2 genomic copy number of AR in 3 representative cell lines and healthy donor white blood cell (WBC) control. VCaP harbors an amplification of AR, while LNCaP and PC3 maintain 2 copies of AR. Figure 4B shows comparison of log2 genomic copy number of PTEN in 3 representative cell lines and healthy donor WBC control. PC3 homozygous PTEN loss was confirmed, LNCaP heterozygous PTEN loss was observed in many cells with significant z-scores. Figure 4C shows comparison of the # of breakpoints (LSTs) across 3 representative cell line and healthy donor WBC control. A higher number of breakpoints were detected in PC3 (PTEN null, p53 mutant) and VCaP (p53 mutant) in comparison to LNCaPs (wt p53 and heterozygous PTEN loss) and the WBC control. Figure 4D shows comparison of the % of genome altered in 3 representative cell lines and healthy donor WBC control. PC3 displayed the highest percent of alterations, revealing genetic instability and polyploidy, likely due to loss of both PTEN and p53.
Figure 5 shows a schematic of the "no cell selection" platform used to isolate and analyze CTCs at the single cell level by morphology/protein chemistry (Facial Recognition).
Figure 6 shows that following determination of protein and morphological features of CTCs, a series of individual cell features were measured on each CTC identified in a patient sample, including nuclear area as well as other features set forth in the the table.
Figure 7 shows a heat map on the right, where the 15 cell types are defined by the colors on the y axis, and the individual features on the x axis. Red reflects features on the low end of dynamic range (i.e. small nuclear area), while green reflects features on the high end of the dynamic range (i.e. large nuclear area).
Figure 8 shows patients were ranked based on how heterogeneous or diverse the cells were at each decision point.
Figure 9 shows the demographics of the mCRPC population.
Figure 10 shows the frequencies of the 15 different phenotypic CTC classes differed by line of therapy and were more heterogeneous over time. Red represents prevalence of a cell type that is overrepresented or which is more diverse. Each column is a patient, such that columns with many vertical red sections have higher phenotypic heterogeneity.
Figure 11 shows that higher Shannon Indexes showed greater diversity (heterogeneity) by line of therapy, notably with the increase in the median, and fewer lower index scores in the 3^{rd} and 4^{th} line of therapy.
Figure 12A shows that high CTC phenotypic heterogeneity predicts shorter progression and survival times on AR therapy but not taxane therapy. Figure 12B shows outcomes on AR Tx based on heterogeneity.
Figure 13 shows that high CTC phenotypic heterogeneity predicts a better outcome with a Taxane over AR Tx in a multivariate model. A range of factors previously shown to be prognostic for survival were studied in univariate and multivariate analysis - only the multivariate is shown. High heterogeneity predicted for sensitivity to taxanes over AR therapies.
Figure 14 shows the prevalence of a CTC subtype (Type K) predicts poor outcome on both ARTx and Taxanes independent of AR status. One particular mathematically defined cell type, type K had a large nucleus, a wide range of nuclear sizes and prominent nuclei - was associated with resistance to both classes of drugs.
Figure 15 shows a schematic of the process by which the CTCs are amplified, prepared for sequencing, followed by sequencing informatics to assess clonality and amplification/deletions.
Figure 16 shows single cell CTC sequencing informs of clonal diversity and phylogenetic disease lineage.
Figure 17 shows that single CTC CNV profiles inform clonal diversity and phylogenetic disease lineage.
Figure 18 shows that single CTC sequencing can also inform of a lack of clonal diversity in a 2nd line post taxane patient who might not be considered for ARTx. This patient responded to enzalutamide.
Figure 19 shows that CTC phenotypic heterogeneity correlates with genomic heterogeneity.
Figure 20A shows and example of Cell Type K genomics, characterized by frequent CNVs, high number of breakpoints and an accompanying phenotype characterized by a large nucleus, high nuclear entropy and frequent nucleoli. Figure 20B shows genomic instability for cell type K compared to all other CTC phenotypes.
Figure 21 shows that high phenotypic heterogeneity is an informative biomarker in AR-V7 negative patients.
Figure 22 shows low phenotypic CTC heterogeneity in 6 CTCs from a patient prior to first line therapy that show a homogenous genomic profile.
Figure 23 show a heatmap of 15 mathematical CTC phenotypic subtypes were identified using unsupervised analysis based on CTC protein and morphological features.
Figures 24A-24O depict selected features of the 15 cell types A-O, respectively. Certain CTC phenotypic subtypes prognosticates patient survival.
Figure 25 shows the prediction of death by 180 days on ARS (n = 150 samples) by CTC enumeration and 15 CTC phenotypic subtypes. Good prognosticators include cell type E (cluster 5), K (cluster 11), and O (cluster 15).
Figure 26 shows that some CTC phenotypic subtypes (cell type E, K and N) predicts mCRPC patient response to AR targeted therapy.
Figure 27 shows CTC phenotypic subtypes (cell type G, K and N) that predict response to taxane therapy.
Figure 28 shows cluster 11 (cell type K) has large nucleus, high nuclear entropy and frequent nucleoli.
Figure 29 shows multiple cell types (cell type G, K, and M) are predictive of genomic instability (LST). These particular subtypes, given the increased genomic instability, may be sensitive to DNA damaging drugs, such as platinum based chemotherapies (i.e. carboplatin, cisplatin), or targeted therapeutics which target homologous recombination deficiencies, including Poly ADP-ribose Polymerase (PARP) inhibitors, DNA-PK inhibitors and therapeutics targeting the ATM pathway.
Figure 30 shows five morphological and protein expression features found to be predictive of CTC genomic instability. The first four features are positively correlated with genomic instability and the last one is negatively correlated.
Figure 31 shows that CK(-) CTCs have higher incidence of and are predictive of genomic instability.
Figure 32 shows that protein and morphological features can predict CTC genomic instability with high accuracy. The Y axis shows the real LSTs (nBreakPoints) and X axis shows the predicted instability (stable vs. unstable). The CTCs predicted as high genomic instability, may be sensitive to DNA damaging drugs, such as platinum based chemotherapies (*i.e.* carboplatin, cisplatin), or targeted therapeutics which target homologous recombination deficiencies, including PARP inhibitors, DNA-PK inhibitors and therapeutics targeting the ATM pathway.
Figure 33 shows that phenotypic heterogeneity is predictive of overall survival and response to AR targeted therapy.
Figure 34 shows that CTC phenotypic heterogeneity is predictive of genotypic heterogeneity. High phenotypic heterogeneity is 40 times more likely to represent multiple genomic clones than low phenotypic heterogeneity.
Figure 35 shows that CTC genomic instability is predictive of mCRPC patient overall survival.
Figure 36 shows that that CTC genomic instability is predictive of mCRPC patient response to Taxane therapy.
Figures 37A-37C show Large-scale state transitions (LST) and percent genome alteration (PGA) measured as the surrogate of genomic instability. LSTs: n of chromosomal breaks between adjacent regions of at least 10 Mb. Popova et al., Cancer Res. 72(21):5454-62 (2012). PGAs: percentage of a patient's genome harboring copy number alterations (amplification or deletions). Zafarana et. al, Cancer 2012 Aug; 118(16): 4053 (2012). Examples: High LST (27) and High PGA (23%)
Figure 38 shows a graph depicting the value of correlation coefficient of each imaging feature (along y-axis) to predict aLST. Correlation coefficients closer to 0 indicate features that do no trend positively/negatively with aLST. Values >> 0 or << 0 indicate features that strongly trend positively or negatively with aLST and therefore may be more predictive of aLST.

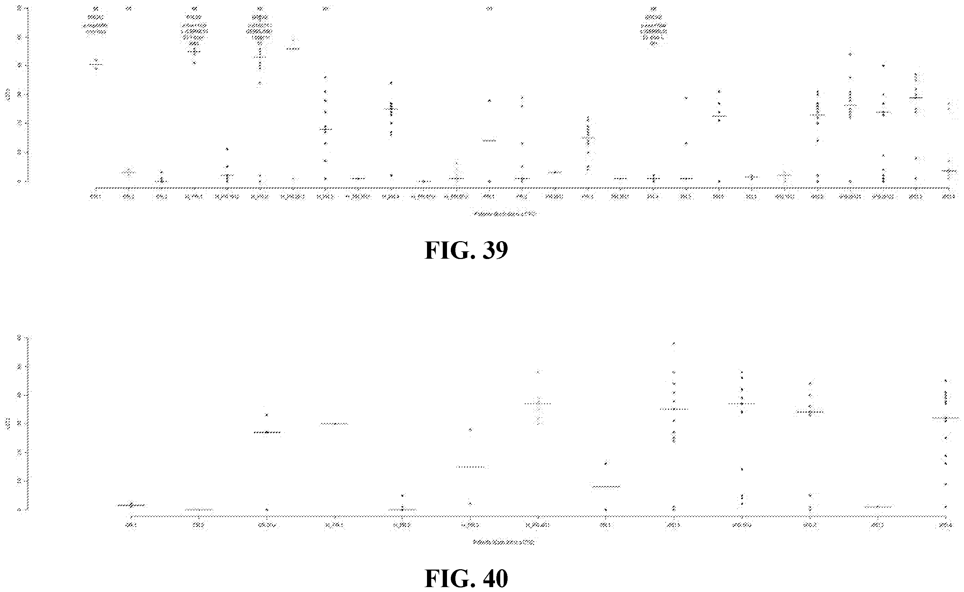

Figure 39 shows that CTCs from mCRPC patients with germline BRCA2 mutations or other HRD (homologous recombination deficiency) pathway gene deleterious mutations commonly have much higher LST scores, with median scores over 40 as observed in our study. Plot below shows three BRCA2 or HRD mutant (Mt) samples (CR. 1, H_PR.1, and H_PR.2) have the highest LSTs than the rest of samples. mCRPC patients with high LST scores (median LST > 30) respond well to PARPi+ARS (AR Signaling inhibitor, including Abiraterone and Enzalutamide) therapy with either complete response or >90% response. CR: complete response; H_PR: >90% response; PR: >50% response; SD: stable disease; xPD: progression.
Figure 40 shows that mCRPC patients with high LST scores (median LST > 30) resist ARS therapy alone.

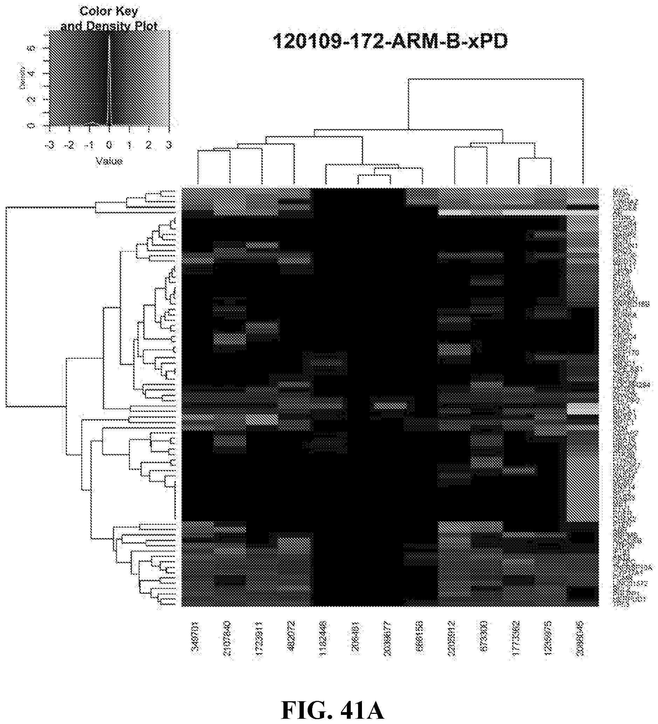

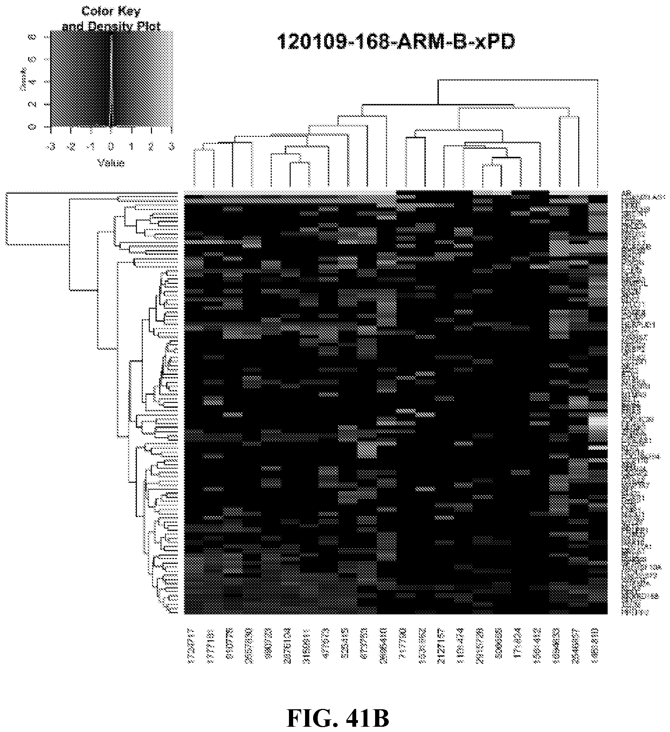

Figures 41A-41B show heat maps for two patients with co-occurrence of AR gain and PTEN loss resist PARPi+ARS therapy. Out of a cohort of 30 mCRPC patients, two patients had co-occurrence of AR gain and PTEN loss. Both patients resistant to PARPi+ARS therapy.

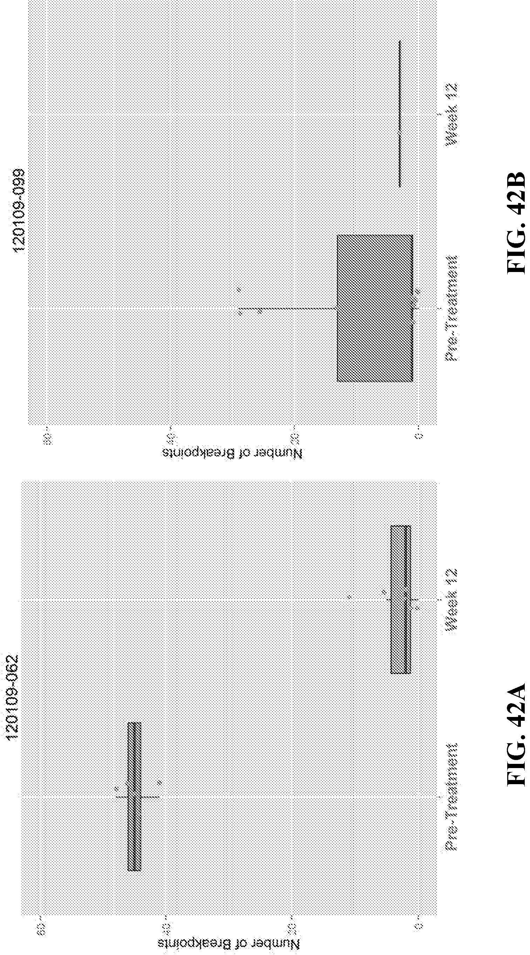

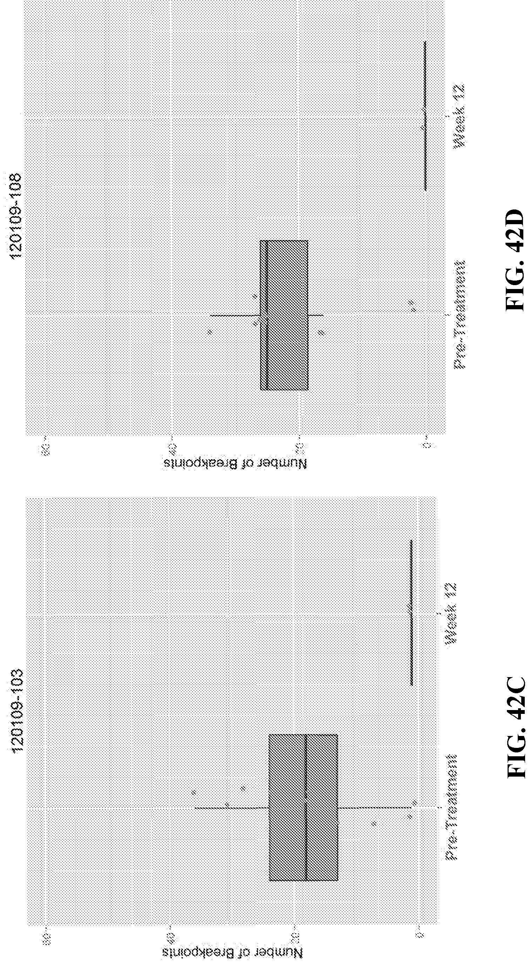

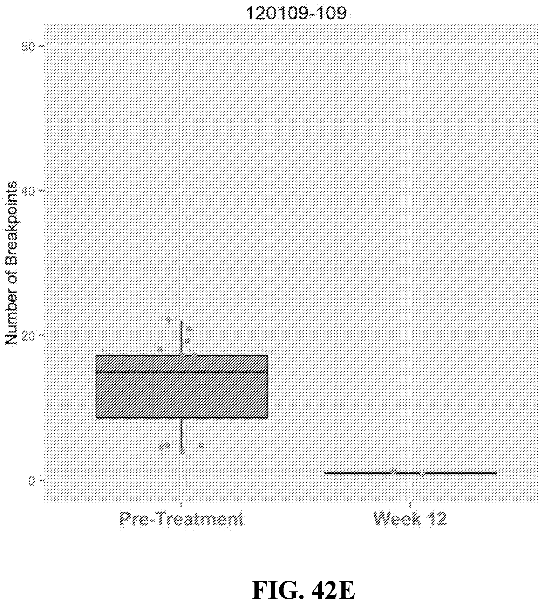

Figures 42A-42E show that for mCRPC patients treated with PARPi+ARS, at the time point that patient responded to therapy, the follow up blood draw CTCs did not have high LST CTCs. This suggested that high LST CTCs were sensitive to the therapy and it can be utilized as a response marker. Figures 42A through 42E correspond to five patient examples.

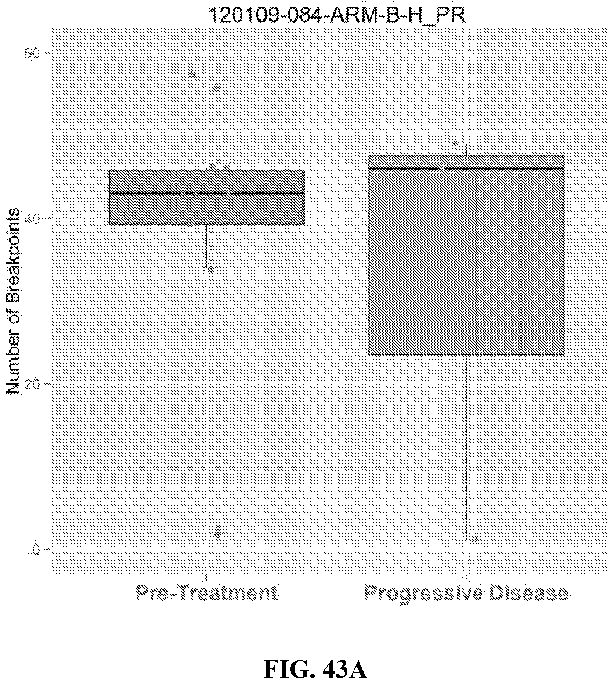

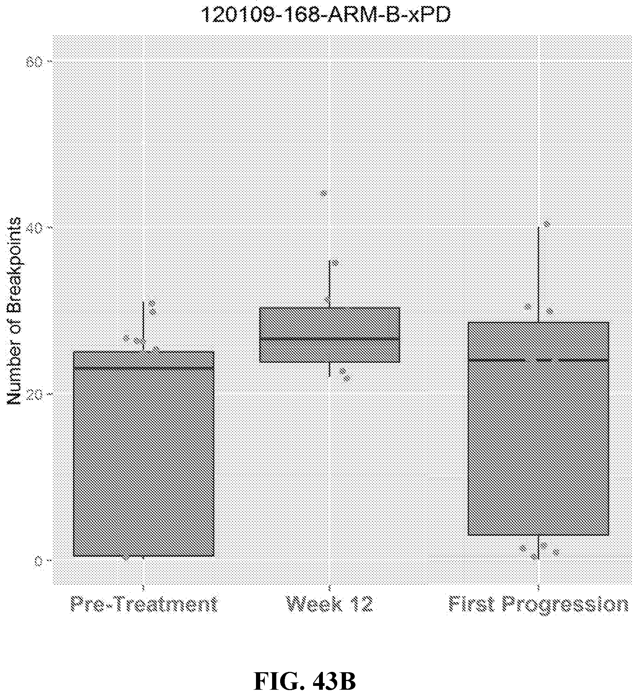

Figures 43A-43B show that for mCRPC patients treated with PARPi+ARS, at the time point that patient disease progressed, the follow up blood draw CTCs did have high LST CTCs. This suggested that high LST CTCs were indicators of disease progress or recurrence. See two patient examples below. Figure 43A, Patient 120109-084 had a short term response to PARPi+ARS and had a recurrence disease when the follow up ("Progressive Disease") sample was taken. Figure 43B, Patient 210109-168 did not respond to PARPi+ARS therapy and two blood draw samples were taken at week 12 and 16.

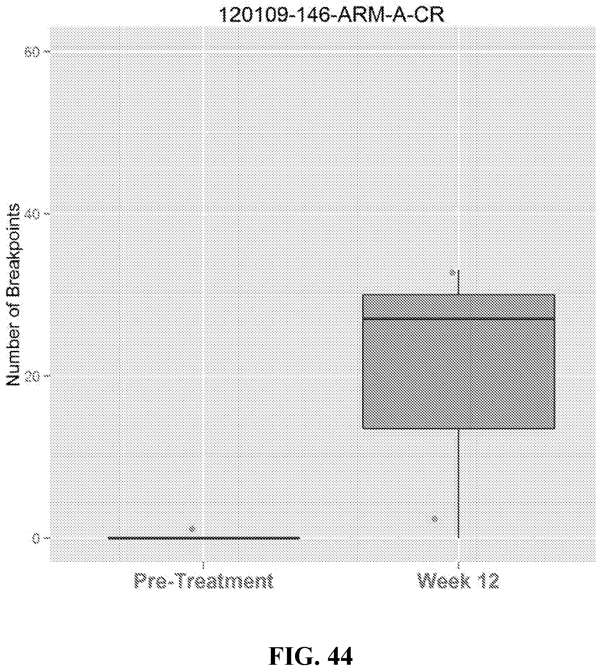

Figure 44 shows that for mCRPC patients treated with ARS alone, at the time point that patient responded to therapy, the follow up blood draw CTCs still have high LST CTCs. This suggested that high LST CTCs were not sensitive to ARS therapy. Other therapy (e.g. PARPi) or combination therapy with PARPi might be needed.

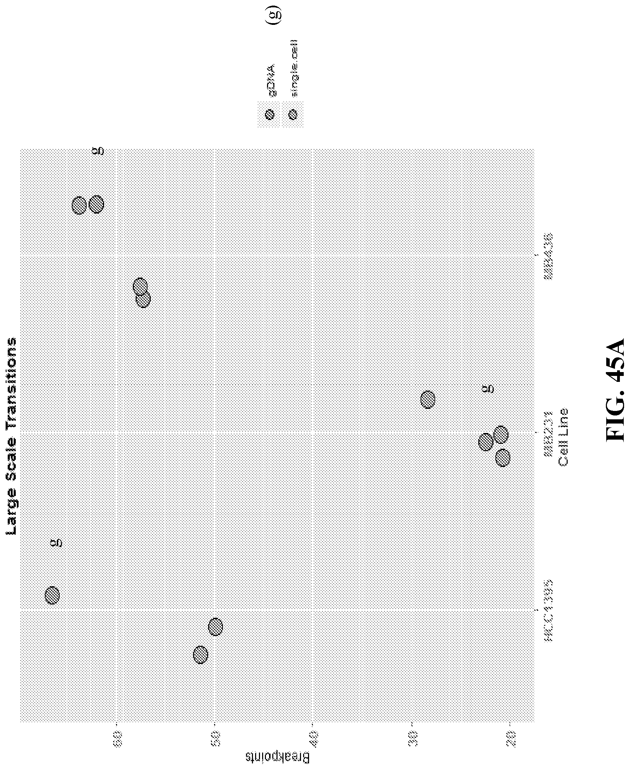

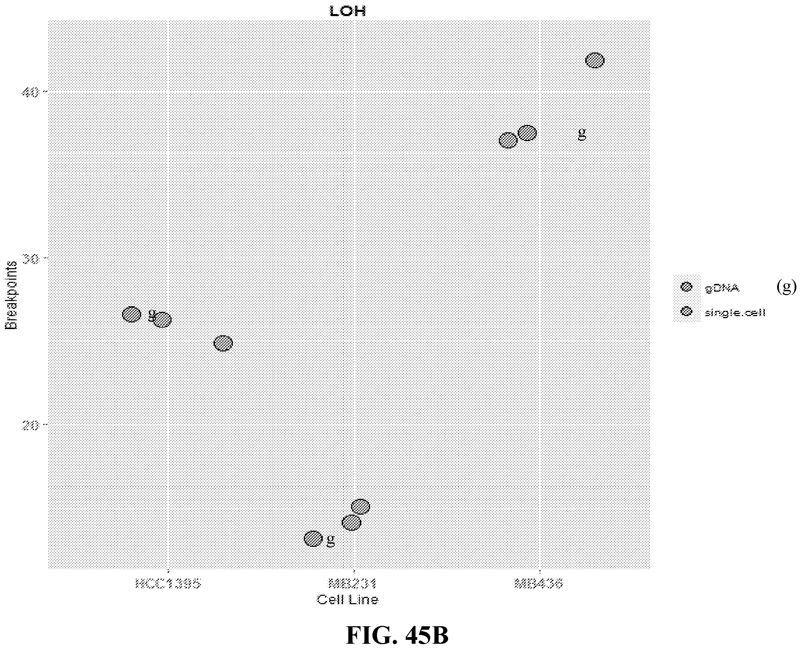

Figures 45A-45B show that cell lines that have high genomic scarring, such as LST and LOH, are more likely PARPi sensitive. 2 BRCA mutant, PARPi sensitive TNBC cell lines (HCC1395 and MB436) have much higher LST scores (Figure 45A) and LOH scores (Figure 45B) than the BRCA wild type, PTEN and TP53 mutant TNBC cell line (MB231).

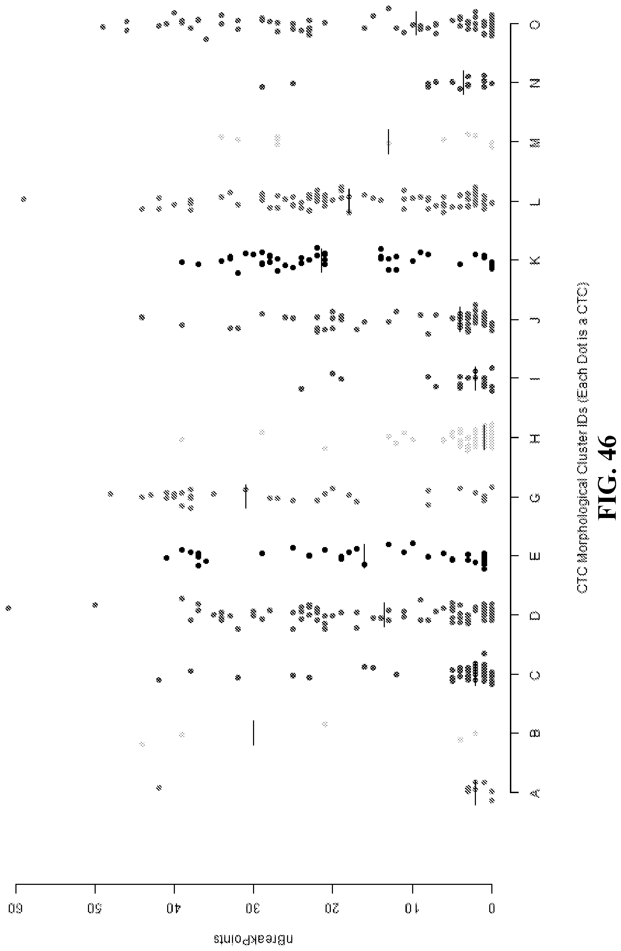

Figure 46 shows that LSTs are associated with phenotypic cell types. Cell type B, D, E, G, K, L, M and O have higher LSTs than the rest of cell types.

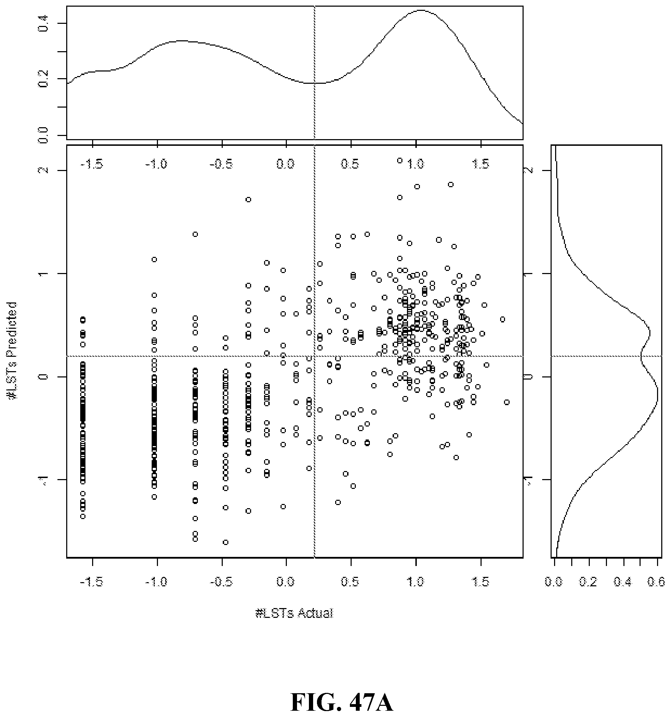

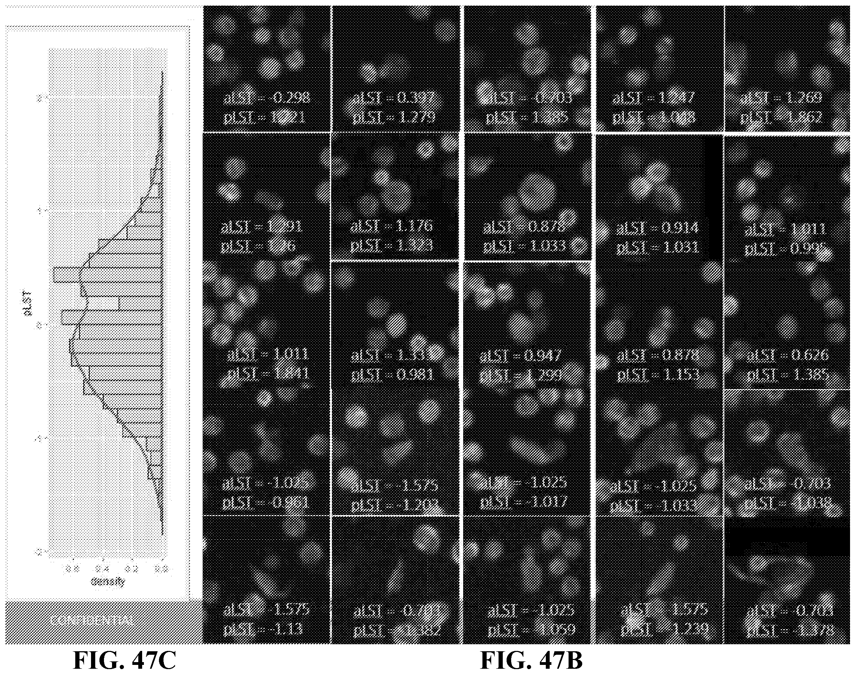

Figures 47A-47C demonstrate that LSTs can be predicted by a regression algorithm using CTC phenotype features, including N/C ratio, nuclear & cytoplasm circularity, nuclear entropy, CK expression and AR expression. AR expression data is preferred but optional in the prediction model. LST prediction model was tested on an independent prostate and breast cancer cohort, with accuracy of 78%. On patient level, the concordance rate between aLST and pLST is 95% (36 out of 38 samples) in determination of patient LST categorization (high or low). High LST patient was defined as patient with at least four CTCs with pLST > 0.37 or aLST > 8. Figure 47A shows actual LST scores via Sequencing (x) vs predicted LST (pLST) scores via Algorithm (y). Figure 47B shows examples of cell images with wide range of LSTs. Both aLST and pLST in these plots were log10 transformed and Z scale normalized (Figure 47C).

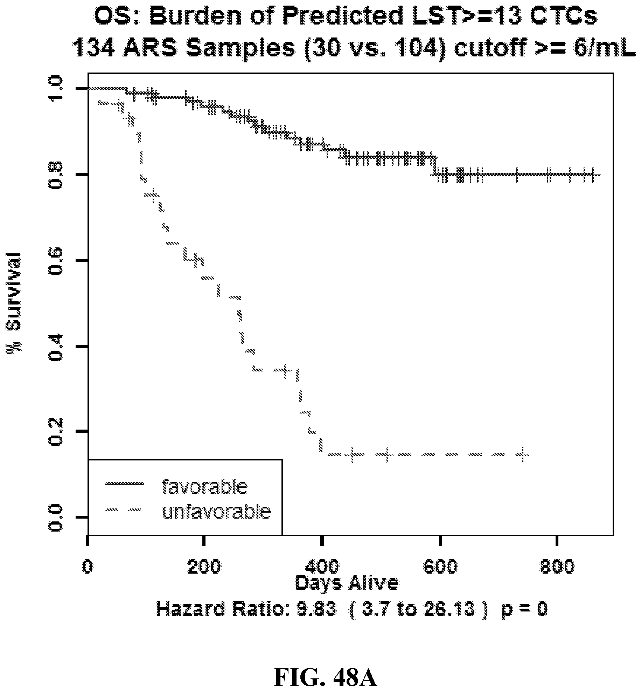

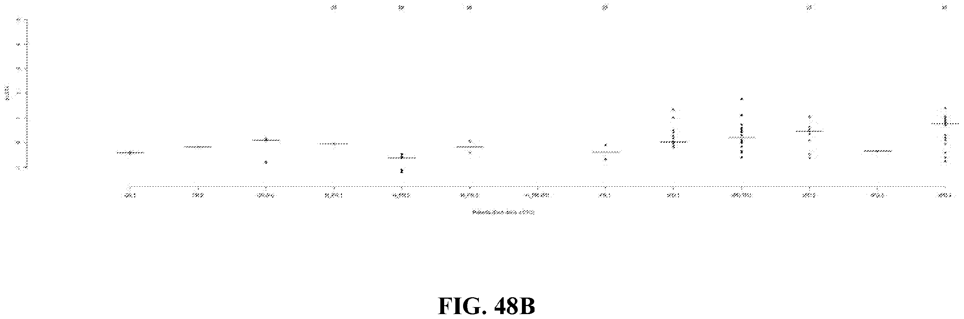

Figures 48A-48B show that patients with high pLSTs are resistant to AR targeted therapy. In first line mCRPC patient with high LSTs, 43% (6/14) patients responded to AR targeted therapy. In seven patients with both baseline and follow-up samples (<18 weeks), number of high pLST went up from 35 cells in baseline to 122 (320%) in follow-up samples. See example data from two independent mCRPC cohort.

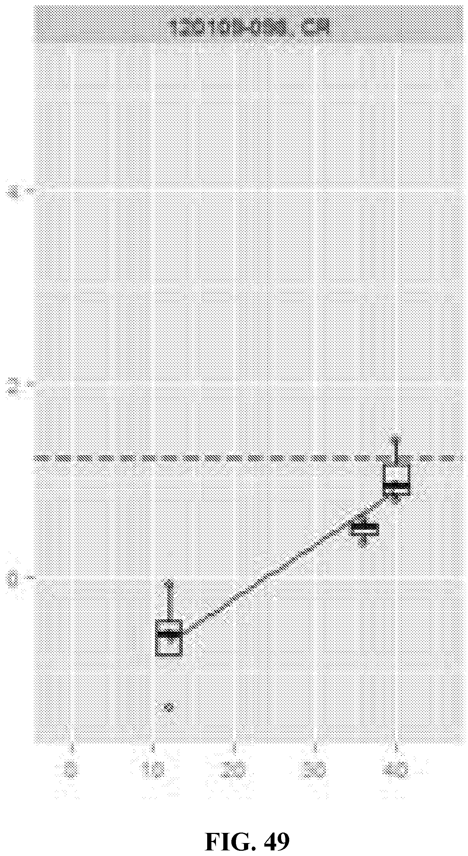

Figure 49 shows that patients with low pLSTs that initially responded to AR targeted therapy, could have high pLST CTCs detected in follow up samples suggesting disease progression and acquired resistance.

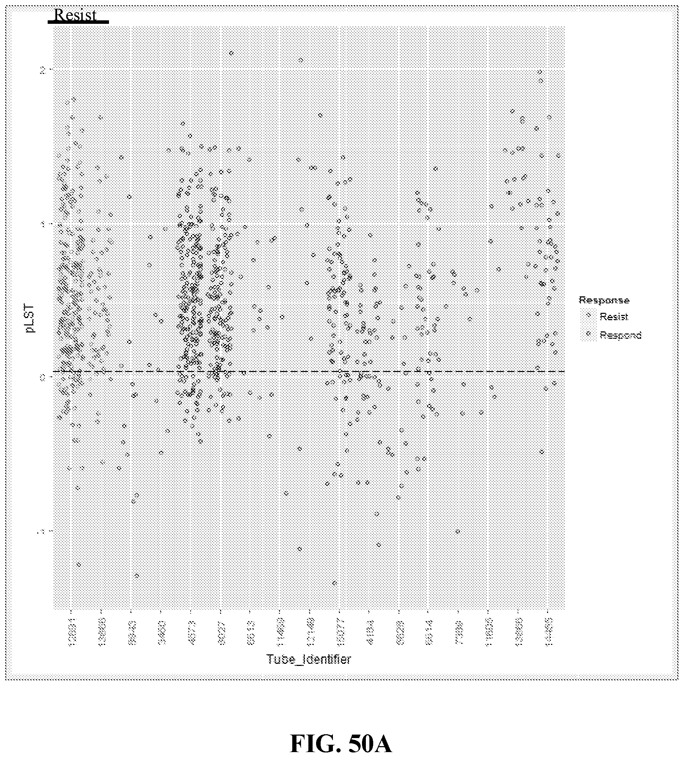

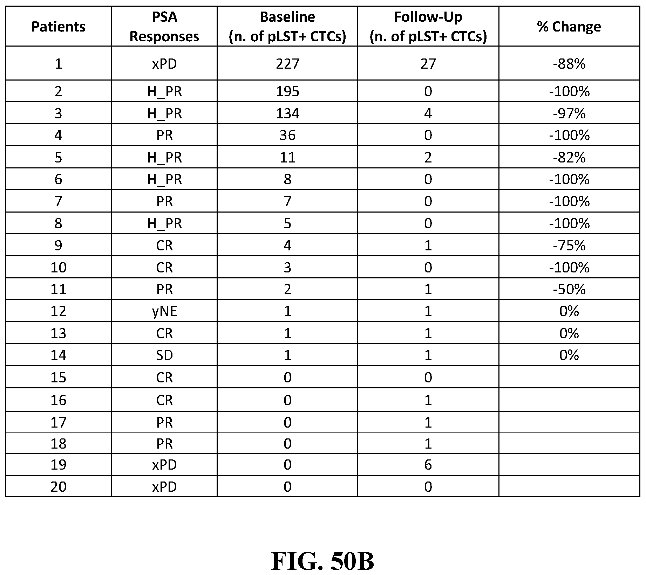

Figures 50A-50B show that patients with high pLSTs respond well to PARPi+ARS therapy. Figure50A shows, in first line mCRPC patient with high LSTs, 88% (15/17) patients responded to PARPi+AR targeted therapy. Figure 50B shows, in 20 patients with both baseline and follow-up samples (<18 weeks), number of high pLST went down from 635 cells in baseline to 33 (down 95%) in follow-up samples.

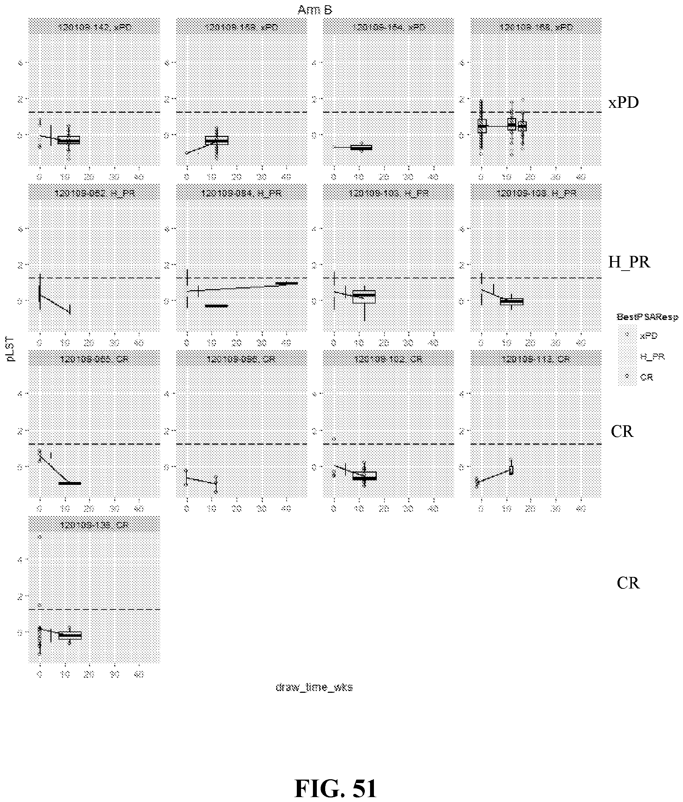

Figure 51 shows that patients with high pLSTs respond to PARPi+ARS therapy, and over time, high pLST CTC populations fall in follow up samples. This indicates that pLST can be used as biomarker for monitoring drug response.

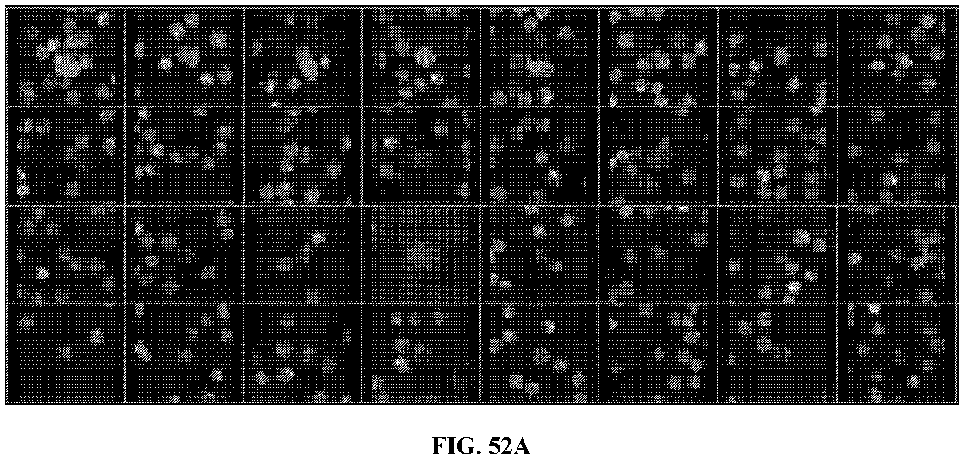

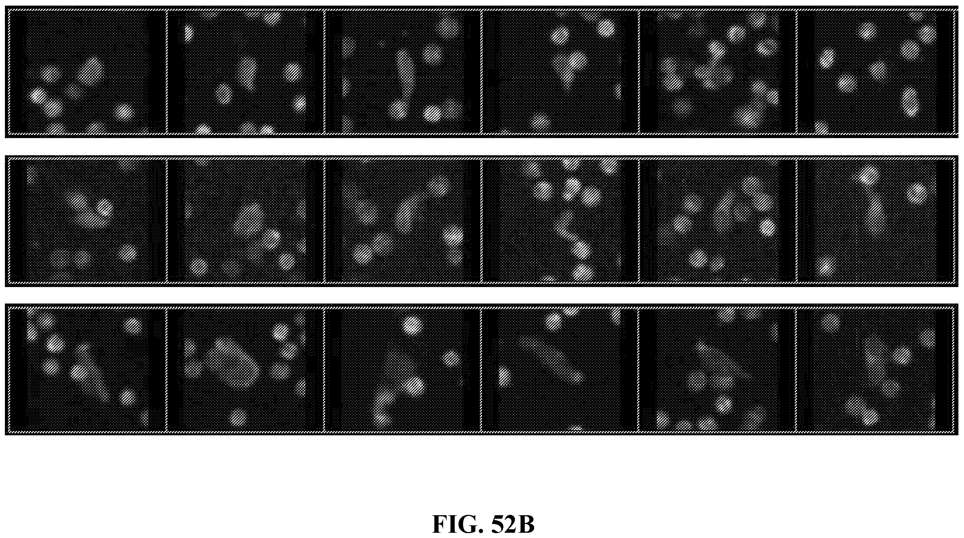

Figures 52A-52B show that mCRPC Patients with high pLST respond to platinum-based agents treatment. Figure 52A shows cell images from one 10^{th} line mCRPC patients with 96% baseline CTCs as high pLST, and the patient responded to carboplatin therapy (12 week PSA change: -50.1%). Figure 52B shows cell images from one 8^{th} line mCRPC patients with 4.3% baseline CTCs as high pLST, and the patient did not respond to carboplatin therapy (12 week PSA change: +2.1%).

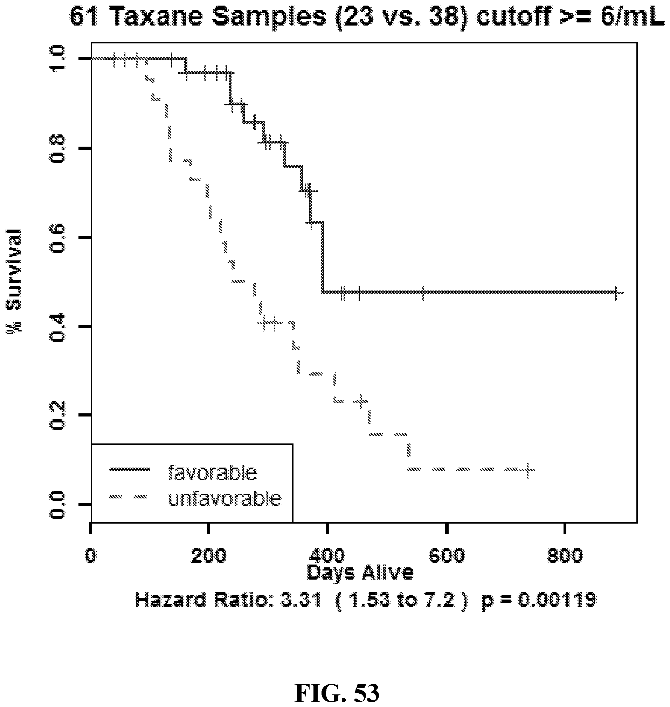

Figure 53 shows that patients with high pLSTs are resistant to Taxane therapy in an overall survival analysis. Favorable group included patients with < 6 high pLST CTCs and unfavorable group included patients with >= 6 high pLST CTCs.

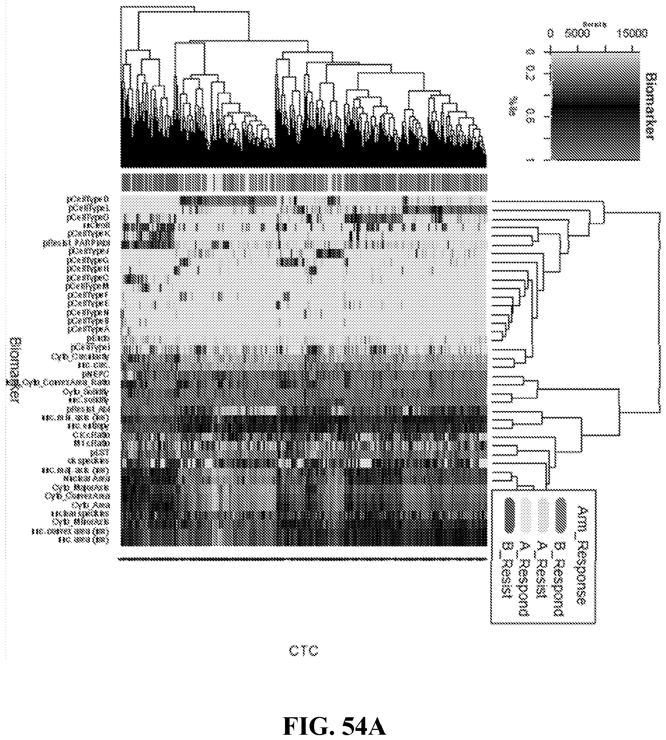

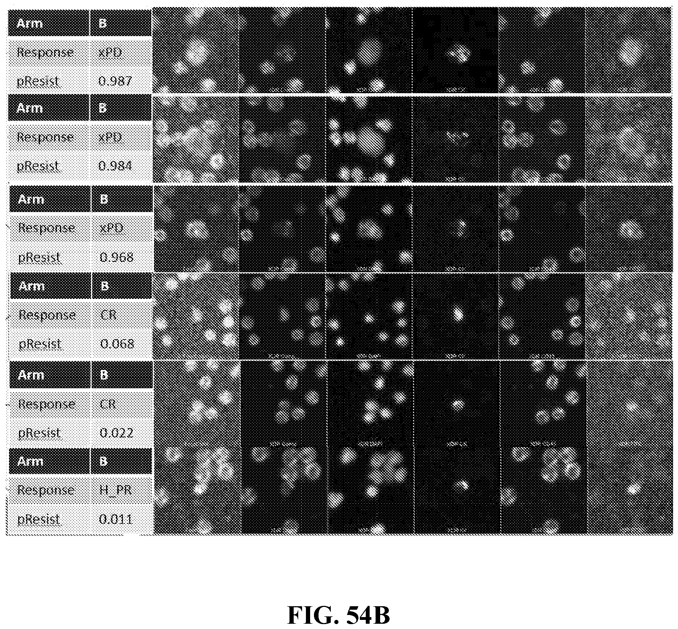

Figure 54A shows the correlation between pResist with cell morphological features and phenotypic cell types. Figure 54B shows examples of cell images for high vs. low pResist cells. The most important features used in the classifier include nuclear area, nuclear convex area, nuclear speckles, nuclear major axis, cytoplasm area, cytoplasm convex area, cytoplasm minor axis, AR expression, cytoplasm major axis. Cell type K, C and M have higher pResist than the rest of cell types.

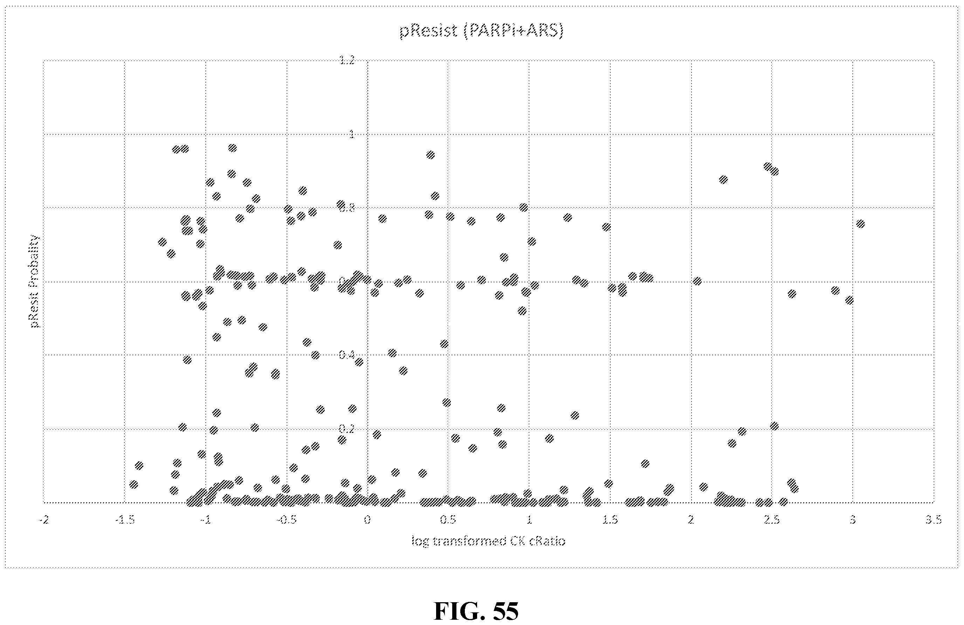

Figure 55 shows many of the pResist cells are CK- CTCs, suggesting their EMT origins.

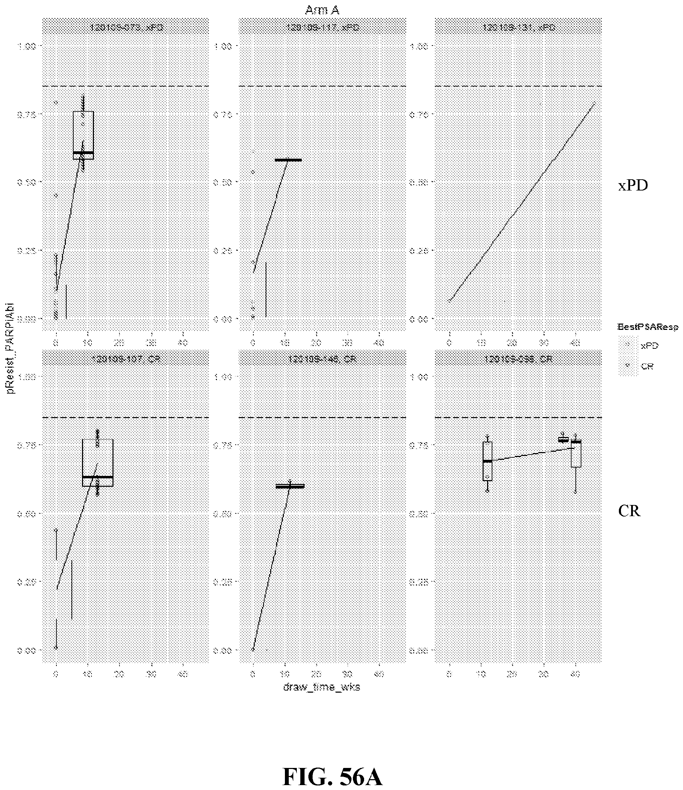

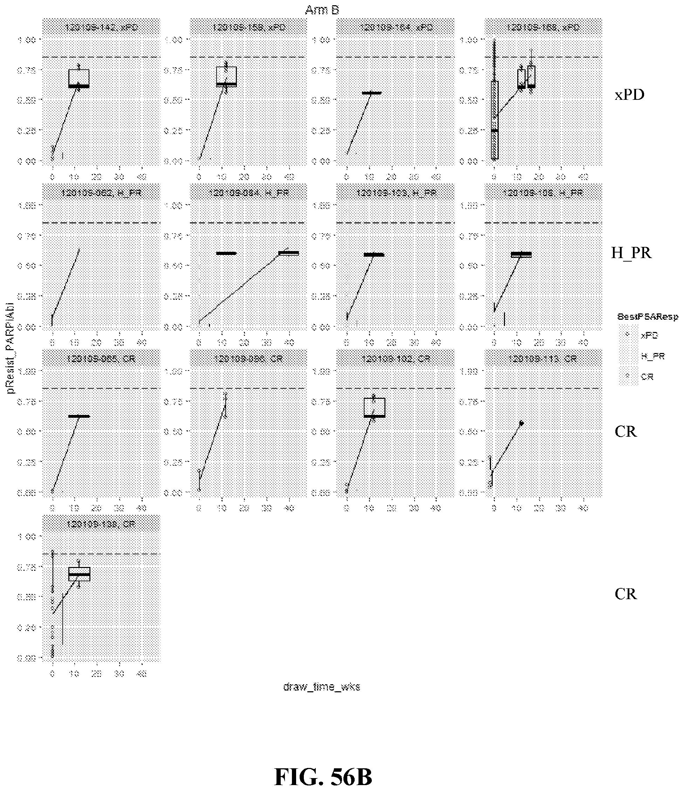

Figures 56A-56B depict longitudinal study showing that pResist cells trends upwards for all patients in ARS only or PARPi+ARS patients.
Figures 57A-57C show Time on therapy (Time on Tx) (Figure 57A), radiographic progression free survival (rPFS) (Figure 57B) and overall survival (OS) (Figure 57C) in patients treated with abiraterone (Abi) or enzalutamide (Enza).
Figures 58A-58C show Time on therapy (Time on Tx) (Figure 58A), radiographic progression free survival (rPFS) (Figure 58B) and overall survival (OS) (Figure 58C) in patients treated with abiraterone (Abi), with or without the presence of cell type K.
Figures 59A-59C show Time on therapy (Time on Tx) (Figure 59A), radiographic progression free survival (rPFS) (Figure 59B) and overall survival (OS) (Figure 59C) in patients treated with enzalutamide (Enza), with or without the presence of cell type K.
Figures 60A-60C show Time on therapy (Time on Tx) (Figure 60A), radiographic progression free survival (rPFS) (Figure 60B) and overall survival (OS) (Figure 60C) in patients with the presence of cell type K, treated with abiraterone (Abi) or enzalutamide (Enza).
Figures 61A-61C show Time on therapy (Time on Tx) (Figure 61A), radiographic progression free survival (rPFS) (Figure 61B) and overall survival (OS) (Figure 61C) in patients without the presence of cell type K, treated with abiraterone (Abi) or enzalutamide (Enza).
Figure 62 shows treatment-specific hazards of death (overall survival) for treatment with abiraterone (Abi) or enzalutamide (Enza).

### DETAILED DESCRIPTION

The present disclosure is based, in part, on the discovery that integrated single cell whole genome CNV analysis provides reproducible copy number profiles across multiple replicates and confirms the presence of known focal CNV events including AR amplification and PTEN loss. The present disclosure is further based, in part, on the discovery that whole genome copy number analysis can be used to reproducibly characterize genomic instability by measuring LSTs and PGA. As disclosed herein, the highest genomic instability detected in p53 mutant cell lines (PC3 & VCaP) compared to wild-type (LNCaP). Understanding the frequency of subclonal CNV driver alterations and genomic instability in individual CTCs in combination with cell phenotype may enable a more accurate view of heterogeneous disease, potential therapeutic response, and identify novel mechanisms of resistance.

The present invention is further based on the identification of rare subtypes of CTCs that, even when composing just a minor fraction of the total CTC population, predict shorter overall survival and drug resistance. As described further below, the methods of the invention are further based, in part, on the surprising identification of a rare CTC subtype via an artificial intelligence algorithm that classifies CTCs based on 20 discrete morphologic and protein expression features, and was found in a subset of patients. Patients whose blood contained this type of CTC universally failed all therapies recorded in their medical records and experienced much shorter overall survival. As exemplified herein, subsequent genome sequencing of this CTC subtype found that the cells shared a genomic signature distinct from other CTCs, confirming that a CTC's genomic features may be inferred by visual analysis.

The present invention is further based on the identification of specific types of CTCs and the correlation of the presence or absence of specific types of CTCs with therapeutic efficacy of certain drug treatments. As disclosed herein, a CTC cell type, referred to herein as cell type K, has been identified as a predictive biomarker that correlates with the effectiveness of treatment with abiraterone or enzalutamide. As disclosed herein, it has been determined that the presence of cell type K in a patient correlates with a more favorable response to abiraterone, whereas the absence of cell type K in a patient correlates with a more favorable response to enzalutamide. The presence or absence of cell type K in a sample of CTCs is predictive of patient responsiveness and outcomes to treatment with abiraterone or enzalutamide. Thus, the methods of the invention can be used to determine whether a cancer patient is better suited to treatment with abiraterone or enzalutamide.

Increased intra-tumor heterogeneity has been correlated with intrinsic resistance to therapy and poor outcome. CTCs have been shown to reflect heterogeneous disease and the active metastatic tumor population in metastatic patients. Exemplified herein is an analysis of heterogeneity in CTCs on a cell by cell basis and the surprising discovery that heterogeneity is a predictive biomarker of sensitivity at decision points in therapy management that enables better sequencing of available therapies. The non-enrichment CTC analysis platform described herein enables the methods of the invention by allowing for single cell resolution and accurate genomic profiling of heterogeneous CTC populations. To characterize intra-tumor heterogeneity single cell whole genome copy number analysis of circulating tumor cells (CTCs) was performed using a non-enrichment CTC analysis platform.

Markers of therapeutic sensitivity, such as PTEN deletion or androgen receptor (AR) amplification for PI3K inhibitors or AR-targeted therapy, respectively, were detected in individual prostate cancer cells spiked into blood to mimic patient samples (Example 1). In addition to the detection of focal actionable alterations, genomic instability was characterized by measuring large scale transitions (LSTs) and % genome altered (PGA).

As shown herein, analysis at the single cell level enables heterogeneity to be explored in different ways. Phenotypic or cellular heterogeneity that measures variation in morphology and cell-by-cell gene expression in tumor cells that emerge from a single clone and can detect lineage switching (plasticity), for example, loss of androgen receptor (AR) expression and detection of the TMPRSS2:ERG gene fusion. Genotypic heterogeneity detects single regions in a tumor with distinct mutational profiles evolving from a single initiating trunk lesion. An important application of the analysis of CTCs at the single cell level is to guide targeted therapy. As exemplified herein, by sequencing and comparing multiple single cells, it is possible to construct a phylogenetic tree and heatmap that reveals the clonal substructure of a tumor. These genetic trees enable identification of founder mutations in the "trunk" of the tree, which are ideal therapeutic targets, since they occurred early in tumor evolution and were inherited by all cells in the tumor. Alternatively, these trees can be used to devise combination therapies to target multiple tumor subpopulations independently.

Genetic plasticity is one of the enabling characteristics of cancer, in which the acquisition of the multiple cancer hallmarks depends on a succession of alterations in the genomes of neoplastic cells. This plasticity results from ongoing accumulation of additional somatic mutations that are then positively selected. This high degree of genetic variability provides a ready substrate for an evolutionary optimization process, as subclones compete over resources and adapt to external pressures such as cancer therapy. Cancer progression, therefore, is fundamentally a process of mutational diversification and clonal selection and tumors are composed of heterogeneous subpopulations. The methods of the invention allow for analysis at the single cell level and enables identification of subclonal populations.

The methods described herein enable characterization of CTCs in the blood of metastatic cancer patients by morphologic and protein features. As exemplified herein, these features, measured through fluorescent microscopy and employing cell segmentation and feature extraction algorithms can develop multiple biomarkers per cell identified. The examples provided show utilization of these feature to characterize >9000 CTCs from 221 metastatic patients to perform unsupervised clustering of the features sets. The features were reduced through principal components and then clustered into unique multi-dimensional subtypes. The present invention further provides a CTC subtype that is a biomarker for predicted resistance and worse survival to commonly used therapeutics (Abiraterone Acetate, Enzalutamide, Docetaxel, and Cabizataxel). Single cell genomic sequencing of this cell type identified the cell harbored increased genomic instability compared to other CTC subtypes through measurement of Large Scale Transitions (LSTs) within the genomes of the CTC. This particular subtype, given the increased genomic instability, is sensitive to DNA damaging drugs, such as platinum based chemotherapies (*i.e.* carboplatin, cisplatin), or targeted therapeutics which target homologous recombination deficiencies, including PARP inhibitors, DNA-PK inhibitors and therapeutics targeting the ATM pathway. Previous approaches to find biomarkers of sensitivity have focused on genomically sequencing tissue from patients for finding HRD genomics, while the present methods confer the ability to utilize digital pathology algorithms and avoid sequencing.

The methods described herein and accompanying examples demonstrate that single CTC phenotypic and genomic characterizations are feasible and can be used to assess tumor heterogeneity in a patient. High phenotypic heterogeneity identifies patients in a cohort with increased risk of death on Abiraterone & Enzalutamide but not taxane chemotherapy and that are 40 times more likely to have genomic heterogeneity (multiple clones). As exemplified herein, CTC clustering identifies a CTC subtype with resistance to both ARS and Taxane therapy and increased genomic instability (high LST breakpoints). The present methods provide a non-invasive liquid biopsy that enables the characterization of individual cells from a patient with metastatic cancer and can be used to guide treatment selection.

The present disclosure is further based, in part, on the discovery that LSTs are associated with phenotypic CTC types. As described herein, LSTs can be predicted by a regression algorithm using CTC phenotypic features, including N/C ratio, nuclear & cytoplasm circularity, nuclear entropy, CK expression and hormone receptor expression. In particular, the most important phenotypic features used in the classifier include nuclear area, nuclear convex area, nuclear speckles, nuclear major axis, cytoplasm area, cytoplasm convex area, cytoplasm minor axis, AR expression, cytoplasm major axis. CTC phenotypic features are used to determine a high versus a low LST score. Morphologic and protein expression features are collectively referred to herein as "phenotypic features."

As described herein, high LST scores in mCRPC patients predict resistance to ARS (AR Signaling inhibitor, including Abiraterone and Enzalutamide) therapy, including *de novo* resistance to ARS therapy as well as acquired resistance where an initially low LST score corresponded to response to ARS therapy. As exemplified herein, high LST CTCs are not sensitive to ARS therapy. In particular, as described herein, mCRPC patients treated with ARS therapy still have high LST CTCs at a follow-up blood draw taken at the time point the patient responded to therapy.

As further described herein, high LST scores in mCRPC patients predict response to PARPi+ARS therapy. Also described herein, high LST scores in mCRPC patients predict response platinum-based agents treatment, for example, carboplatin therapy.

As disclosed herein, high LST scores predict sensitivity to PARPi+ARS therapy and high LST CTCs can be utilized as a response marker in the methods of the invention. As exemplified herein, mCRPC patients treated with PARPi+ARS that responded to therapy did not have high LST CTCs on the follow up blood draw. As further described herein, high LST CTCs are indicators of disease progress or recurrence. As exemplified herein, mCRPC patients treated with PARPi+ARS, at the time point that of disease progression, the follow up blood draw CTCs did have high LST CTCs.

Disclosed (not claimed) is a method of identifying a cell type associated with response to abiraterone in a cancer patient, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) number of nucleoli; and (iv) optionally other features listed in Table 1; (c) classifying individual cells into CTC subtypes; and (d) identifying a biomarker CTC in the CTC subtypes, wherein identification of the biomarker CTC indicates a cell type associated with response in the patient to abiraterone. In some cases, the cells are classified with a CTC subtype classifier developed from unsupervised classification. In some cases, the biomarker CTC has the characteristics of a large nucleus, high nuclear entropy, and frequent nucleoli. In some cases, the method further comprises the step of isolating the CTCs from said sample. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of determining the presence or absence of a cell type, wherein the absence of the cell type is associated with response to enzalutamide in a cancer patient, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) number of nucleoli; and (iv)optionally other features listed in Table 1; (c) classifying individual cells into CTC subtypes; and (d) determining the presence or absence of a biomarker CTC in the CTC subtypes, wherein absence of the biomarker CTC indicates the absence of a cell type associated with response in the patient to enzalutamide. In some cases, the cells are classified with a CTC subtype classifier developed from unsupervised classification. In some cases, the biomarker CTC has the characteristics of a large nucleus, high nuclear entropy, and frequent nucleoli. In some cases, the method further comprises the step of isolating the CTCs from said sample. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of identifying a cancer patient for treatment with a drug, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) number of nucleoli; and (iv) optionally other features listed in Table 1; (c) classifying individual cells into CTC subtypes; and (d) determining the presence or absence of a biomarker CTC in the CTC subtypes, wherein presence of the biomarker CTC indicates to administer abiraterone to the cancer patient, or wherein absence of the biomarker CTC indicates to administer enzalutamide to the cancer patient. In some cases, the cells are classified with a CTC subtype classifier developed from unsupervised classification. In some cases, the biomarker CTC has the characteristics of a large nucleus, high nuclear entropy, and frequent nucleoli. In some cases, the method further comprises the step of isolating the CTCs from said sample. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC). In some cases, the method further comprises the step of administering abiraterone to the cancer patient. In some cases, the method further comprises the step of administering enzalutamide to the cancer patient.

The invention provides a method of predicting responsiveness in a hormon-refractory prostate cancer patient to treatment with abiraterone, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) number of nucleoli; and (iv) optionally other features listed in Table 1; (c) classifying individual cells into CTC subtypes; and (d) identifying a biomarker CTC in the CTC subtypes, wherein identification of the biomarker CTC predicts responsiveness of the cancer patient to treatment with abiraterone. In some embodiments, the cells are classified with a CTC subtype classifier developed from unsupervised classification. The biomarker CTC has the characteristics of a large nucleus, high nuclear entropy, and frequent nucleoli. In some embodiments, the method further comprises the step of isolating the CTCs from said sample. Prostate cancer which is hormone refractory, is also referred to as castration-resistant prostate cancer. In certain embodiments, the prostrate cancer is metastatic hormone resistant prostate cancer, also referred to as metastatic castration-resistant prostate cancer (mCRPC).

The present invention provides a method of predicting responsiveness in a hormon-refractory prostate cancer patient to treatment with enzalutamide, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) number of nucleoli; and (iv) optionally other features listed in Table 1; (c) classifying individual cells into CTC subtypes; and (d) determining the presence or absence of a biomarker CTC in the CTC subtypes, wherein absence of the biomarker CTC predicts responsiveness of the cancer patient to treatment with enzalutamide. In some embodiments, the cells are classified with a CTC subtype classifier developed from unsupervised classification. The biomarker CTC has the characteristics of a large nucleus, high nuclear entropy, and frequent nucleoli. In some embodiments, the method further comprises the step of isolating the CTCs from said sample. Prostate cancer which is hormone refractory, is also referred to as castration-resistant prostate cancer. In certain embodiments, the prostrate cancer is metastatic hormone resistant prostate cancer, also referred to as metastatic castration-resistant prostate cancer (mCRPC).

Disclosed (not claimed) a method of identifying a cell type associated with response to abiraterone in a cancer patient, comprising (a) performing a direct analysis comprising immunofluorescent staining and morphological characterization of nucleated cells in a blood sample obtained from the patient to identify and enumerate circulating tumor cells (CTC); (b) isolating the CTCs from the sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) number of nucleoli, and (vi) optionally one or more other features (parameters) listed in Table 1; and (d) identifying a biomarker CTC having the characteristics of frequent CNVs, a high number of breakpoints, a large nucleus, high nuclear entropy, and frequent nucleoli, wherein identification of the biomarker CTC indicates a cell type associated with response in the patient to abiraterone. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of determining the presence or absence of a cell type, wherein the absence of the cell type is associated with response to enzalutamide in a cancer patient, comprising (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) number of nucleoli; and (vi) optionally one or more other features (parameters) listed in Table 1; and (d) determining the presence or absence of a biomarker CTC having the characteristics of frequent CNVs, a high number of breakpoints, a large nucleus, high nuclear entropy, and frequent nucleoli, wherein absence of the biomarker CTC indicates the absence of a cell type associated with response in the patient to enzalutamide. In some embodiments, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of identifying a cancer patient for treatment with a drug, comprising (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) number of nucleoli; and (vi) optionally one or more other features (parameters) listed in Table 1; and (d) determining the presence or absence of a biomarker CTC having the characteristics of frequent CNVs, a high number of breakpoints, a large nucleus, high nuclear entropy, and frequent nucleoli, wherein presence of the biomarker CTC indicates to administer abiraterone to the cancer patient, or wherein absence of the biomarker CTC indicates to administer enzalutamide to the cancer patient. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC). In some cases, the method further comprises the step of administering abiraterone to the cancer patient. In some cases, the method further comprises the step of administering enzalutamide to the cancer patient.

Disclosed (not claimed) is a method of predicting responsiveness in a cancer patient to treatment with abiraterone, comprising: (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a genomic profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) number of nucleoli; and (vi) optionally one or more other features (parameters) listed in Table 1; and (d) identifying a biomarker CTC having the characteristics of frequent CNVs, a high number of breakpoints, a large nucleus, high nuclear entropy, and frequent nucleoli, wherein identification of the biomarker CTC predicts responsiveness of the cancer patient to treatment with abiraterone. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of predicting responsiveness in a cancer patient to treatment with enzalutamide, comprising (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) number of nucleoli; and (vi) optionally one or more other features (parameters) listed in Table 1; and (d) determining the presence or absence of a biomarker CTC having the characteristics of frequent CNVs, a high number of breakpoints, a large nucleus, high nuclear entropy, and frequent nucleoli, wherein absence of the biomarker CTC predicts responsiveness of the cancer patient to treatment with enzalutamide. In some cases, the cancer is prostate cancer. In some cases, the prostate cancer is hormone refractory, also referred to as castration-resistant prostate cancer. In certain cases, the prostrate cancer is metastatic hormone resistant prostate cancer. also referred to as metastatic castration-resistant prostate cancer (mCRPC).

Disclosed (not claimed) is a method of identifying a cell type associated with response to abiraterone in a cancer patient, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) size of the cytoplasm; and (iv) optionally other features listed in Table 1; (c) classifying individual cells into CTC subtypes; and (d) identifying a biomarker CTC in the CTC subtypes, wherein identification of the biomarker CTC indicates a cell type associated with response in the patient to abiraterone. In some cases, the cells are classified with a CTC subtype classifier developed from unsupervised classification. In some cases, the biomarker CTC has the characteristics of a large nucleus, high nuclear entropy, and large cytoplasm. In some cases, the method further comprises the step of isolating the CTCs from said sample. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of determining the presence or absence of a cell type, wherein the absence of the cell type is associated with response to enzalutamide in a cancer patient, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) size of the cytoplasm; and (iv)optionally other features listed in Table 1; (c) classifying individual cells into CTC subtypes; and (d) determining the presence or absence of a biomarker CTC in the CTC subtypes, wherein absence of the biomarker CTC indicates the absence of a cell type associated with response in the patient to enzalutamide. In some cases, the cells are classified with a CTC subtype classifier developed from unsupervised classification. In some cases, the biomarker CTC has the characteristics of a large nucleus, high nuclear entropy, and large cytoplasm. In some cases, the method further comprises the step of isolating the CTCs from said sample. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of identifying a cancer patient for treatment with a drug, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) size of the cytoplasm; and (iv) optionally other features listed in Table 1; (c) classifying individual cells into CTC subtypes; and (d) determining the presence or absence of a biomarker CTC in the CTC subtypes, wherein presence of the biomarker CTC indicates to administer abiraterone to the cancer patient, or wherein absence of the biomarker CTC indicates to administer enzalutamide to the cancer patient. In some cases, the cells are classified with a CTC subtype classifier developed from unsupervised classification. In some cases, the biomarker CTC has the characteristics of a large nucleus, high nuclear entropy, and large cytoplasm. In some cases, the method further comprises the step of isolating the CTCs from said sample. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC). In some cases, the method further comprises the step of administering abiraterone to the cancer patient. In some cases, the method further comprises the step of administering enzalutamide to the cancer patient.

The invention provides a method of predicting responsiveness in a hormon-refractory prostate cancer patient to treatment with abiraterone, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) size of the cytoplasm; and (iv) optionally other features listed in Table 1; (c) classifying individual cells into CTC subtypes; and (d) identifying a biomarker CTC in the CTC subtypes, wherein identification of the biomarker CTC predicts responsiveness of the cancer patient to treatment with abiraterone. In some embodiments, the cells are classified with a CTC subtype classifier developed from unsupervised classification. The biomarker CTC has the characteristics of a large nucleus, high nuclear entropy, and large cytoplasm. In some embodiments, the method further comprises the step of isolating the CTCs from said sample. Prostate cance which is hormone refractory, is also referred to as castration-resistant prostate cancer. In certain embodiments, the prostrate cancer is metastatic hormone resistant prostate cancer, also referred to as metastatic castration-resistant prostate cancer (mCRPC).

The present invention provides a method of predicting responsiveness in a hormon-refractory prostate cancer patient to treatment with enzalutamide, comprising (a) performing a direct analysis comprising immunofluorescent staining and morphological characterization of nucleated cells in a blood sample obtained from the patient to identify and enumerate circulating tumor cells (CTC); (b) individually characterizing digital pathology parameters to generate a profile for each of the CTCs, wherein the parameters comprise (i) size of the nucleus; (ii) nuclear entropy; (iii) size of the cytoplasm; and (iv) optionally other features listed in Table 1; (c) classifying individual cells into CTC subtypes; and (d) determining the presence or absence of a biomarker CTC in the CTC subtypes, wherein absence of the biomarker CTC predicts responsiveness of the cancer patient to treatment with enzalutamide. In some embodiments, the cells are classified with a CTC subtype classifier developed from unsupervised classification. The biomarker CTC has the characteristics of a large nucleus, high nuclear entropy, and large cytoplasm. In some embodiments, the method further comprises the step of isolating the CTCs from said sample. Prostate cance which is hormone refractory, is also referred to as castration-resistant prostate cancer. In certain embodiments, the prostrate cancer is metastatic hormone resistant prostate cancer, also referred to as metastatic castration-resistant prostate cancer (mCRPC).

Disclosed (not claimed) is a method of identifying a cell type associated with response to abiraterone in a cancer patient, comprising (a) performing a direct analysis comprising immunofluorescent staining and morphological characterization of nucleated cells in a blood sample obtained from the patient to identify and enumerate circulating tumor cells (CTC); (b) isolating the CTCs from the sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) size of the cytoplasm, and (vi) optionally one or more other features (parameters) listed in Table 1; and (d) identifying a biomarker CTC having the characteristics of frequent CNVs, a high number of breakpoints, a large nucleus, high nuclear entropy, and large cytoplasm, wherein identification of the biomarker CTC indicates a cell type associated with response in the patient to abiraterone. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of determining the presence or absence of a cell type, wherein the absence of the cell type is associated with response to enzalutamide in a cancer patient, comprising (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) size of the cytoplasm; and (vi) optionally one or more other features (parameters) listed in Table 1; and (d) determining the presence or absence of a biomarker CTC having the characteristics of frequent CNVs, a high number of breakpoints, a large nucleus, high nuclear entropy, and large cytoplasm, wherein absence of the biomarker CTC indicates the absence of a cell type associated with response in the patient to enzalutamide. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of identifying a cancer patient for treatment with a drug, comprising (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) size of the cytoplasm; and (vi) optionally one or more other features (parameters) listed in Table 1; and (d) determining the presence or absence of a biomarker CTC having the characteristics of frequent CNVs, a high number of breakpoints, a large nucleus, high nuclear entropy, and large cytoplasm, wherein presence of the biomarker CTC indicates to administer abiraterone to the cancer patient, or wherein absence of the biomarker CTC indicates to administer enzalutamide to the cancer patient. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC). In some cases, the method further comprises the step of administering abiraterone to the cancer patient. In some cases, the method further comprises the step of administering enzalutamide to the cancer patient.

Disclosed (not claimed) is a method of predicting responsiveness in a cancer patient to treatment with abiraterone, comprising: (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a genomic profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) size of the cytoplasm; and (vi) optionally one or more other features (parameters) listed in Table 1; and (d) identifying a biomarker CTC having the characteristics of frequent CNVs, a high number of breakpoints, a large nucleus, high nuclear entropy, and large cytoplasm, wherein identification of the biomarker CTC predicts responsiveness of the cancer patient to treatment with abiraterone. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

Disclosed (not claimed) is a method of predicting responsiveness in a cancer patient to treatment with enzalutamide, comprising (a) performing a direct analysis comprising immunofluorescent staining and morphological characterization of nucleated cells in a blood sample obtained from the patient to identify and enumerate circulating tumor cells (CTC); (b) isolating the CTCs from said sample; (c) individually characterizing parameters to generate a profile for each of the CTCs, wherein the parameters comprise one or more parameters selected from the group consisting of (i) copy number variation (CNV) signatures; (ii) number of breakpoints; (iii) size of the nucleus; (iv) nuclear entropy; (v) size of the cytoplasm; and (vi) optionally one or more other features (parameters) listed in Table 1; and (d) determining the presence or absence of a biomarker CTC having the characteristics of frequent CNVs, a high number of breakpoints, a large nucleus, high nuclear entropy, and large cytoplasm, wherein absence of the biomarker CTC predicts responsiveness of the cancer patient to treatment with enzalutamide. In some cases, the cancer is prostate cancer. In some cases, the prostate cancer is hormone refractory, also referred to as castration-resistant prostate cancer. In certain cases, the prostrate cancer is metastatic hormone resistant prostate cancer, also referred to as metastatic castration-resistant prostate cancer (mCRPC).

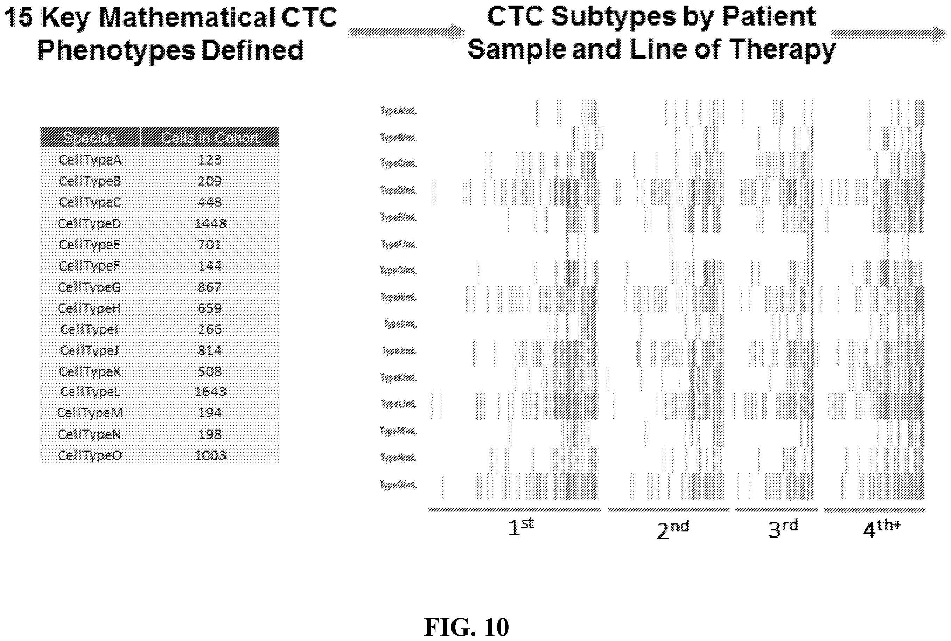

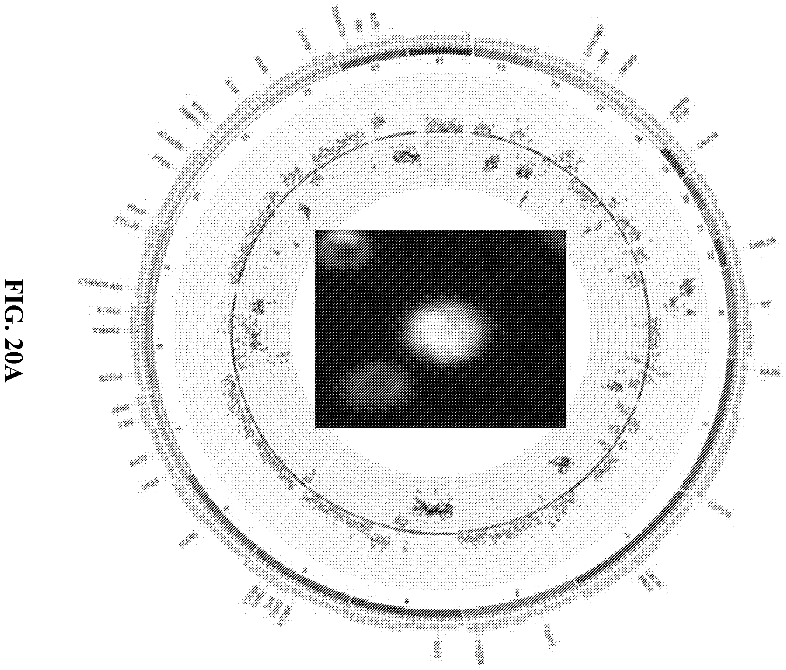

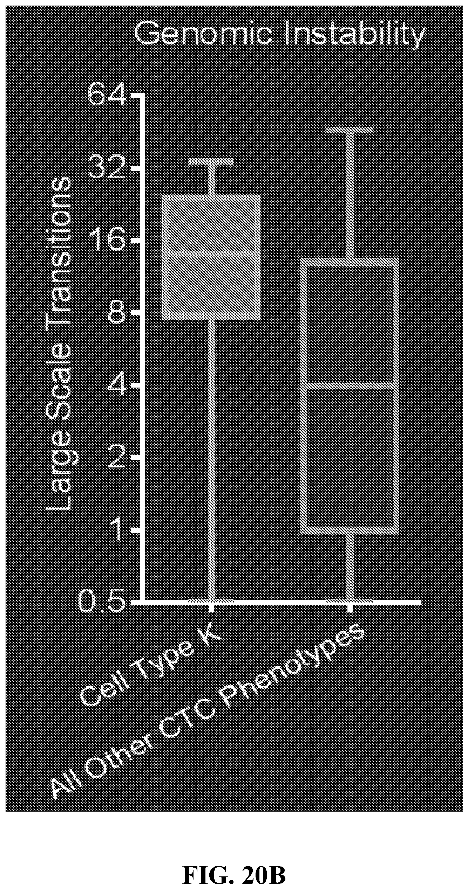

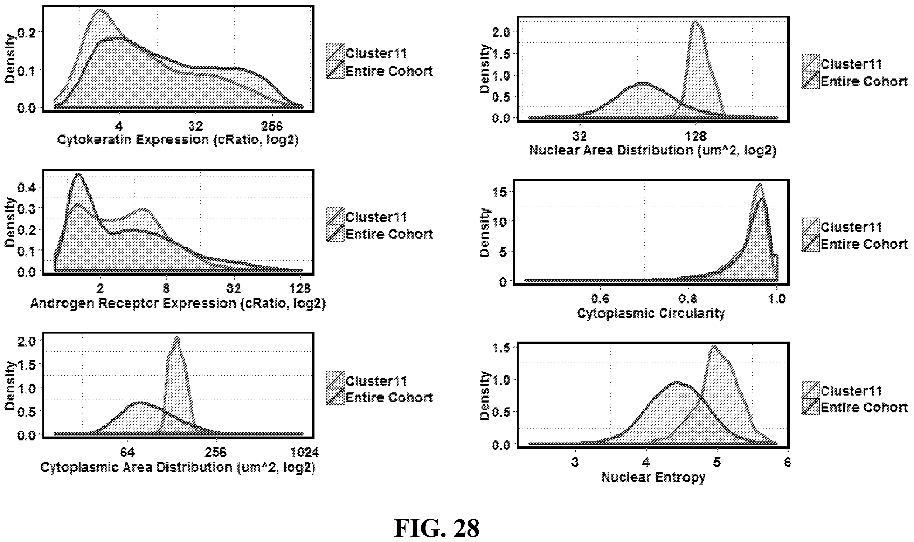

As disclosed herein, CTC cell type K was found to be a predictive biomarker for the effectiveness of treatment with abiraterone or enzalutamide. As disclosed herein, 15 phenotypic cell types can be determined by measuring phenotypic parameters, followed by running a classifier that predicts 15 cell types. The probability of the 15 cell types are assigned to each cell, and a specific cell type (e.g., K) with the highest probability is assigned to the cell. Phenotypic parameters include protein biomarkers and digital pathology features as listed in Table 1. As disclosed herein, cell type K can be identified by measuring phenotypic parameters and running classifier developed from unsupervised classification that are predictive of cell type K (see Figure 10). Such parameters include common digital pathology features, see Table 1. In particular, cell type K has the characteristics of frequent CNVs (see Figure 20A), a high number of breakpoints (see Figure 20B), a large nucleus, high nuclear entropy, large cytoplasm (see Figure 28), and/or frequent nucleoli.

Figure 28 shows exemplary phenotypic parameters of cell type K (cluster 11). For example, cell type K exhibits a large nucleus, which as shown in Figure 28 has a nuclear area ranging from 107 to 169 µm² with peak at 128 µm², than other cell types (ranging from 35 to 143 µm², peak at 63 µm²). Cell type K also exhibits a high nuclear entropy, which as shown in Figure 28 has a nuclear entropy ranging from 4.4 to 5.6 with peak at 5, than other cell types (ranging from 3.6 to 5.1, peak at 4.4). Cell type K also exhibits a higher cytoplasmic area, which as shown in Figure 28 has a cytoplasmic area ranging from 115 to 178 µm² with peak at 137 µm², than other cell types (ranging from 43 to 226 µm², peak at 70 µm²). As disclosed herein and described above, cell type K can be identified by measuring phenotypic parameters, such as those in Table 1 (e.g., protein biomarker features such as CK cRatio (protein expression), and AR cRatio (protein expression); digital pathology features such as nuclear area (µm²), cytoplasmic area (µm²), nuclear convex area (µm²), cytoplasmic convex area (µm²), nuclear major axis (µm), cytoplasmic major axis (µm), nuclear minor axis (µm), cytoplasmic minor axis (µm), nuclear circularity, cytoplasmic circularity, nuclear solidity, cytoplasmic solidity, nuclear entropy, nuclear to cytoplasmic convex area ratio, nucleoli, CK speckles, and nuclear speckles. The measured phenotypic parameters are analyzed by a classifier that utilizes the models and/or algorithms described herein to predict 15 cell types. Based on the classifications of the cell types, a determination is made as to whether a sample contains or does not contain cell type K.

In one example of how a sample from a patient is analyzed for the presence or absence of cell type K, take a scenario of one patient having 20 CTCs detected. The phenotypic parameters are measured for these 20 cells first, followed by running the classifier to assess the probability of each of these 20 cells being one of the 15 cell types. After computation, each cell will have 15 probabilities of being one of the 15 cell types. Then each cell is ranked by its 15 probabilities, and the cell is determined as one cell type with the highest probability. For example, 10 cells can be cell type D, 5 cells can be cell type F, 3 cells can be cell type K, and 2 cells can be cell type L (10+5+3+2=20). In this scenario, the patient has 3 cell type K cells. It is understood that such a scenario is merely illustrative and exemplary of how an embodiment of the invention can be applied to determine whether cell type K is present or absent from a sample.

As disclosed herein, the presence of cell type K correlated with a more favorable patient response to abiraterone. The absence of cell type K correlated with a more favorable patient response to enzalutamide. Thus, determining whether a sample of CTCs from a cancer patient contains cell type K is predictive of responsiveness to abiraterone or enzalutamide.

Disclosed herein (not claimed) is a method of determining an LST score based on phenotypic analysis of circulating tumor cells (CTCs) in a cancer patient comprising (a) performing a direct analysis comprising immunofluorescent staining and morphological characterization of nucleated cells in a blood sample obtained from the patient to identify and enumerate CTCs; (b) detecting the presence of multiple morphologic and protein expression features for each of said CTCs to identify CTC subtypes, and (c) determining an LST score for the cancer patient based on the frequency of one or more CTC subtypes. In some cases, the features are selected from the features set forth in Table 1. In some cases the features include nuclear to cytoplasmic (N/C) ratio, nuclear & cytoplasm circularity, nuclear entropy, CK expression and cases hormone receptor expression, for example, AR expression. In some cases the features include nuclear area, nuclear convex area, nuclear speckles, nuclear major axis, cytoplasm area, cytoplasm convex area, cytoplasm minor axis, AR expression, cytoplasm major axis. In some cases, the cancer is prostate cancer. In some cases, the prostate cancer is metastatic hormone resistant prostate cancer (mCRPC), also referred to as hormone refractory. In some cases, the immunofluorescent staining of nucleated cells comprises pan cytokeratin, cluster of differentiation (CD) 45 and diamidino-2-phenylindole (DAPI).

In some cases, a high LST score further predicts resistance to ARS therapy. In further cases, a high LST score predicts response and/or sensitivity to PARPi+ARS therapy. In additional cases, a high LST score predicts response to platinum-based agents treatment. In some cases, a high LST score detected in a follow up sample predicts disease progression, disease recurrence and/or acquired resistance. In patients that initially responded to ARS therapy, a high LST score in a follow up sample predicts acquired resistance and disease progression. In patients that initially responded to PARPi+ARS therapy, a high LST score in a follow up sample predicts disease recurrence and/or progression.

Disclosed herein (not claimed) is a method of detecting phenotypic heterogeneity of disease in a cancer patient comprising (a) performing a direct analysis comprising immunofluorescent staining and morphological characterization of nucleated cells in a blood sample obtained from the patient to identify and enumerate circulating tumor cells (CTC); (b) detecting the presence of multiple morphologic and protein expression features for each of said CTCs to identify CTC subtypes, and (c) determining phenotypic heterogeneity of disease in the cancer patient based on the number of said CTC subtypes. In some cases, the features are selected from the features set forth in Table 1. In some cases, high phenotypic heterogeneity identifies a patient resistant to androgen receptor targeted therapy. In some cases, high phenotypic heterogeneity, among CTCs, is not associated with resistance to taxane based chemotherapy. In some cases, the method further comprises detection of a CTC subtype characterized by a large nucleus, high nuclear entropy and frequent nucleoli. In a related cases, detection of a prevalence of the CTC subtype characterized by a large nucleus, high nuclear entropy and frequent nucleoli, wherein said prevalence is associated with poor outcome on both androgen receptor targeted therapy and taxane based chemotherapy.

Disclosed herein (not claimed) is a method of detecting heterogeneity of disease in a cancer patient comprising (a) performing a direct analysis comprising immunofluorescent staining and morphological characterization of nucleated cells in a blood sample obtained from the patient to identify and enumerate circulating tumor cells (CTC); (b) isolating the CTCs from the sample; (c) individually characterizing genomic parameters to generate a genomic profile for each of the CTCs, and (c) determining heterogeneity of disease in the cancer patient based on the profile. In some cases, the cancer is prostate cancer. In some cases, the prostate cancer is hormone refractory.

In some cases, the immunofluorescent staining of nucleated cells comprises pan cytokeratin, cluster of differentiation (CD) 45, diamidino-2-phenylindole (DAPI) and a hormone receptor, for example and without limitation, androgen receptor (AR), Estrogen Receptor (ER), Progesterone Receptor (PR), or human epidermal growth factor receptor 2 (HER2). One skilled in the art understands that various cancers, including prostate, ovarian, endometrial and breast cancer, have subtypes associated with particular hormone receptor expression and can select a hormone receptor based on the particular cancer.

In some cases, the immunofluorescent staining of nucleated cells comprises pan cytokeratin, cluster of differentiation (CD) 45, diamidino-2-phenylindole (DAPI) and androgen receptor (AR).

In some cases, the genomic parameters comprise copy number variation (CNV) signatures. In some cases, the CNV signatures comprise gene amplifications or deletions. In some cases, the gene amplifications comprise amplification of AR gene. In cases some embodiments, the deletions comprise loss of Phosphatase and tensin homolog gene (PTEN). In some cases, the CNV signatures comprise genes associated with androgen independent cell growth.

In some cases, the genomic parameters comprise genomic instability. In some cases, the genomic instability is characterized by measuring large scale transitions (LSTs). In some cases, the genomic instability is characterized bv measuring percent genome altered (PGA).

In some cases, determining heterogeneity of disease in the cancer patient based on the profile identifies novel mechanisms of disease.

In some cases, determining heterogeneity of disease in the cancer patient based on the profile predicts a positive response to a treatment.

In some cases, determining heterogeneity of disease in the cancer patient based on the profile predicts a resistance to a treatment.

In some cases, high heterogeneity identifies a patient resistant to androgen receptor targeted therapy.

In some cases, high diversity among CTCs is not associated with resistance to taxane based chemotherapy.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

As used herein, the term "providing" used in the context of a liquid biopsy sample is meant to encompass any and all means of obtaining the sample. The term encompasses all direct and indirect means that result in presence of the sample in the context of practicing the claimed methods The step of obtaining the sample is not part of the claimed methods though.

The term "patient," as used herein preferably refers to a human, but also encompasses other mammals. It is noted that, as used herein, the terms "organism," "individual," "subject," or "patient" are used as synonyms and interchangeably.

The cancer of the invention is hormone-refractory prostate cancer. Other examples of cancers are not part of the claimed invention. As used in the compositions and methods described herein, the term "cancer" refers to or describes the physiological condition in mammals that is typically characterized by unregulated cell growth. In one example, the cancer is an epithelial cancer. In one example, the cancer is prostate cancer. The cancer can include, without limitation, breast cancer, lung cancer, prostate cancer, colorectal cancer, brain cancer, esophageal cancer, stomach cancer, bladder cancer, pancreatic cancer, cervical cancer, head and neck cancer, ovarian cancer, melanoma, and multidrug resistant cancer, or subtypes and stages thereof. In some examples the cancer is an "early stage" cancer. In still another example, the cancer is a "late stage" cancer. The term "tumor," as used herein, refers to all neoplastic cell growth and proliferation, whether malignant or benign, and all pre-cancerous and cancerous cells and tissues. The cancer can be a lymphoproliferative cancer, for example, a precursor B lymphoblastic leukemia/lymphoblastic lymphoma, a B cell non-Hodgkin lymphomas of follicular origin, a Hodgkin lymphoma precursor T cell lymphoblastic leukemia/lymphoblastic lymphoma, a neoplasm of immature T cells, a neoplasm of peripheral, post-thymic T cells, a T cell prolymphocytic leukemia, a peripheral T cell lymphoma, an unspecified, anaplastic large cell lymphoma, an adult T cell leukemia/lymphoma, a chronic lymphocytic leukemia, a mantle cell lymphoma, a follicular lymphoma, a marginal zone lymphoma, a hairy cell leukemia, a diffuse large B cell lymphoma, a Burkitt lymphoma, a lymphoplasmacytic lymphoma, a precursor T lymphoblastic leukemia/lymphoblastic lymphoma, a T cell prolymphocytic leukemia, an angioimmunoblastic lymphoma, or a nodular lymphocyte predominant Hodgkin lymphoma.

As used herein, the term "circulating tumor cell" or "CTC" is meant to encompass any rare cell that is present in a biological sample and that is related to cancer. CTCs, which can be present as single cells or in clusters of CTCs, are often epithelial cells shed from solid tumors found in very low concentrations in the circulation of patients.

As used herein, a "traditional CTC" refers to a single CTC that is cytokeratin positive, CD45 negative, contains a DAPI nucleus, and is morphologically distinct from surrounding white blood cells.

As used herein, a "non-traditional CTC" refers to a CTC that differs from a traditional CTC in at least one characteristic.

In its broadest sense, a biological sample can be any sample that contains CTCs. The sample of the invention is blood. Other examples of samples are not part of the claimed invention. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulations that are distinguishable by methods well known in the art (*e.g.*, FACS, immunohistochemistry). For example, a blood sample can contain populations of non-nucleated cells, such as erythrocytes (*e.g.,* 4-5 million/µl) or platelets (150,000-400,000 cells/µl), and populations of nucleated cells such as WBCs (*e.g.,* 4,500 - 10,000 cells/µl), CECs or CTCs (circulating tumor cells; *e.g.,* 2-800 cells/ µl). WBCs may contain cellular subpopulations of, *e.g.,* neutrophils (2,500-8,000 cells/µl), lymphocytes (1,000-4,000 cells/µl), monocytes (100-700 cells/µl), eosinophils (50-500 cells/µl), basophils (25 - 100 cells/ µl) and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

In some cases the sample is a blood sample obtained from a healthy subject or a subject deemed to be at high risk for cancer or metastasis of existing cancer based on art known clinically established criteria including, for example, age, race, and family history. In some cases the blood sample is from a subject who has been diagnosed with cancer based on tissue or liquid biopsy and/or surgery or clinical grounds. In some cases, the blood sample is obtained from a subject showing a clinical manifestation of cancer and/or well known in the art or who presents with any of the known risk factors for a particular cancer. In some cases, the cancer is bladder cancer, for example, urothelial bladder cancer.

As used herein in the context of generating CTC data, the term direct analysis means that the CTCs are detected in the context of all surrounding nucleated cells present in the sample as opposed to after enrichment of the sample for CTCs prior to detection. In some cases, the methods comprise microscopy providing a field of view that includes both CTCs and at least 200 surrounding white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTC, including subtypes of CTCs described herein, that allows for identification of phenotypes and heterogeneity that cannot be achieved with other CTC detection methods and that enables the analysis of biomarkers in the context of the claimed methods.

The methods disclosed herein can further take encompass individual patient risk factors and imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. As described herein, the methods of the invention can encompass one or more pieces of imaging data. In the methods disclosed herein, one or more individual risk factors can be selected from the group consisting of age, race, family history. It is understood that one skilled in the art can select additional individual risk factors based on a variety of art known criteria. As described herein, the methods of the invention can encompass one or more individual risk factors. Accordingly, biomarkers can include imaging data, individual risk factors and CTC data. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.,* glycoproteins, ribonucleoproteins, lipoproteins) as well as portions or fragments of a biological molecule.

CTC data can include morphological, genetic, epigenetic features and immunofluorescent features. As will be understood by those skilled in the art, biomarkers can include a biological molecule, or a fragment of a biological molecule, the change and/or the detection of which can be correlated, individually or combined with other measurable features, with cancer. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1: 1,000 in a blood cell population, *e.g.,* an abundance of less than 1:5,000, 1:10,000, 1:30,000, 1:50:000, 1:100,000, 1:300,000, 1:500,000, or 1:1,000,000. In some embodiments, the a CTC has an abundance of 1:50:000 to 1:100,000 in the cell population.

The samples of this disclosure may be obtained by any means, including, *e.g.,* by means of solid tissue biopsy or fluid biopsy (*see, e.g.,* Marrinucci D. et al., 2012, Phys. Biol. 9 016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (*e.g.,* procedures for drawing and processing whole blood). In some cases, a blood sample is drawn into anticoagulent blood collection tubes (BCT), which may contain EDTA or Streck Cell-Free DNA^{™}. In other cases, a blood sample is drawn into CellSave^{®} tubes (Veridex). A blood sample may further be stored for up to 12 hours, 24 hours, 36 hours, 48 hours, or 60 hours before further processing.

In some cases, the methods of this disclosure comprise an initial step of obtaining a white blood cell (WBC) count for the blood sample. In certain cases, the WBC count may be obtained by using a HemoCue^{®} WBC device (Hemocue, Ängelholm, Sweden). In some cases, the WBC count is used to determine the amount of blood required to plate a consistent loading volume of nucleated cells per slide and to calculate back the equivalent of CTCs per blood volume.

In some cases, the methods of this disclosure comprise an initial step of lysing erythrocytes in the blood sample. In some cases, the erythrocytes are lysed, *e.g.,* by adding an ammonium chloride solution to the blood sample. In certain cases, a blood sample is subjected to centrifugation following erythrocyte lysis and nucleated cells are resuspended, *e.g.,* in a PBS solution.

In some cases, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some cases, the material is a film. In some cases the material is a glass slide. In certain cases, the method encompasses an initial step of depositing nucleated cells from the blood sample as a monolayer on a glass slide. The glass slide can be coated to allow maximal retention of live cells (*See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some cases, the methods of this disclosure comprise depositing about 3 million cells onto a glass slide. In additional cases, the methods of this disclosure comprise depositing between about 2 million and about 3 million cells onto the glass slide. In some cases, the glass slide and immobilized cellular samples are available for further processing or experimentation after the methods of this disclosure have been completed.

In some cases, the methods of this disclosure comprise an initial step of identifying nucleated cells in the non-enriched blood sample. In some cases, the nucleated cells are identified with a fluorescent stain. In certain cases, the fluorescent stain comprises a nucleic acid specific stain. In certain cases, the fluorescent stain is diamidino-2-phenylindole (DAPI). In some cases, immunofluorescent staining of nucleated cells comprises pan cytokeratin (CK), cluster of differentiation (CD) 45 and DAPI. In some cases further described herein, CTCs comprise distinct immunofluorescent staining from surrounding nucleated cells. In some cases, the distinct immunofluorescent staining of CTCs comprises DAPI (+), CK (+) and CD 45 (-). In some cases, the identification of CTCs further comprises comparing the intensity of pan cytokeratin fluorescent staining to surrounding nucleated cells. In some cases, the CTC data is generated by fluorescent scanning microscopy to detect immunofluorescent staining of nucleated cells in a blood sample. Marrinucci D. et al., 2012, Phys. Biol. 9 016003).

In particular cases, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further cases, the CTC data includes traditional CTCs also known as high definition CTCs (HD-CTCs). Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described. Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the invention can be practiced with any other biomarkers that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to generate CTC data, including microscopy based approaches, including fluorescence scanning microscopy (*see, e.g.,* Marrinucci D. et al., 2012, Phys. Biol. 9 016003), sequencing approaches, mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunoprecipitation, radioimmunoassay, dot blotting, and FACS. Immunoassay techniques and protocols are generally known to those skilled in the art ( Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998). Polymerase chain reaction (PCR) can be carried out generally as in PCR Protocols: A Guide to Methods and Applications, Academic Press, San Diego, Calif. (1990). Any method capable of determining a DNA copy number profile of a particular sample can be used for molecular profiling according to the invention provided the resolution is sufficient to identify the biomarkers of the invention. The skilled artisan is aware of and capable of using a number of different platforms for assessing whole genome copy number changes at a resolution sufficient to identify the copy number of the one or more biomarkers of the invention.

*In situ* hybridization assays are well known and are generally described in Angerer et al., Methods Enzymol. 152:649-660 (1987). In an in situ hybridization assay, cells, e.g., from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters. FISH (fluorescence in situ hybridization) uses fluorescent probes that bind to only those parts of a sequence with which they show a high degree of sequence similarity.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

Nucleic acid sequencing technologies are suitable methods for analysis of gene expression. The principle underlying these methods is that the number of times a cDNA sequence is detected in a sample is directly related to the relative expression of the RNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9): 1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

A person of skill in the art will further appreciate that the presence or absence of biomarkers may be detected using any class of marker-specific binding reagents known in the art, including, e.g., antibodies, aptamers, fusion proteins, such as fusion proteins including protein receptor or protein ligand components, or biomarker-specific small molecule binders. In some embodiments, the presence or absence of CK or CD45 is determined by an antibody. The skilled person will further appreciate that the presence or absence of biomarkers can be measured by evaluating a chromosome copy number change at a chromosome locus of a biomarker. Genomic biomarkers can be identified by any technique such as, for example, comparative genomic hybridization (CGH), or by single nucleotide polymorphism arrays (genotyping microarrays) of cell lines, such as cancer cells. A bioinformatics approach can identify regions of chromosomal aberrations that discriminate between cell line groups and that are indicative of the biomarker, using appropriate copy number thresholds for amplifications and deletions in addition to further analysis using techniques such as qPCR or in situ hybridization. Nucleic acid assay methods for detection of chromosomal DNA copy number changes include: (i) in situ hybridization assays to intact tissue or cellular samples, (ii) microarray hybridization assays to chromosomal DNA extracted from a tissue sample, and (iii) polymerase chain reaction (PCR) or other amplification assays to chromosomal DNA extracted from a tissue sample. Assays using synthetic analogs of nucleic acids, such as peptide nucleic acids, in any of these formats can also be used.

The biomarker may be detected through hybridization assays using detectably labeled nucleic acid-based probes, such as deoxyribonucleic acid (DNA) probes or protein nucleic acid (PNA) probes, or unlabeled primers which are designed/selected to hybridize to the specific designed chromosomal target. The unlabeled primers are used in amplification assays, such as by polymerase chain reaction (PCR), in which after primer binding, a polymerase amplifies the target nucleic acid sequence for subsequent detection. The detection probes used in PCR or other amplification assays are preferably fluorescent, and still more preferably, detection probes useful in "real-time PCR". Fluorescent labels are also preferred for use in situ hybridization but other detectable labels commonly used in hybridization techniques, e.g., enzymatic, chromogenic and isotopic labels, can also be used. Useful probe labeling techniques are described in Molecular Cytogenetics: Protocols and Applications, Y.-S. Fan, Ed., Chap. 2, "Labeling Fluorescence In Situ Hybridization Probes for Genomic Targets", L. Morrison et al., p. 21-40, Humana Press,.COPYRGT. 2002, incorporated herein by reference. In detection of the genomic biomarkers by microarray analysis, these probe labeling techniques are applied to label a chromosomal DNA extract from a patient sample, which is then hybridized to the microarray.

A biomarker protein may be detected though immunological means or other protein assays. Protein assay methods useful in the invention to measure biomarker levels may comprise (i) immunoassay methods involving binding of a labeled antibody or protein to the expressed biomarker, (ii) mass spectrometry methods to determine expressed biomarker, and (iii) proteomic based or "protein chip" assays for the expressed biomarker. Useful immunoassay methods include both solution phase assays conducted using any format known in the art, such as, but not limited to, an ELISA format, a sandwich format, a competitive inhibition format (including both forward or reverse competitive inhibition assays) or a fluorescence polarization format, and solid phase assays such as immunohistochemistry (referred to as "IHC").

The antibodies of this disclosure bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green^{™}, rhodamine, Texas red, tetrarhodimine isothiocynate (TRITC), Cy3, Cy5, Alexa Fluor^{®} 647, Alexa Fluor^{®} 555, Alexa Fluor^{®} 488), fluorescent markers (*e.g.,* green fluorescent protein (GFP), phycoerythrin, etc.), enzymes (*e.g.,* luciferase, horseradish peroxidase, alkaline phosphatase, etc.), nanoparticles, biotin, digoxigenin, metals, and the like.

For mass-sectrometry based analysis, differential tagging with isotopic reagents, *e.g.,* isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

In some cases, the biomarkers are immunofluorescent markers. In some cases, the immunofluorescent makers comprise a marker specific for epithelial cells In some cases, the immunofluorescent makers comprise a marker specific for white blood cells (WBCs). In some cases, one or more of the immunofluorescent markers comprise CD 45 and CK.

In some cases, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some cases, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some cases, CTCs comprise distinct morphological characteristics compared to surrounding nucleated cells. In some cases, the morphological characteristics comprise nucleus size, nucleus shape, cell size, cell shape, and/or nuclear to cytoplasmic (N/C) ratio. In some cases, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some embodiments, the method is performed by fluorescent scanning microscopy. In certain cases the microscopic method provides high-resolution images of CTCs and their surrounding WBCs (*see, e.g.,* Marrinucci D. et al., 2012, Phys. Biol. 9 016003)). In some cases, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some cases, microscopic data collection and analysis is conducted in an automated manner.

In some cases, a CTC data includes detecting one or more biomarkers, for example, CK and CD 45. A biomarker is considered "present" in a cell if it is detectable above the background noise of the respective detection method used (*e.g.,* 2-fold, 3-fold, 5-fold, or 10-fold higher than the background; *e.g.,* 2σ or 3σ over background). In some cases, a biomarker is considered "absent" if it is not detectable above the background noise of the detection method used (*e.g.,* <1.5-fold or <2.0-fold higher than the background signal; *e.g.,* <1.5σ or <2.0σ over background).

In some cases, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; e.g., 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.,* the marker is considered absent) if the cell's fluorescence signal for the respective marker is not significantly above the background signal (*e.g.,* <1.5-fold or <2.0-fold higher than the background signal; *e.g.,* <1.5σ or <2.0σ over background).

Typically, each microscopic field contains both CTCs and WBCs. In certain cases the microscopic field shows at least 1, 5, 10, 20, 50, or 100 CTCs. In certain cases, the microscopic field shows at least 10, 25, 50, 100, 250, 500, or 1,000 fold more WBCs than CTCs. In certain cases, the microscopic field comprises one or more CTCs or CTC clusters surrounded by at least 10, 50, 100, 150, 200, 250, 500, 1,000 or more WBCs.

In some cases of the methods described herein, generation of the CTC data comprises enumeration of CTCs that are present in the blood sample. In some cases, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some cases of methods described herein, generation of the CTC data comprises detecting distinct subtypes of CTCs, including non-traditional CTCs. In some cases, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular case, the methods described herein encompass detection of at least 1 CTC cluster/mL of blood.

In some cases, the disclosed methods encompass the use of a predictive model. In further cases, the disclosed methods methods encompass comparing a measurable feature with a reference feature. As those skilled in the art can appreciate, such comparison can be a direct comparison to the reference feature or an indirect comparison where the reference feature has been incorporated into the predictive model. In further embodiments, analyzing a measurable feature encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional cases, the determination is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, *e.g*. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some cases, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, e.g. log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider. In some embodiments, the method has a specificity of >60%, >70%, >80%, >90% or higher.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

The disclosure provides kits (not claimed) for the measurement of biomarker levels that comprise containers containing at least one labeled probe, protein, or antibody specific for binding to at least one of the expressed biomarkers in a sample. These kits may also include containers with other associated reagents for the assay. In some cases, a kit comprises containers containing a labeled monoclonal antibody or nucleic acid probe for binding to a biomarker and at least one calibrator composition. The kit can further comprise components necessary for detecting the detectable label (e.g., an enzyme or a substrate). The kit can also contain a control sample or a series of control samples which can be assayed and compared to the test sample. Each component of the kit can be enclosed within an individual container and all of the various containers can be within a single package, along with instructions for interpreting the results of the assays performed using the kit.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

The following examples are provided by way of illustration, not limitation.

### EXAMPLES

### Example 1

Sample evaluation for CTCs was performed as reported previously using the Epic Sciences Platform. Marrinucci et al. Phys Biol 9:016003, 2012. The Epic CTC collection and detection process, which flows as follows: (1) Blood lysed, nucleated cells from blood sample placed onto slides; (2) Slides stored in -80C biorepository; (3) Slides stained with CK, CD45, DAPI and AR; (4) Slides scanned; (5) Multi-parametric digital pathology algorithms run, and (6) Software and human reader confirmation of CTCs & quantitation of biomarker expression. During the subsequent CTC recovery and genomic profiling workflow, individual cells were isolated, subjected to Whole Genome Amplification, and NGS library preparation. Sequencing was performed on an Illumina NextSeq 500.

Blood samples underwent hemolysis, centrifugation, re-suspension and plating onto slides, followed by -80⁰C storage. Prior to analysis, slides were thawed, labeled by immunofluorescence (pan cytokeratin, CD45, DAPI) and imaged by automated fluoroscopy then manual validation by a pathologist-trained technician (MSL). Marrinucci et al. Phys Biol 9:016003, 2012. DAPI (+), CK (+) and CD45 (-) intensities were categorized as features during CTC enumeration as previously described.

More specifically, peripheral blood sample was collected in Cell-free DNA BCT (Streck, Omaha, NE, USA) and shipped immediately to Epic Sciences (San Diego, CA, USA) at ambient temperature. Upon receipt, red blood cells were lysed and nucleated cells were dispensed onto glass microscope slides as previously described (Marrinucci et al. Hum Pathol 38(3): 514-519 (2007); Marrinucci et al. Arch Pathol Lab Med 133(9): 1468-1471 (2009); Mikolajczyk et al. J Oncol 2011: 252361. (2011); Marrinucci et al. Phys Biol 9(1): 016003 (2012); Werner et al. J Circ Biomark 4: 3 (2015)) and stored at -80 °C until staining. The millilitre equivalent of blood plated per slide was calculated based upon the sample's white blood cell count and the volume of post-RBC lysis cell suspension used. Circulating tumour cells were identified by immunofluorescence, as described (Marrinucci et al, 2007, *supra;* Marrinucci et al, 2009,*supra;* Mikolajczyk et al, 2011, *supra;* Marrinucci et al, 2012, *supra;* Werner et al, 2015, *supra).* During the subsequent CTC recovery and genomic profiling workflow, individual cells were isolated, subjected to Whole Genome Amplification, and NGS library preparation. Sequencing was performed on an Illumina NextSeq 500.

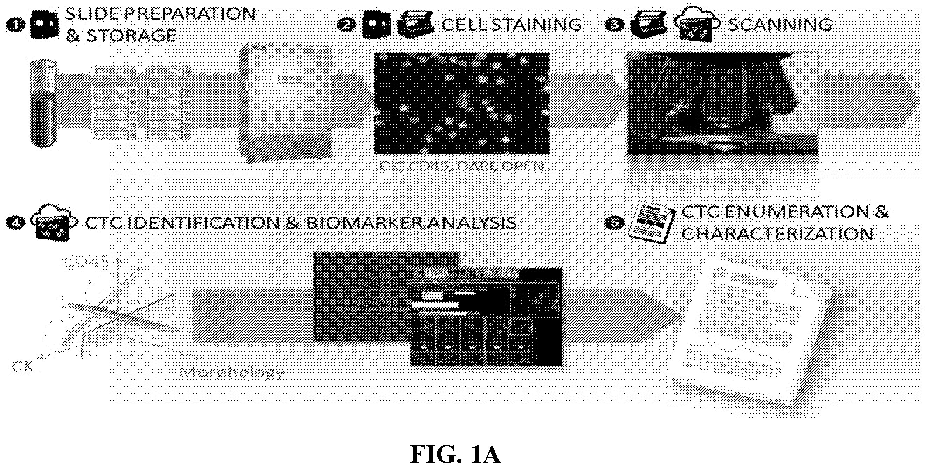

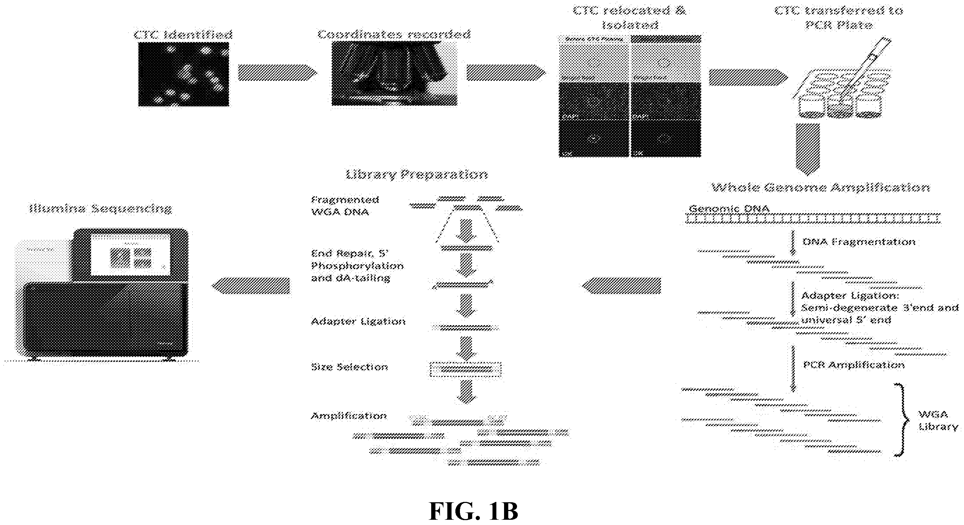

Figures 1 through 4 and the corresponding brief descriptions of the drawings describe further experimental details.

Example 2. Single CTC Characterization Identifies Phenotypic and Genomic Heterogeneity as a Mechanism of Resistance to Androgen Receptor Signaling Directed Therapies (AR Tx) in mCRPC Patients

Tumor heterogeneity (diversity) has been proposed as a biomarker of sensitivity. This example demonstrates analysis of heterogeneity in CTCs on a cell by cell basis to as a predictive biomarker of sensitivity at decision points in management aiming to better sequence available therapies.

An initial focus was to characterize CTC's at phenotypic (facial recognition) or cellular level, including variations in morphology and protein expression of cells that emerge from a single clone (lineage switching or plasticity), for example, AR+ → AR- neuroendocrine with TMPRSS2-ERG fusion.

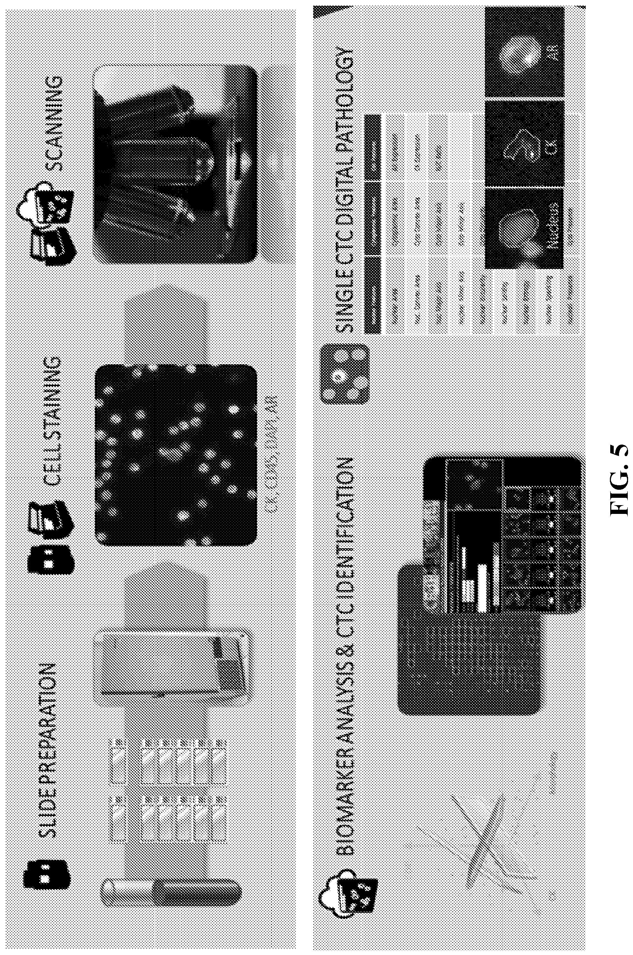

CTCs were isolated using a "no cell selection" platform and analyzed at the single cell level by morphology/protein chemistry (Facial Recognition) (Figure 5). No Cell Selection enables characterization of any rare cell type: inclusive of CK-, small, apoptotic and CTC clusters.

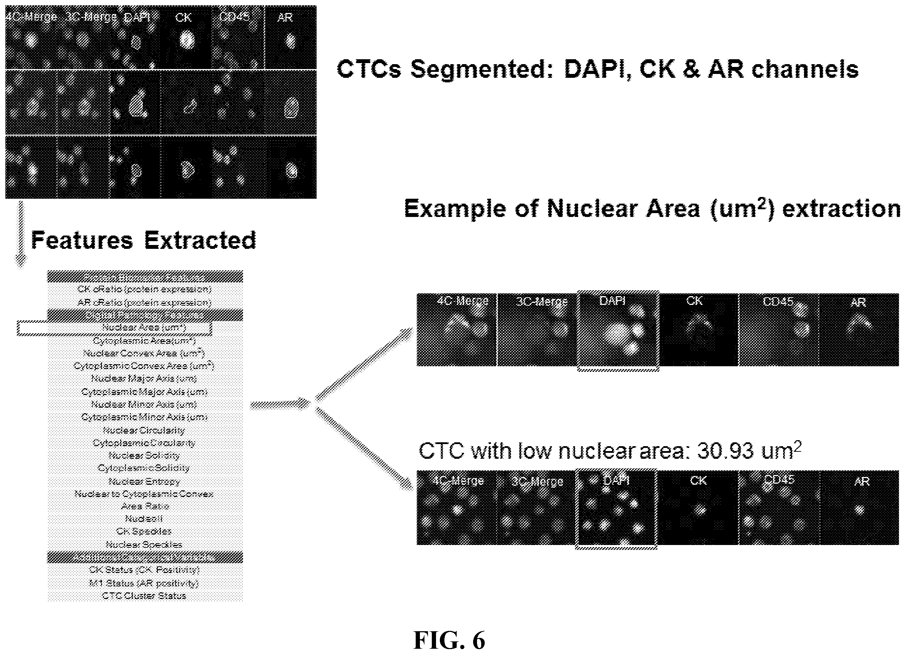

Following protein and morphological features of CTCs, a series of individual cell features were measured on each CTC identified in a patient sample, including nuclear area as well as other features set forth in Table 1 (Figure 6).

**Table 1. Protein Biomarker and Digital Pathology Features**

| **PROTEIN BIOMARKER FEATURES** |
|---|
| CK cRatio (protein expression) |
| AR cRatio (protein expression) |

| **DIGITAL PATHOLOGY FEATURES** |
|---|
| Nuclear Area (um²) |
| Cytoplasmic Area(um²) |
| Nuclear Convex Area (um²) |
| Cytoplasmic Convex Area (um²) |
| Nuclear Major Axis (um) |
| Cytoplasmic Major Axis (um) |
| Nuclear Minor Axis (um) |
| Cytoplasmic Minor Axis (um) |
| Nuclear Circularity |
| Cytoplasmic Circularity |
| Nuclear Solidity |
| Cytoplasmic Solidity |
| Nuclear Entropy |
| Nuclear to Cytoplasmic Convex Area Ratio |
| Nucleoli |
| CK Speckles |
| Nuclear Speckles |

| **ADDITIONAL CATEGORICAL VARIABLES** |
|---|
| CK Status (CK Positivity) |
| M1 Status (AR positivity) |
| Cluster Status |

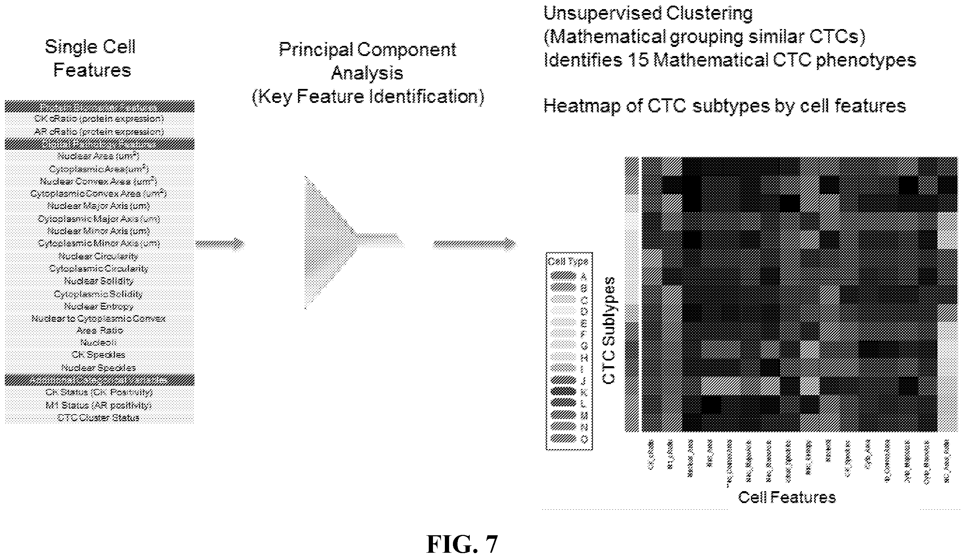

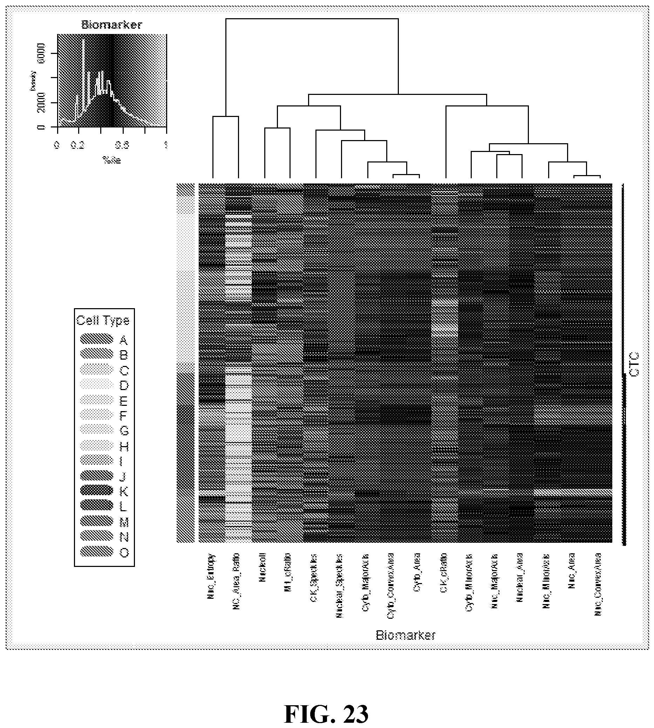

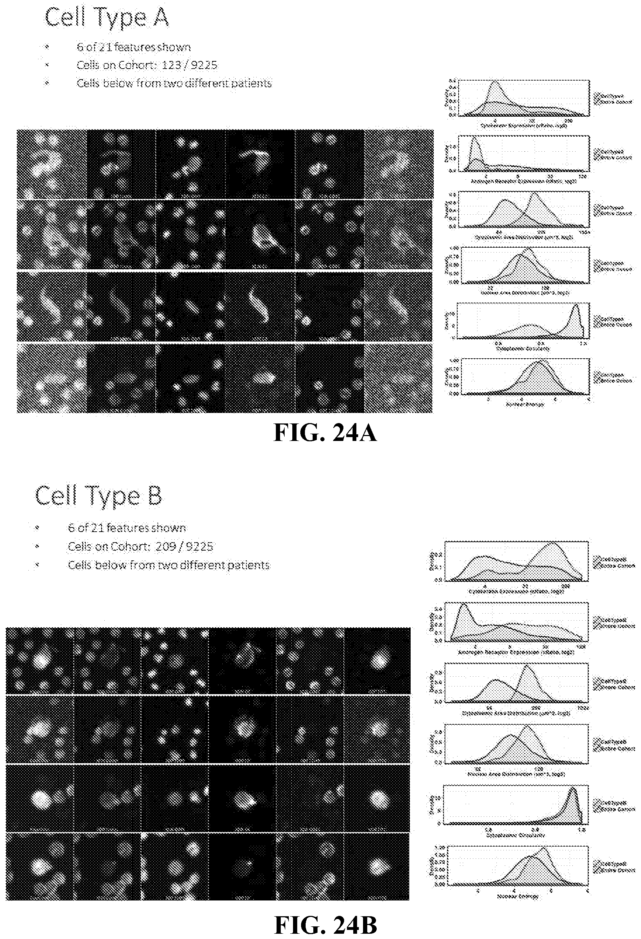

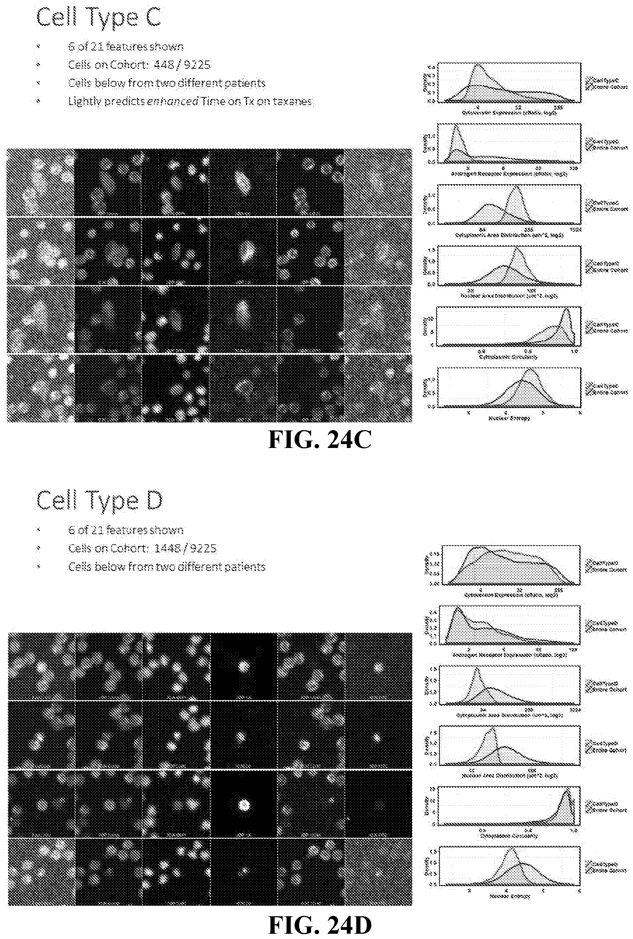

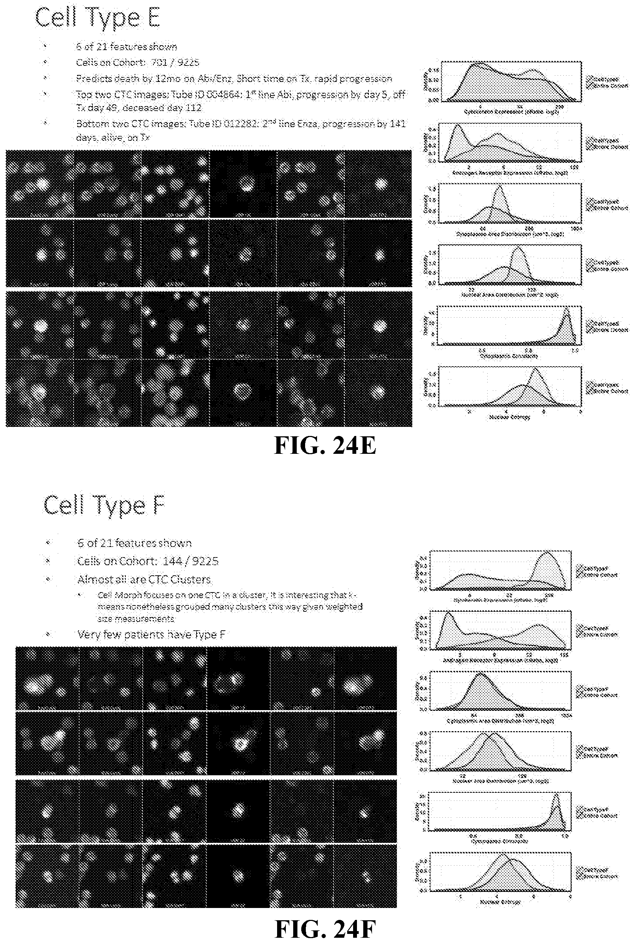

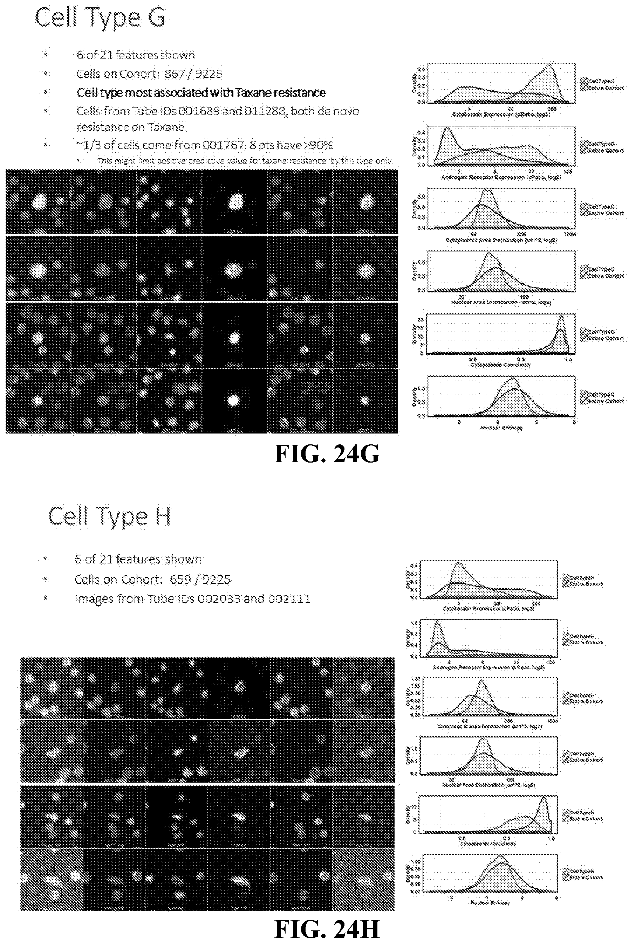

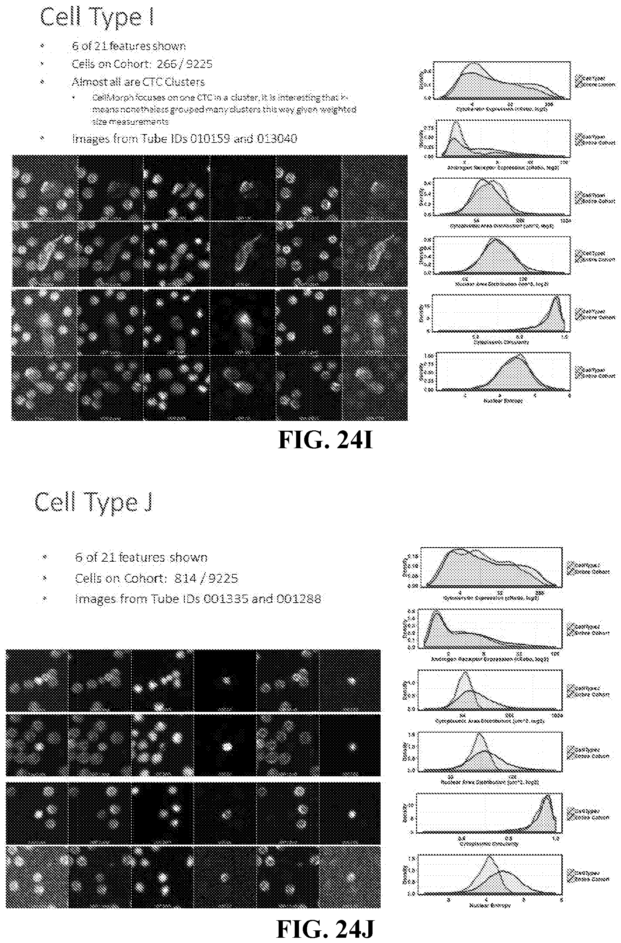

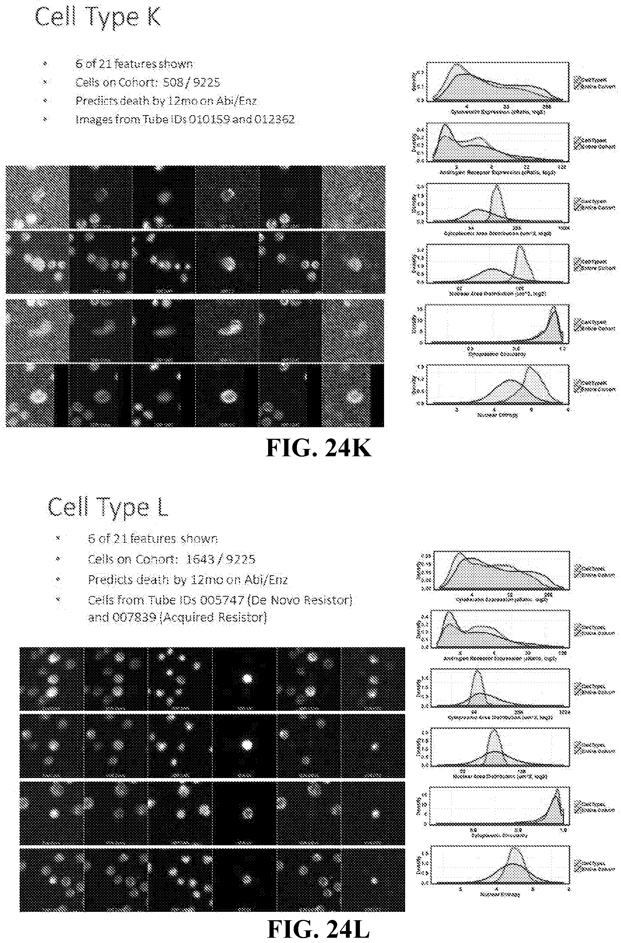

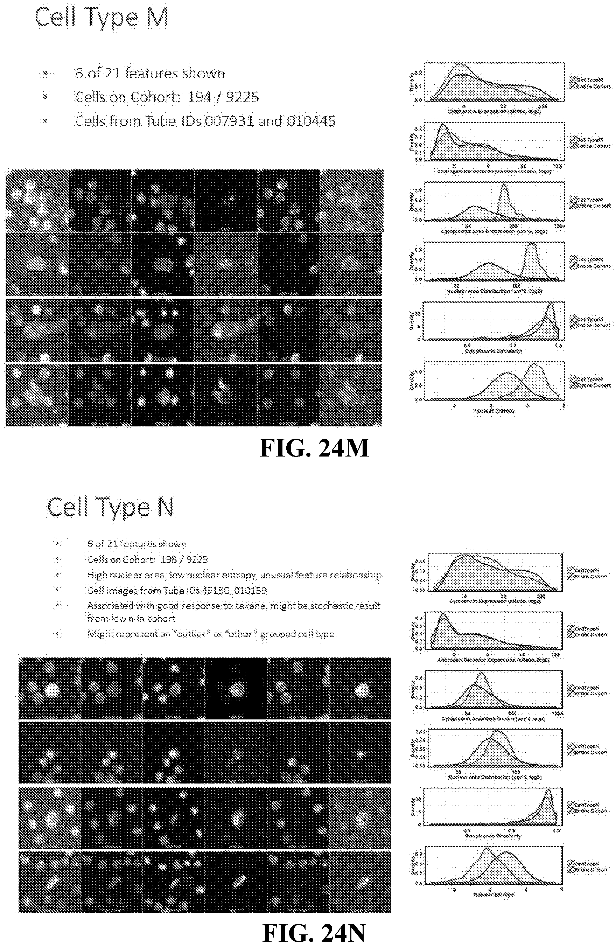

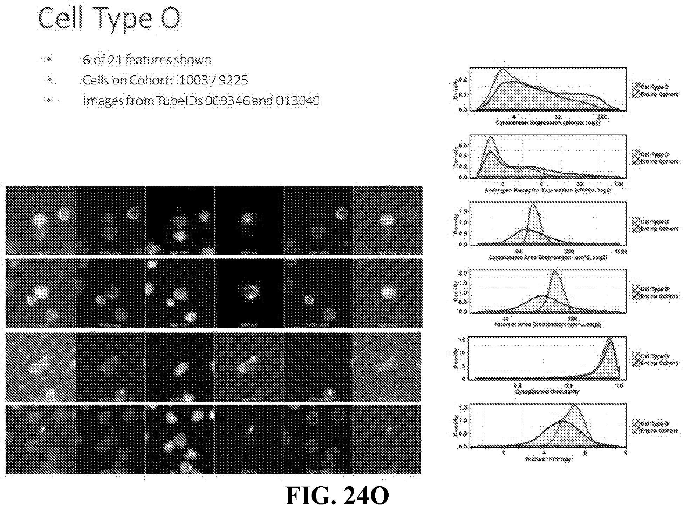

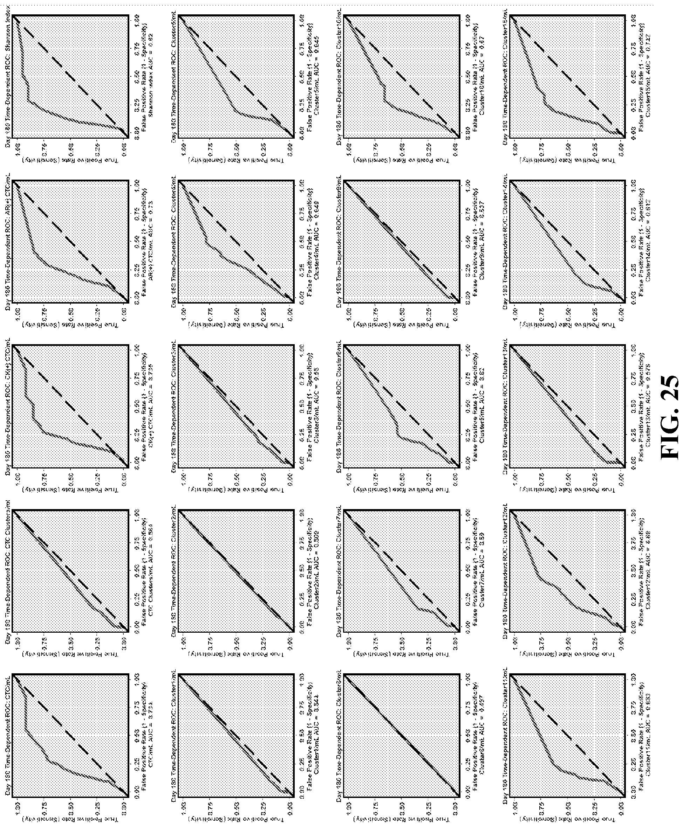

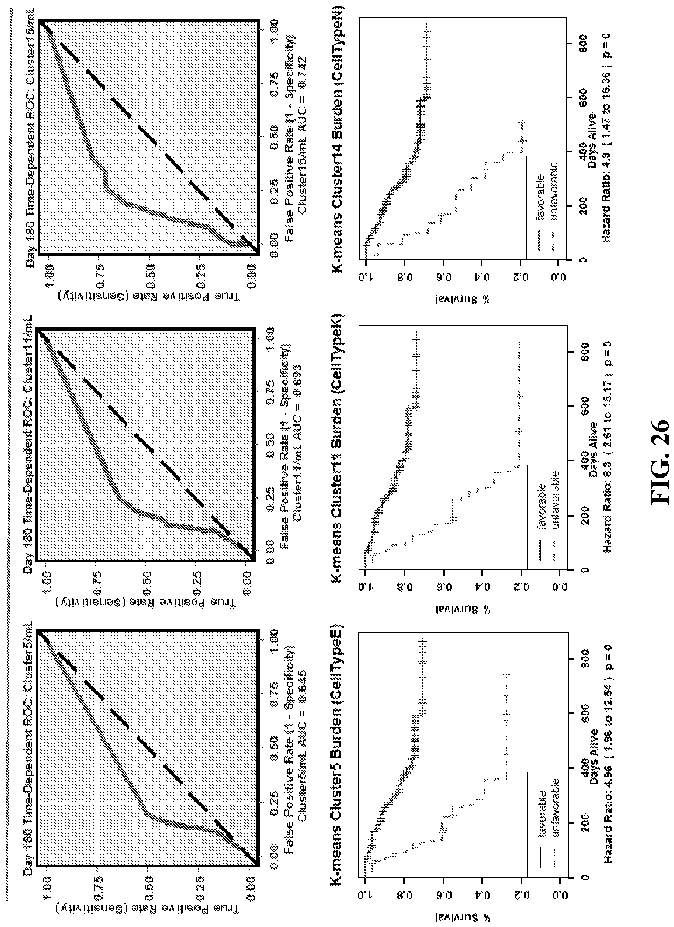

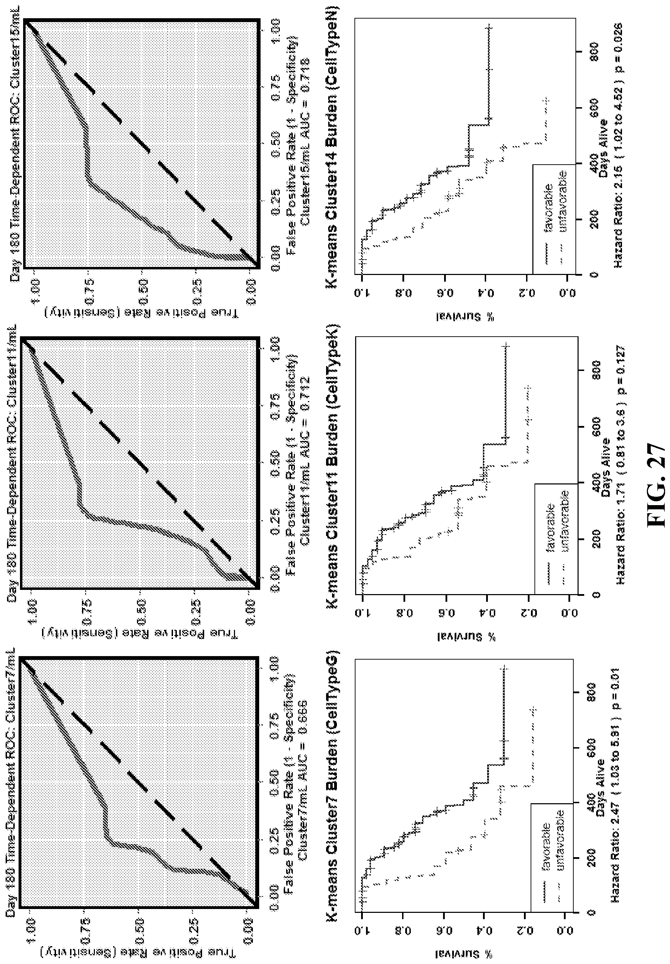

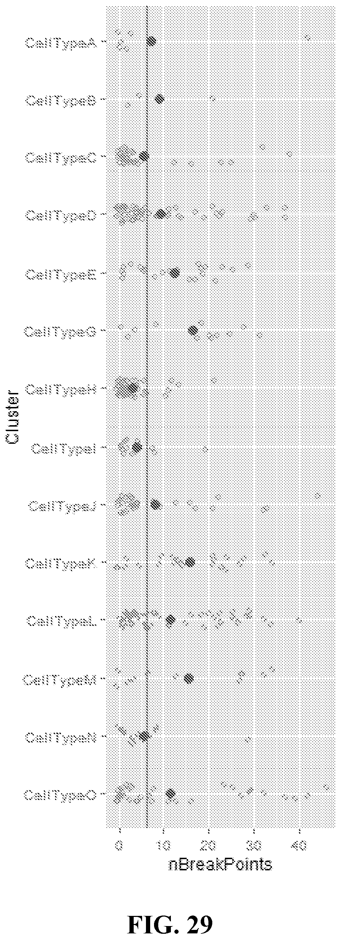

Twenty protein and morphology features were recorded individually, analogous to what is done with gene expression and unsupervised analysis of the >9000 CTCs was performed, where principal components, or key features were determined and then clustered (Figure 7). This led to mathematical groupings which defined 15 distinct CTC phenotypes (CTC subtypes A-O). Figure 7 shows a heat map on the right, where the 15 cell types are defined by the colors on the y axis, and the individual features on the x axis. Red reflects features on the low end of dynamic range (i.e. small nuclear area), while green reflects features on the high end of the dynamic range (i.e. large nuclear area) (Figure 7). Figure 23 also shows a heatmap depicting the 15 mathematical CTC phenotypic subtypes were identified using unsupervised analysis based on CTC protein and morphological features. Figure 24, panels A-O depict selected features of the 15 cell types. Certain CTC phenotypic subtypes prognosticates patient survival. Figure 25 shows the prediction of death by 180 days on ARS (n = 150 samples) by CTC enumeration and 15 CTC phenotypic subtypes. Good prognosticators include cell type E (cluster 5), K (cluster 11), and O (cluster 15). As depicted in Figure 26, some CTC phenotypic subtypes (cell type E, K and N) predicts mCRPC patient response to AR targeted therapy. Figure 27 depicts CTC phenotypic subtypes (cell type G, K and N) that predict response to taxane therapy. Twenty protein and morphology features were recorded individually, analogous to what is done with gene expression and unsupervised analysis of the >9000 CTCs was performed, where principal components, or key features were determined and then clustered (Figure 7). This led to mathematical groupings which defined 15 distinct CTC phenotypes. Figure 7 shows a heat map on the right, where the 15 cell types are defined by the colors on the y axis, and the individual features on the x axis. Red reflects features on the low end of dynamic range (i.e. small nuclear area), while green reflects features on the high end of the dynamic range (i.e. large nuclear area) (Figure 7). Figure 23 also shows a heatmap depicting the 15 mathematical CTC phenotypic subtypes were identified using unsupervised analysis based on CTC protein and morphological features. Figure 24, panels A-O depict selected features of the 15 cell types. Certain CTC phenotypic subtypes prognosticates patient survival. Figure 25 shows the prediction of death by 180 days on ARS (n = 150 samples) by CTC enumeration and 15 CTC phenotypic subtypes. Good prognosticators include cell type E (cluster 5), K (cluster 11), and O (cluster 15). As depicted in Figure 26, some CTC phenotypic subtypes (cell type E, K and N) predicts mCRPC patient response to AR targeted therapy. Figure 27 depicts CTC phenotypic subtypes (cell type G, K and N) that predict response to taxane therapy. Each cell types have unique morphological patterns. For example, as shown in Figure 28, cluster 11 (cell type K) has large nucleus, high nuclear entropy and frequent nucleoli. Also as shown in Figure 28, cell type K has a large cytoplasm. Multiple cell types (cell type G, K, and M) are predictive of genomic instability (LST) (Figure 29). These particular subtypes, given the increased genomic instability, may be sensitive to DNA damaging drugs, such as platinum based chemotherapies (i.e. carboplatin, cisplatin), or targeted therapeutics which target homologous recombination deficiencies, including PARP inhibitors, DNA-PK inhibitors and therapeutics targeting the ATM pathway.

Classifiers for classifying CTCs into 15 mathematical CTC phenotypic subtypes were derived from the unsupervised clustering (see above). Classifier can be applied to any mCRPC patient CTCs which have the same twenty protein and morphology features, and each CTC can be assigned as one of the 15 phenotypic subtypes (A- O, including cell type K) based on the maximum likelihood.

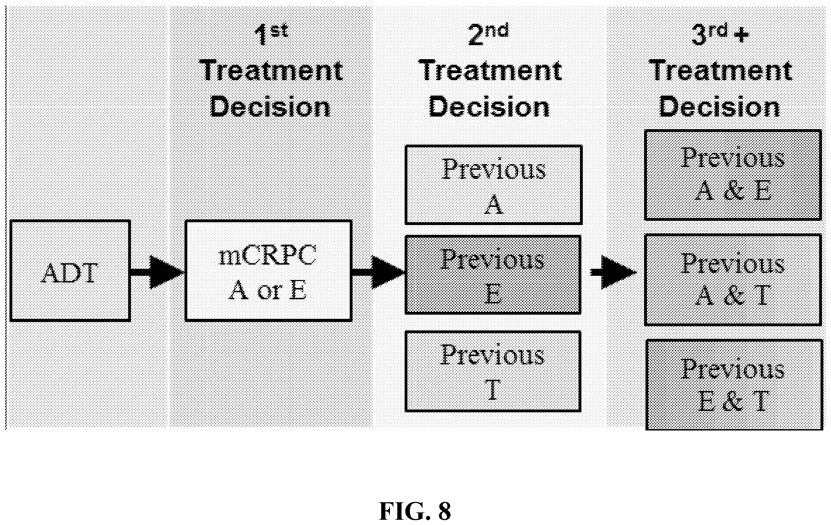

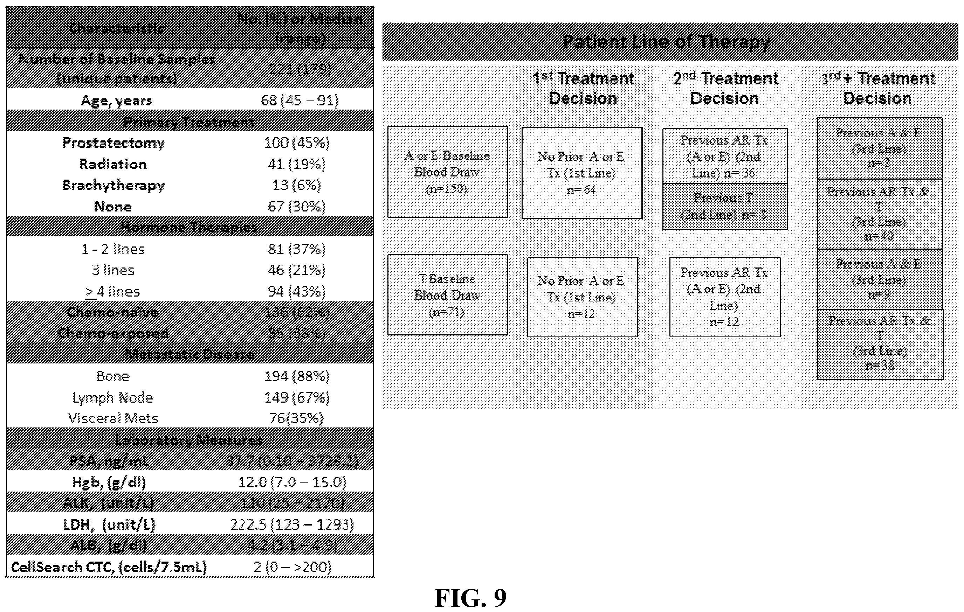

Phlebotomy samples were obtained at a Decision Point in management: therapy was chosen by the treating physician. Standard of care collection from 221 mCRPC patients at decision points. Baseline blood draw prior to A, E or T. Followed by PSA, time on drug, radiographic progression free (rPFS) & overall survival (OS) . 9225 CTCs identified and characterized phenotypically. 741 CTCs from 31 patients were studied by whole genome CNV for clonality and gene amplifications/deletions. Patients were ranked based on how heterogeneous or diverse the cells were at each decision point. (Figure 8). Figure 9 shows the demographics of the mCRPC population. The frequencies of the 15 different phenotypic CTC classes differed by line of therapy and were more heterogeneous over time (Figure 10). In Figure 10 red represents prevalence of a cell type that is overrepresented or which is more diverse. Each column is a patient, such that columns with many vertical red sections have higher phenotypic heterogeneity.

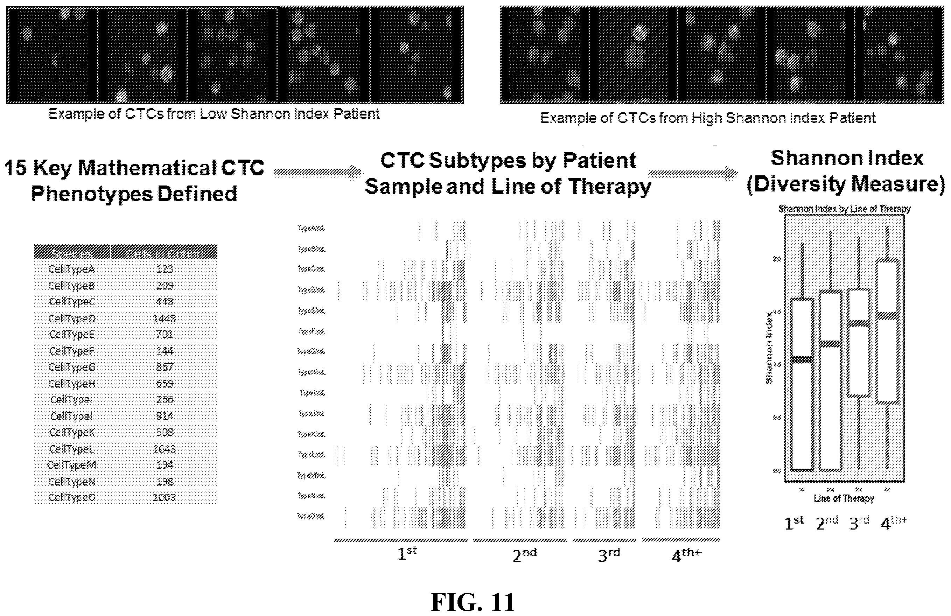

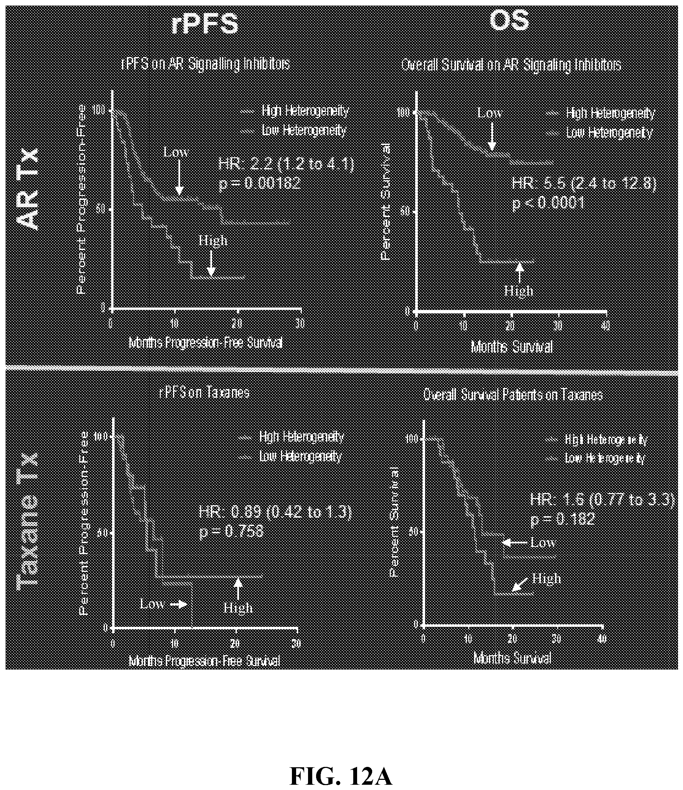

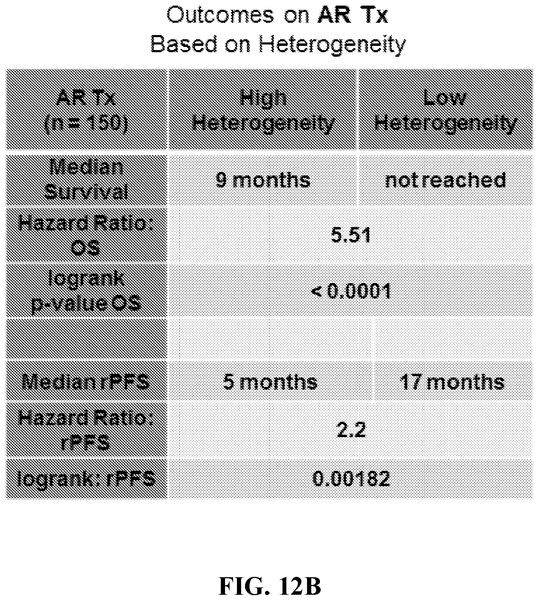

For each patient sample, the number of different Cell Types observed is counted, and CTC heterogeneity is quantified by calculating a Shannon Index. The Shannon Index is widely used in ecology to quantify the diverseness of ecosystems, based on the number of different species present in an ecosystem. The Shannon Index increases in value when the number of different species present in the ecosystem increases or the evenness increases (i.e. when each species has a similar number of entities present in the ecosystem). The Shannon Index is maximized when all species are present and they are present in equal numbers, and minimized when only 1 species is present. Therefore, low Shannon Index values indicate patients with low heterogeneity due to uniformity of CTCs found in the sample, and high Shannon Index values indicate patients with high heterogeneity due to having all types of CTCs present. As shown in Figure 11, the higher Shannon Indexes showed greater diversity (heterogeneity) by line of therapy, notably with the increase in the median, and fewer lower index scores in the 3^{rd} and 4^{th} line of therapy. High CTC phenotypic heterogeneity predicts shorter progression and survival times on AR therapy but not taxane therapy (Figure 12 A). Figure 12 B shows outcomes on AR Tx based on heterogeneity.

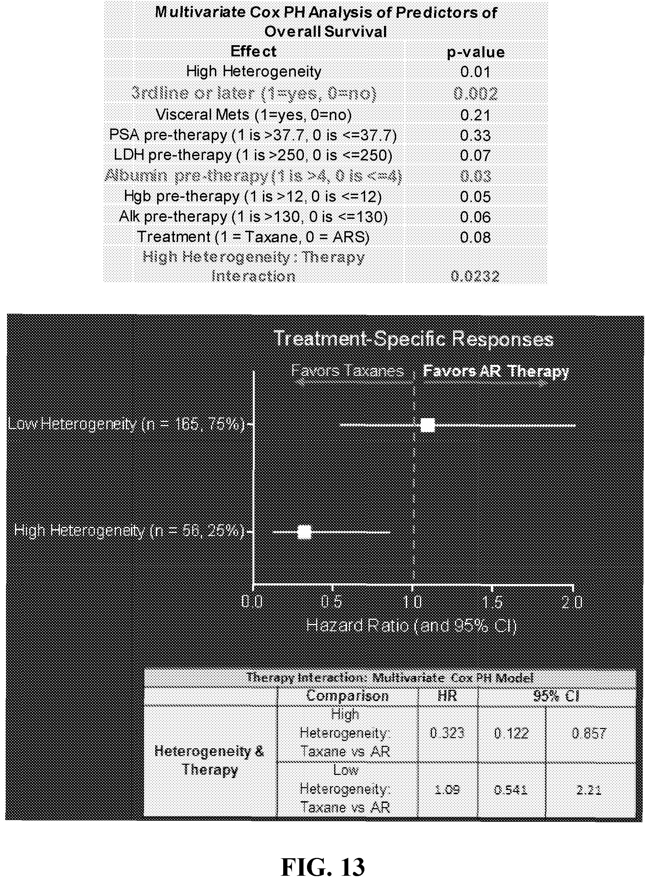

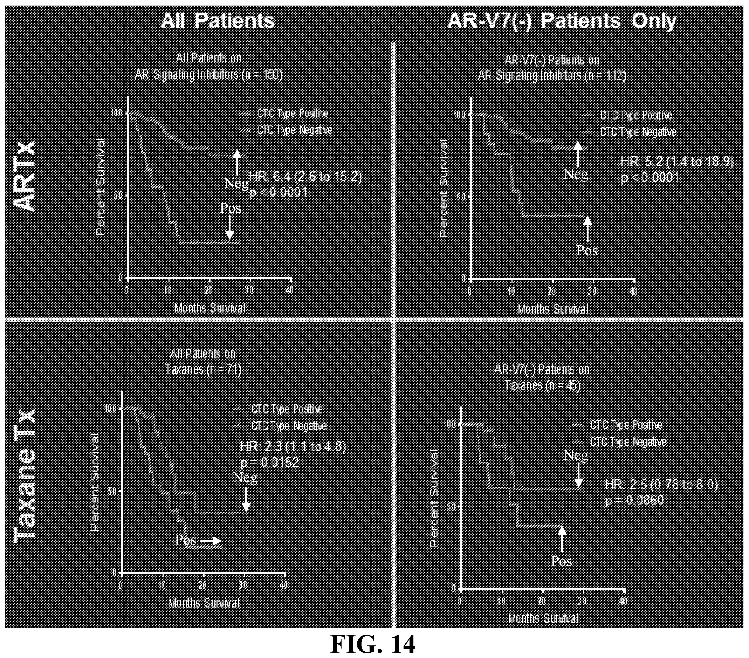

High CTC phenotypic heterogeneity predicts a better outcome with a Taxane over AR Tx in a multivariate model. A range of factors previously shown to be prognostic for survival were studied in univariate and multivariate analysis - only the multivariate is shown (Figure 13). High heterogeneity predicted for sensitivity to taxanes over AR therapies (Figure 13). Figure 14 shows the prevalence of a CTC subtype (Type K) predicts poor outcome on both ARTx and Taxanes independent of AR status. One particular mathematically defined cell type, type K had a large nucleus, a wide range of nuclear sizes and prominent nuclei - was associated with resistance to both classes of drugs.

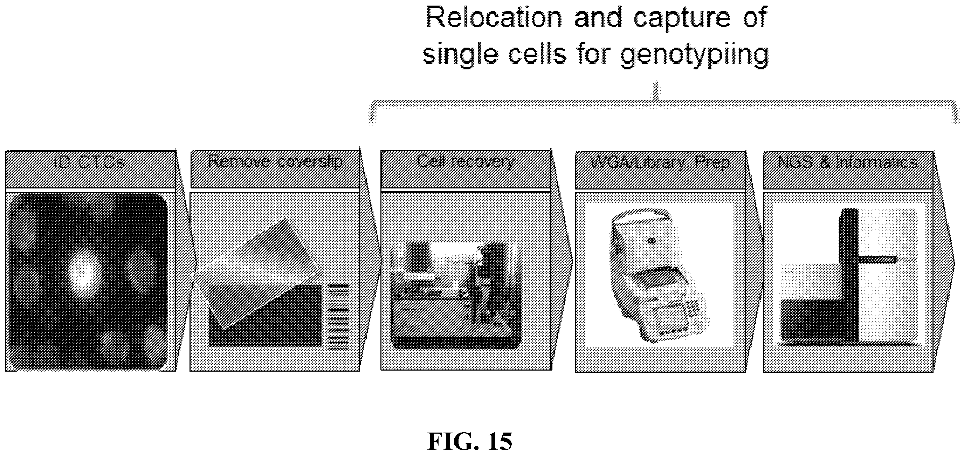

Recognizing that available therapies do not eliminate "all cells" within a tumor, the genotypic heterogeneity (single regions in a tumor with distinct mutational profiles evolving from a single initiating trunk lesion) of the CTCs in a patient sample was examined. After a CTC is phenotypically measured, the coverslip is removed and the individual CTC is aspirated and put into an individual tube. The CTCs are amplified and prepared for sequencing (Figure 15). Following sequencing informatics were performed to assess clonality and amplification/deletions (Figure 15).

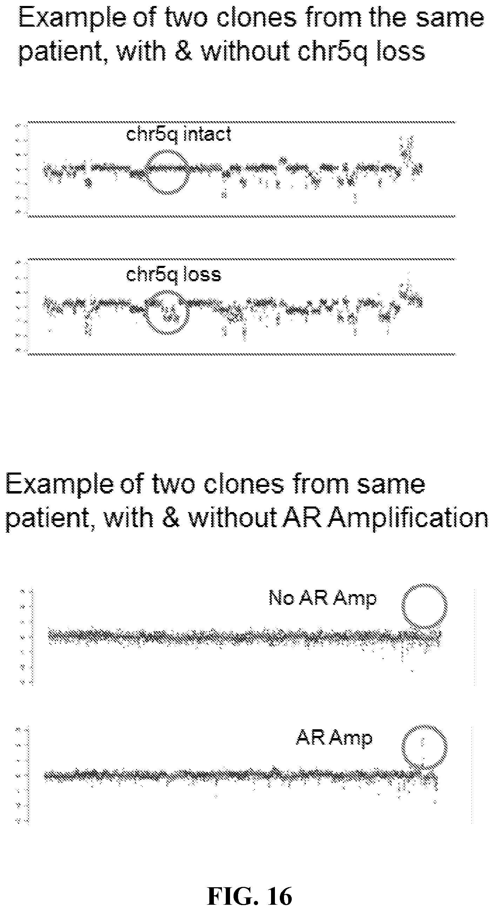

Single Cell CTC Sequencing Informs of Clonal Diversity and Phylogenetic Disease Lineage. Each patient sample was analyzed separately. Single CTC genomic CNV plots were curated individually versus other CTCs in patient sample. Clonality was characterized based on large genomic variations and focal amplifications or deletion of known driver alterations in at least 2 CTCs, for example, two cells from same patient with or without a loss of chromosome 5q or two clones from a patient with and without AR amplification (Figure 16).

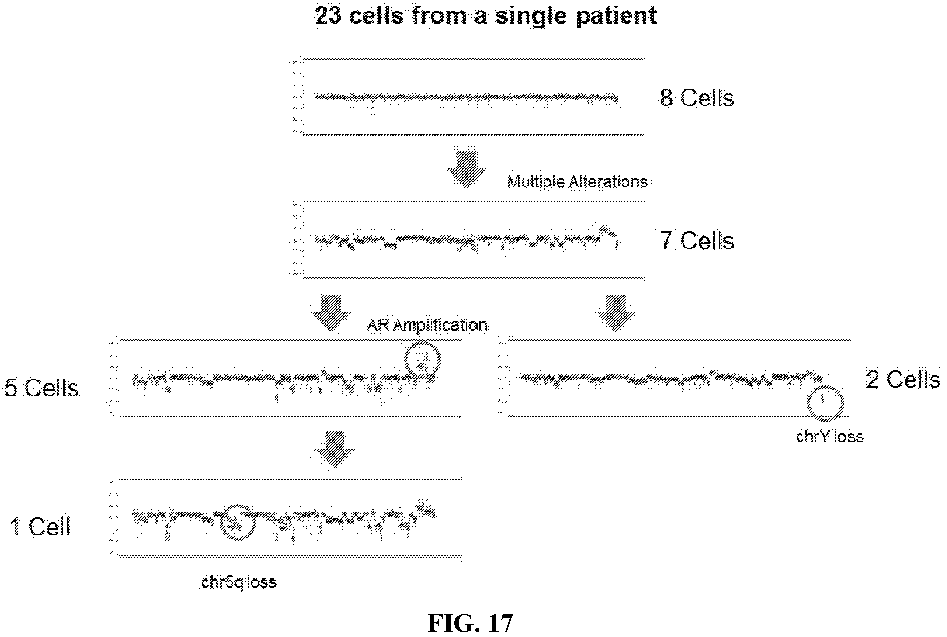

Single CTC CNV profiles inform clonal diversity and phylogenetic disease lineage. In 23 cells obtained from an individual patient 8 were relatively flat, 7 had multiple alterations, and then changes were divergent: 5 with more on one path with a second change, 2 with more on another path, and 1 (Figure 17) . This analysis provides 3 major values: One, tissue/cfDNA analysis would have tremendous difficulties in resolving the subclones. Two, clonal evolution occurs where different cells branched from earlier lesions, allowing for monitoring patients over time to understand which subclonal alterations have specific drug sensitivities/resistances, and ultimately for predicting a weighted average of response to new drug therapies or combinations. Three, understanding the co-occurrence of different alterations within a single cell could potentially help us inform of exploitations of pathways (*i.e*. if they have an AR amp and PTEN deletion in the same cell or different cells may make a difference).

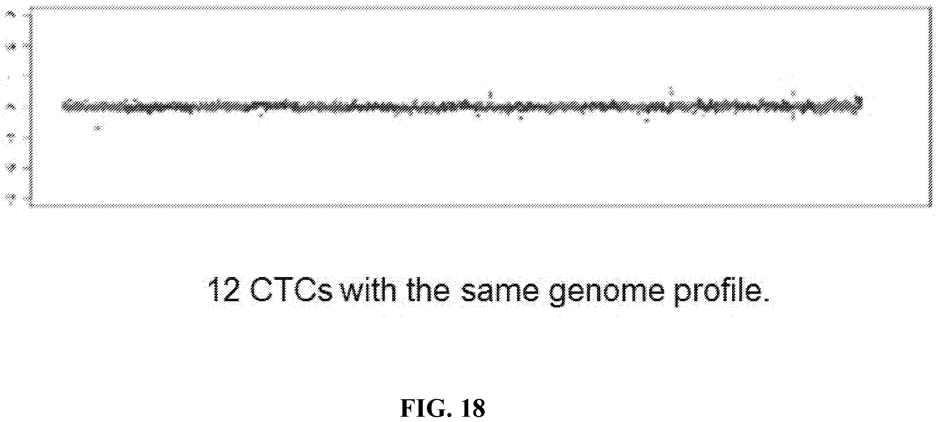

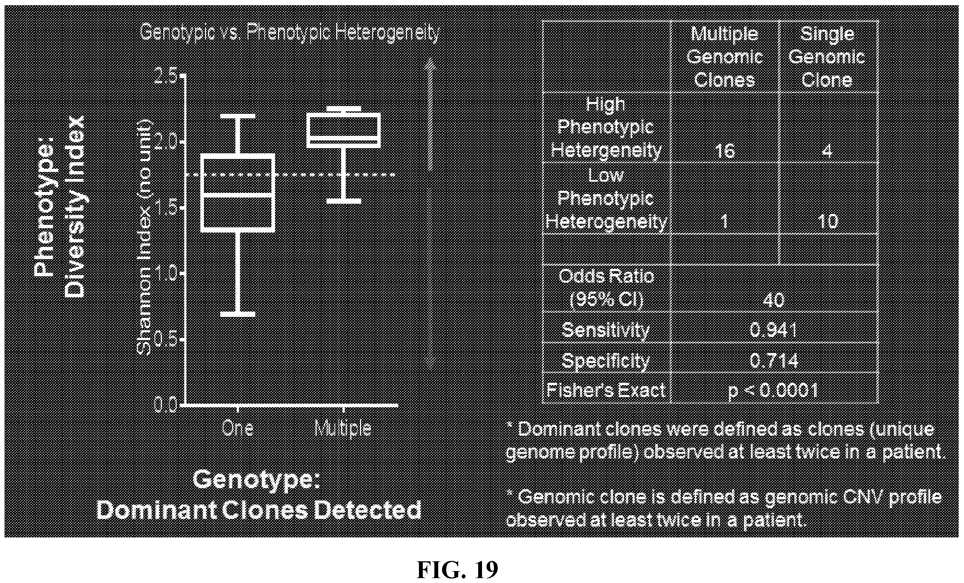

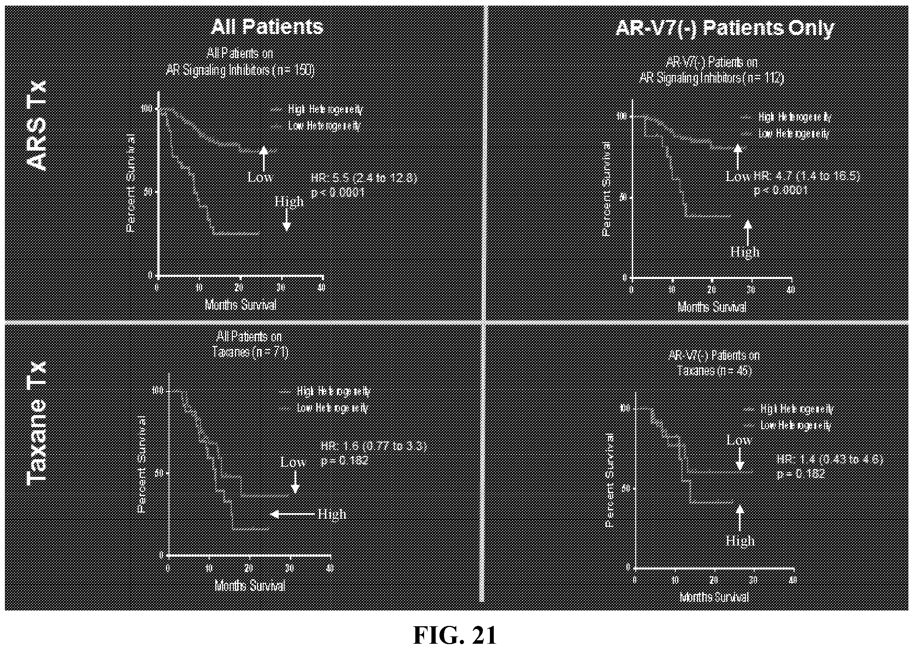

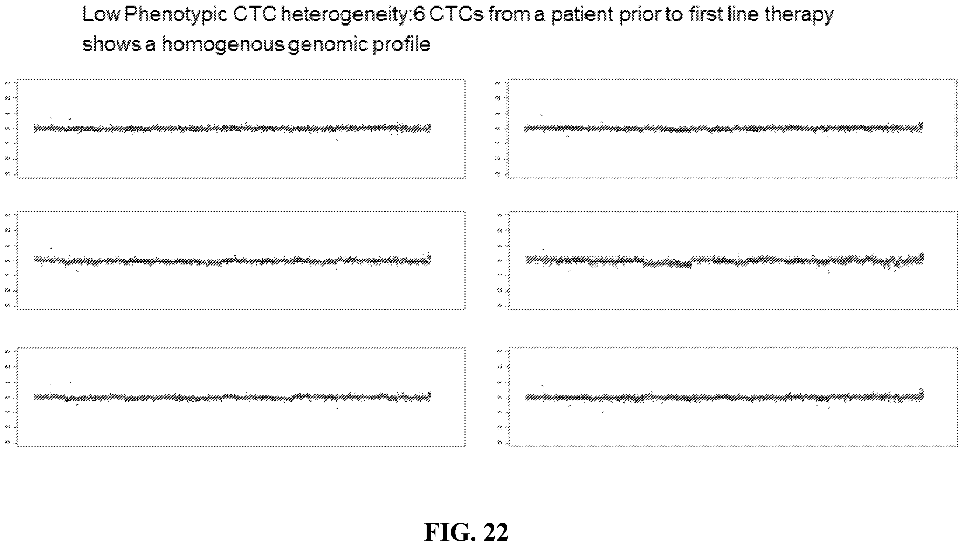

Single CTC sequencing can also inform of a lack of clonal diversity in a 2nd line post taxane patient who might not be considered for ARTx. This patient responded to enzalutamide (Figure 18). As shown in Figure 19, CTC phenotypic heterogeneity correlates with genomic heterogeneity. Figure 20 A shows and example of Cell Type K genomics, characterized by frequent CNVs, high number of breakpoints and an accompanying phenotype characterized by a large nucleus, high nuclear entropy and frequent nucleoli. Cell type K also has a large cytoplasm. Figure 20 B shows genomic instability for cell type K compared to all other CTC phenotypes. Figure 21 shows that high phenotypic heterogeneity is an informative biomarker in AR-V7 negative patients. Figure 22 shows low phenotypic CTC heterogeneity in 6 CTCs from a patient prior to first line therapy that show a homogenous genomic profile.

Figure 23 show a heatmap of 15 mathematical CTC phenotypic subtypes were identified using unsupervised analysis based on CTC protein and morphological features.

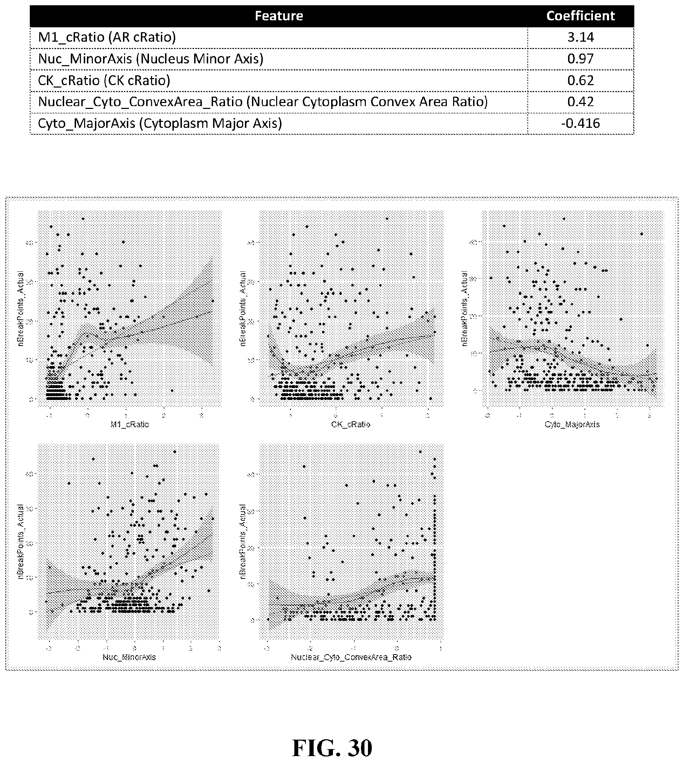

Using supervised cluster analysis, 5 morphological and protein expression features are found to be predictive of CTC genomic instability. The first four features are positively correlated with genomic instability and the last one is negatively correlate (Figure 30).

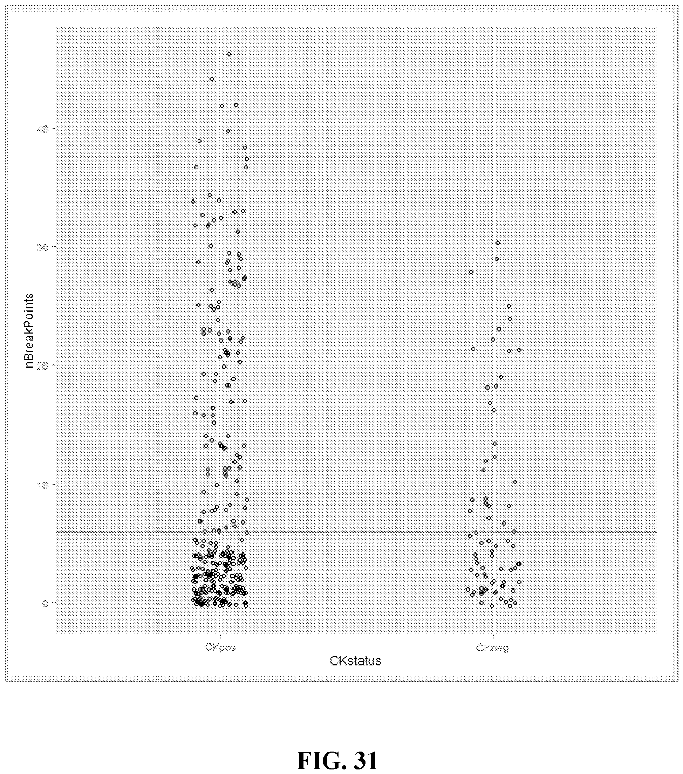

As shown in Figure 31, CK(-) CTCs have higher incidence of and are predictive of genomic instability.

Amplification of following genes is predictive of genomic instability: ACADSB, AR, BRAF, CCDC69, ETV1, EZH2, KRAS, NDRG1, PTK2, SRCIN1, YWHAZ. Deletion of following genes is predictive of genomic instability: ABR, ACADSB, BCL2, CCDC6, CDKN2B-AS1, CXCR4, KLF5, KRAS, LOC284294, MAP3K7, MTMR3, PTEN, PTK2B, RB1, RBPMS, RND3, SMAD4, SNX14, WWOX, ZDHHC20.

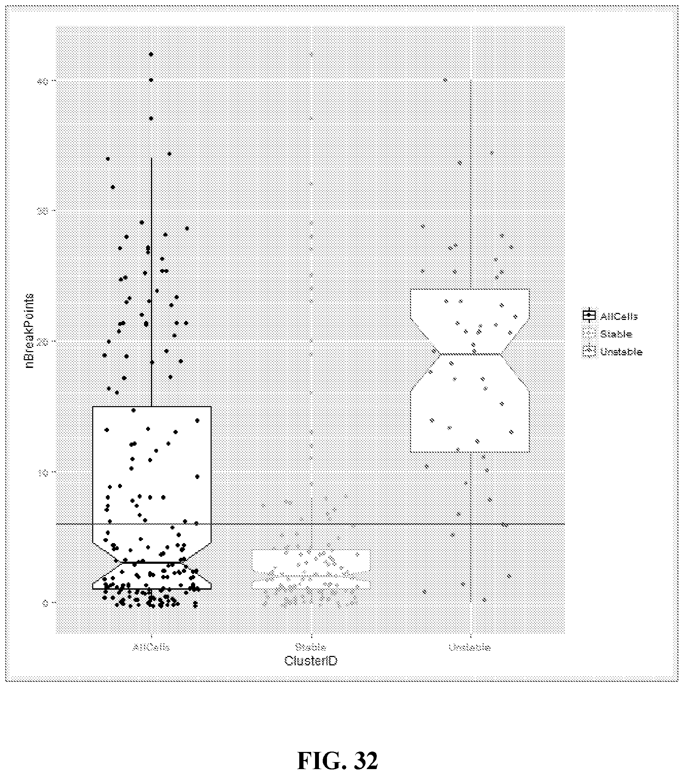

A classifier was developed based on protein and morphological features for the prediction of CTC genomic instability with high accuracy. In Figure 32, the Y axis shows the real LSTs (nBreakPoints) and X axis shows the predicted instability (stable vs. unstable). The CTCs predicted as high genomic instability, may be sensitive to DNA damaging drugs, such as platinum based chemotherapies (i.e. carboplatin, cisplatin), or targeted therapeutics which target homologous recombination deficiencies, including PARP inhibitors, DNA-PK inhibitors and therapeutics targeting the ATM pathway.

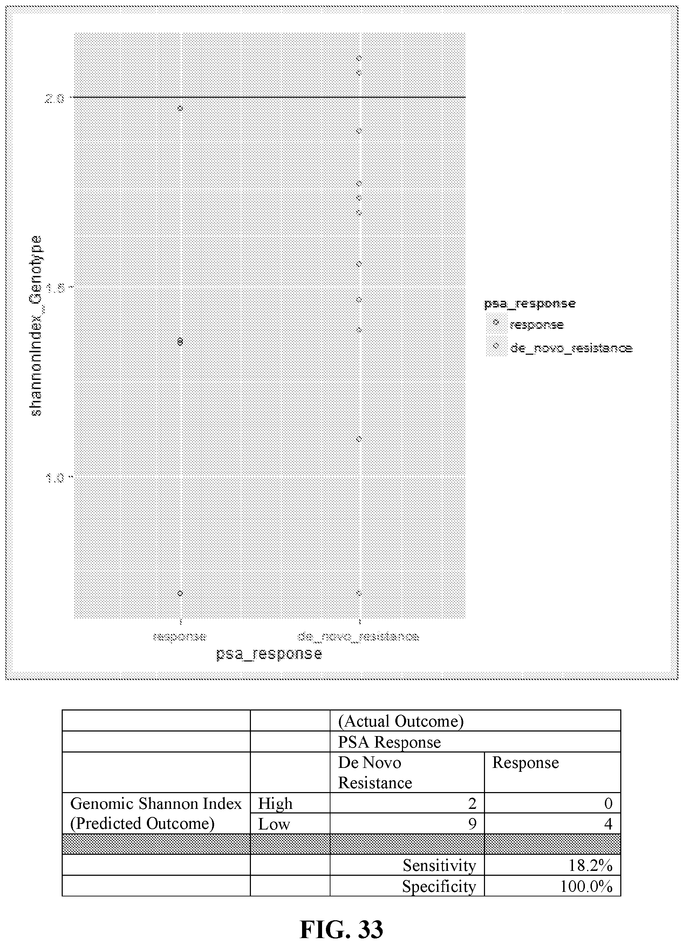

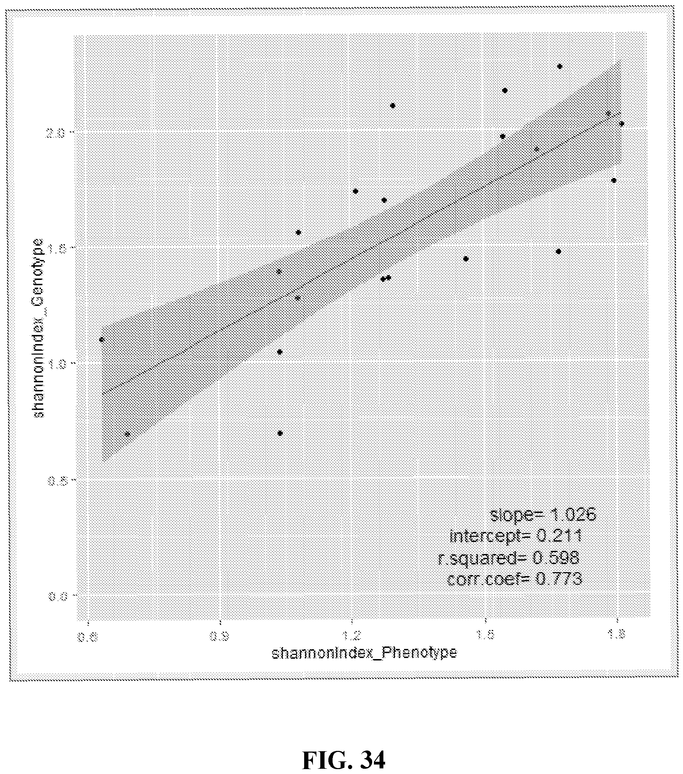

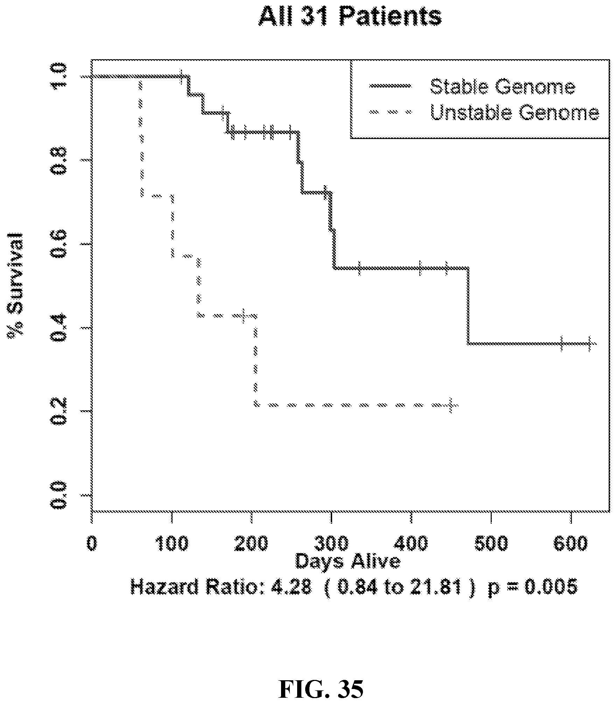

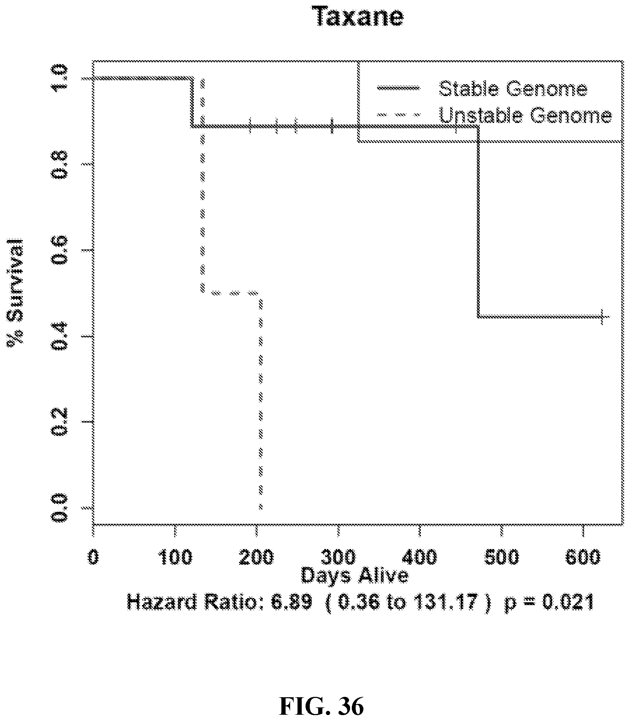

Figure 33 shows that phenotypic heterogeneity is predictive of overall survival and response to AR targeted therapy. Figure 34 shows that CTC phenotypic heterogeneity is predictive of genotypic heterogeneity. High phenotypic heterogeneity is 40 times more likely to represent multiple genomic clones than low phenotypic heterogeneity. Figure 35 shows that CTC genomic instability is predictive of mCRPC patient overall survival. Figure 36 shows that that CTC genomic instability is predictive of mCRPC patient response to Taxane therapy.

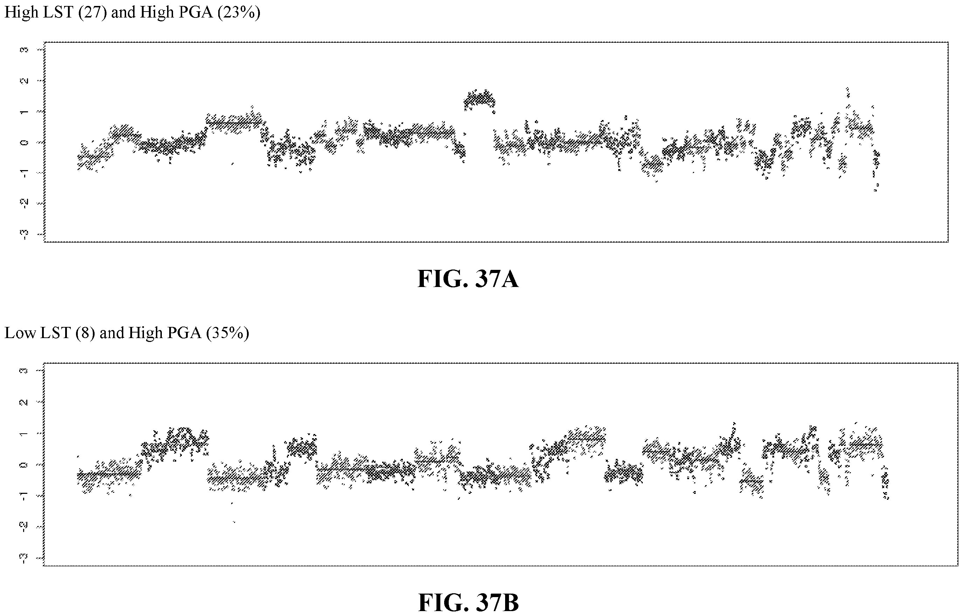

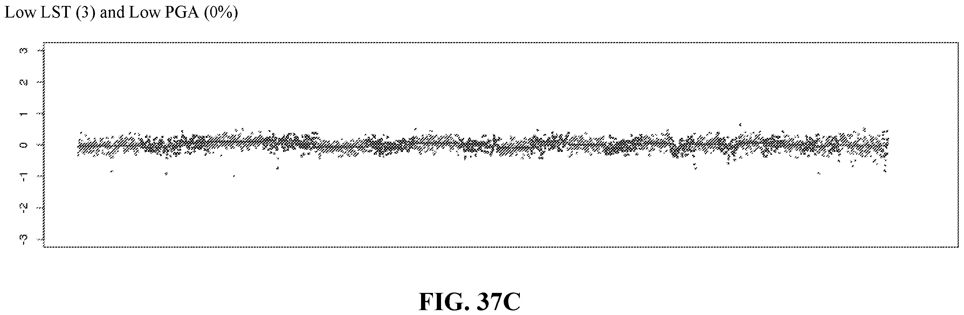

Genomic instability. LST and PGA was measured as the surrogate of genomic instability. LSTs: n of chromosomal breaks between adjacent regions of at least 10 Mb. Popova et al., Cancer Res. 72(21):5454-62 (2012). PGAs: percentage of a patient's genome harboring copy number alterations (amplification or deletions). Zafarana et. al, Cancer 2012 Aug; 118(16): 4053 (2012). Examples: High LST (27) and High PGA (23%) Figure 37 A-C.

### Example 3: Development of a Liquid Biopsy HRD+ Signature

This example demonstrates the development of CTC based methods to detect HRD in circulating tumor cells (CTCs) isolated from a simple peripheral blood draw at critical clinical decision points prior to treatment. Trained with HRD genomic alterations (LSTs) detected by > 600 individual CTCs sequenced, multi-parametric high content image analysis algorithms were used to determine the HRD status of individual CTCs based on cellular and nuclear morphological features that are associated with these alterations. Based on the subclonal prevalence of CTCs with HRD+ phenotypes within both heterogeneous and homogeneous disease states, this test can predict HRD genomics with 78% accuracy and 86% specificity at the cellular level. Utilizing patient scoring guides improves HRD+ phenotypic accuracy to 95% at the patient level.

Epic Sciences HRD+ signature prevalence and clinical validity: In a validation cohorts of 168 and 86 mCRPC patients, the developed HRD signature was detected in 32% & 37% of patients respectively. Marker prevalence increases in patients in later lines of systemic therapies (1^{st} line 25%, 4^{th} line 41%) compared to the 10-20% prevalence of HRD associated genomic alterations recently reported within similar cohorts. Patients identified as HRD+ have worse OS on AR Tx (HR=9.83, p<0.0001) and Taxanes (HR=3.31, p=0.001) compared to patients who are HRD-.

Epic Sciences HRD+ signature predicts PARPi and Platinum therapy response in mCRPC: In a prospective phase II clinical trial randomizing AR Tx vs. AR Tx + PARPi, HRD+ patients had statistically significant improvement in overall response rate (ORR, >50% PSA drop) in AR Tx + PARPi arm (**88% vs. 42%**). Additionally, patients on the AR Tx arm demonstrated a 320% increase in HRD+ CTCs from baseline to on-therapy blood draws. Patients on the AR Tx + PARPi arm demonstrated a 95% decrease in HRD+ CTCs from baseline to on-therapy blood draws. Early data supports the HRD+ signature also predicts ORR of platinum chemotherapy sensitivities as well as similar reduction of HRD+ CTCs from baseline to during therapy blood draws with platinum chemotherapy.

Epic Sciences PARPi resistance signature: In addition to the HRD+ CTC biomarker signature, Epic Sciences has also developed a signature for predicting primary resistance to PARPi. The PARPi resistance signature identified specific CTC phenotypes associated with epithelial plasticity as well as AR/PI3K reciprocal feedback which demonstrate resistance to combination therapy AR Tx + PARPi. Epic Sciences' CTC HRD sensitivity and PARPi resistance signatures are non-invasive alternative tests on a robust clinically compatible platform that can be performed in less than 5 days with significantly less associated COGS. The higher prevalence of the Epic Sciences HRD+ CTC marker in mCRPC patients, and the ability to stratify patients based on both PARPi response and resistance markers make this a valuable tool for guiding clinical decisions in practice and throughout clinical trials.

Briefly, blood samples were collected, red blood cells were lysed and remaining nucleated cells, inclusive of leukocytes and CTCs were deposited onto glass slides. For each sample, up to 12 replicate slides were created, depending on the sample volume and WBC count. 2 replicate slides were stained by IF using a cocktail of antibodies targeting multiple cytokeratins (CK), CD45 and the N-terminal AR expression. DAPI staining was used to define nuclear area and context. Algorithms to identify CTCs were employed utilizing the fluorescent and morphologic features identified outlier cells with high probability of being CTCs. Trained readers classified CTCs based on marker expression and morphology. Reportable values included CTC/mL, AR+/- CTC/mL, CK+/- CTC/mL, apoptotic CTC/mL and CTC clusters/mL.

Following CTC classification, confirmed CTCs underwent single cell digital pathology segmentation where clear segments of the nucleus (DAPI), cytoplasm (CK), and AR were created and recorded. Automated cell segmentation followed by trained reader confirmation of segments was performed on all identified CTCs in a patient blood sample. Single cell feature extraction extracts 20 quantitative features, and 2 categorical features. These included:

**Quantitative features:** (1) Protein Features: AR protein expression, CK protein expression; (2) Morphologic Features: Nuclear Area (um2), Cytoplasmic Area(um2), Nuclear Convex Area (um2), Cytoplasmic Convex Area (um2), Nuclear Major Axis (um), Cytoplasmic Major Axis (um), Nuclear Minor Axis (um), Cytoplasmic Minor Axis (um), Nuclear Circularity, Cytoplasmic Circularity, Nuclear Solidity, Cytoplasmic Solidity, Nuclear Entropy, Nuclear to Cytoplasmic Convex, Area Ratio, Nucleoli, CK Speckles, and Nuclear Speckles.

**Qualitative Features:** CK ⁺ or CK⁻, AR ⁺ or AR⁻.

### Following single cell feature extraction individual CTCs were NGS sequenced

**Whole genome CNV analysis:** Non-apoptotic individual CTCs were relocated on the slide based on a mathematical algorithm that converts the original CTC positions (x and y coordinates) computed during the scanning procedure into a new set of x, y references compatible with the Nikon TE2000 inverted immunofluorescent microscope used for cell capture. Single cells were captured using an Eppendorf TransferMan NK4 micromanipulator. Cells were deposited into individual 0.2 mL PCR tubes using 1 µL of TE buffer and immediately lysed by the addition of 1.5 µL of high pH lysis buffer as previously described. Tubes containing individual cells were spun down and frozen on dry ice until further processing. Single cell whole genome amplification (WGA) was performed using SeqPlex Enhanced (Sigma) according to the manufacturer's instructions with minor modifications. Post-WGA, DNA concentrations were determined by UV/Vis. NGS libraries were constructed using NEBNext Ultra DNA Library Prep Kit for Illumina (NEB) from 100ng of WGA DNA as per manufacturer recommendation with minor modifications. After NGS library preparation, library concentrations and size distributions were determined by PicoGreen (ThermoFisher Scientific) and Fragment Analyzer (Advanced Analytical). Equinanomolar concentrations from each library were pooled and sequenced on an Illumina NextSeq 500 using a Rapid Run Paired-End 2x150 format (PE 2x150).

Raw sequencing data (FASTQ) were aligned to hg38 human reference genome from UCSC (http://hgdownload.soe.ucsc.edu/goldenPath/hg38/bigZips/) using Burrows-Wheeler Aligner (BWA, http://bio-bwa.sourceforge.net). Alignment files (BAM) were filtered for quality (MAPQ 30) to keep only the reads that have one or just a few "good" hits to the reference sequence. The filtered alignment files were further processed using two separate pipelines (Fig 1). To generate a CNV analysis control genome from single cell WGA DNA, 15 WBCs were collected from different human adult male individuals without hematological disease and were used as a universal reference. For each sample, read counts per bin (window size per bin varies between two pipelines, see below) were normalized proportionally to bring the total read counts to 1 million. Then median, mean, and standard deviation (sd) of normalized reads number of these controls were calculated for each bin for further use.

Analysis pipeline 1 was utilized for genomic instabilities estimation. Hg38 human genome was divided into ~3000 bins of 1 million base pair and reads were counted within each bin for each sample. For each sample, read counts per bin were normalized proportionally to make the total read counts to 1 million, followed by GC content adjustment for each bin. Median values of each bin read counts of WBC controls were used to exclude low coverage bins from downstream analyses (<100 reads). Ratios between test samples and WBC controls were calculated and reported after Log2 transformation. Chromosomal segments were predicted using R Bioconductor package DNA copy, which found break points where DNA copy number changed. LSTs were calculated as number of chromosomal breaks between adjacent regions of at least 10 Mb, and PGAs were calculated as the percentage of a patient's genome harboring copy number alterations (amplification cut-off: >0.4; deletion cut-off: < -0.7).

### Phenotypic prediction of LSTs (pLST):

A training set of 608 patient CTCs were analyzed for quantitative and qualitative digital pathology features. CTCs were sequentially processed via image analysis and via sequencing. A multivariate classifier was developed utilizing the below techniques.

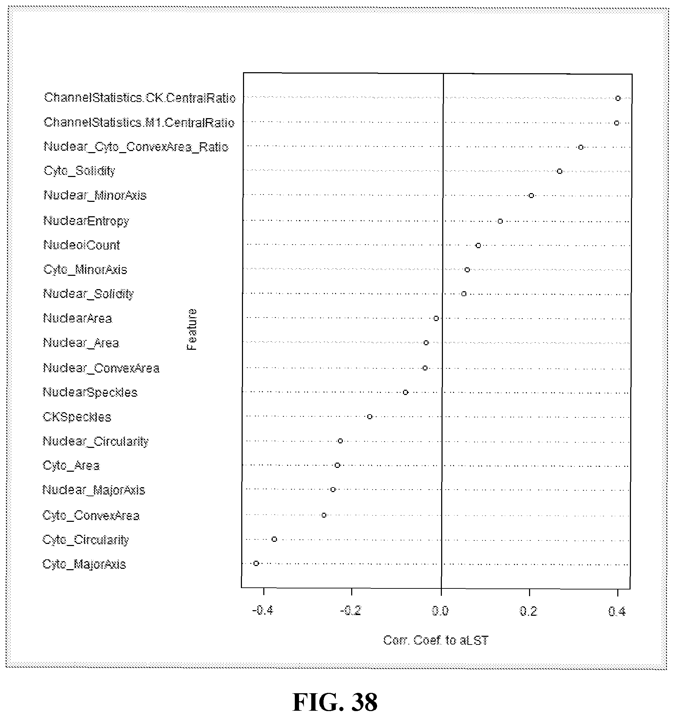

Image analysis yields p protein/morphologic features per CTC (X1, X2, ..., Xp). Sequencing yields the "actual" number of LSTs per CTC (aLST). Next, a multivariate linear regression algorithm is trained to predict aLST given the series of protein/morphology features from imaging (aLST ~ X1 + X2 + ... + Xp). After training (and when making predictions on new test data), the algorithm outputs a predicted number of LSTs (terms 'pLST') given the series of protein/morphologic features from imaging (X1, X2, ..., Xp) per CTC. Prior to training or testing, commonly used data transformation and normalization techniques are used to linearize the imaging features (X1, X2, ..., Xp) with aLST. Any normalizations applied to the training set are done on the test set. To assess feature importance, one technique used was to evaluate how strongly each imaging feature (X1, X2, ..., Xp) correlates with aLST on a univariate basis. First, for each imaging feature, Pearson's correlation coefficient with aLST is calculated. Correlation coefficients >> 0 indicate features that strongly trend positively with aLST (ex. Greater values for X lead to greater values for aLST). Correlation coefficients << 0 indicate features that strongly trend negatively with aLST (ex. Lower values for X lead to greater values for aLST). Correlation coefficients near 0 indicate features that do not trend either way with aLST (and therefore may not be as predictive of aLST). Taking the absolute value of the correlation coefficients for each feature is done to sort features having strong predictive association with aLST (positively or negatively) vs features with less powerful predictive associations with aLST. This is represented in Figure 38. pLST analysis of an independent mCRPC cohort of patients with blood draws immediate prior to initiation of AR targeted therapy (via cyp17 inhibitor, Abiraterone, or AR inhibitor, Enzalutamide) or taxane chemotherapy (docetaxel or cabazitaxel). Algorithms encompassing varying levels of pLST+ cells led to patients with worse outcomes than those who were negative for the marker.

Example 4: Identification of Patient Populations for Drug Treatment Based on the Presence of CTC Types.

Patients included in this study were all first or second line metastatic castration-resistant prostate cancer (mCRPC) patients. Patient demographics for the samples used in these studies are shown in Table 2.

**Table 2. Patient Demographics¹.**

| **Patient Characteristics** | |
|---|---|
| **Unique Patients** | 98 |
| **Age, years: median (range)** | 70 (45-87) |

| **Primary Treatment** | |
|---|---|
| **Prostatectomy** | 47 (48%) |
| **Radiation** | 21 (21%) |
| **Brachytherapy** | 3 (3%) |
| **None** | 27 (28%) |

| **Sample Characteristics** | |
|---|---|
| **Total Baseline (pre-therapy) Samples** | 107 |

| **Metastatic Therapy Initiated after Baseline** | |
|---|---|
| **Abiraterone** | 47 (44%) |
| **Enzalutamide** | 60 (56%) |

| **Line of Metastatic Therapy at Baseline** | |
|---|---|
| **1^{st} Line** | 64 (60%) |
| **2^{nd} Line** | 43 (40%) |

| **Chemotherapy Status at Baseline** | |
|---|---|
| **Chemo-naïve** | 97 (91%) |
| **Chemo-exposed** | 10 (9%) |

| **Metastatic Sites of Disease at Baseline** | |
|---|---|
| **Bone Only** | 32 (30%) |
| **Lymph Node Only ^{a}** | 20 (19%) |
| **Bone and Lymph Node ^{a}** | 45 (42%) |
| **Bone and Visceral +/- Lymph Node ^{a}** | 8 (7%) |
| **Other Soft Tissue Only** | 2 (2%) |
| **Laboratory Measures at Baseline** | |
| **PSA, ng/mL: median (range)** | 20.13 (0.09-2006.14) |
| **Hgb, g/dl: median (range)** | 12.7 (7.2-15.0) |
| **ALK, unit/L: median (range)** | 99 (25-2170) |
| **LDH, unit/L: median (range) ^{b}** | 207 (123-1293) |
| **ALB, g/dl: median (range)** | 4.2 (3.3-4.9) |
| ^{a} - includes patients with other soft tissue disease | |
| ^{b} - two samples did not have LDH available | |

| | |
|---|---|
| PSA (prostate specific antigen); Hgb (hemoglobin); ALK (alkaline phosphatase); LDH (lactate dehydrogenase); ALB (albumin). | |

Cells of type K were identified essentially as described above.

Table 3 shows the incidence of cell type K by treatment and line of therapy. 25% of first and second line patients are cell type K positive.

**Table 3. Incidence of cell type K by treatment and line of therapy.**

| | Treatment | # Positive | # Negative | Total | % Positive |
|---|---|---|---|---|---|
| 1^{st} Line | Abi | 10 | 14 | 24 | 42% |
| | Enza | 7 | 33 | 40 | 18% |
| | Abi + Enza | 17 | 47 | 64 | 27% |
| 2^{nd} Line | Abi | 3 | 20 | 23 | 13% |
| | Enza | 7 | 13 | 20 | 35% |
| | Abi + Enza | 10 | 33 | 43 | 23% |
| 1^{st} & 2^{nd} Line | Abi | 13 | 34 | 47 | 28% |
| | Enza | 14 | 46 | 60 | 23% |
| | Abi + Enza | 27 | 80 | 107 | 25% |

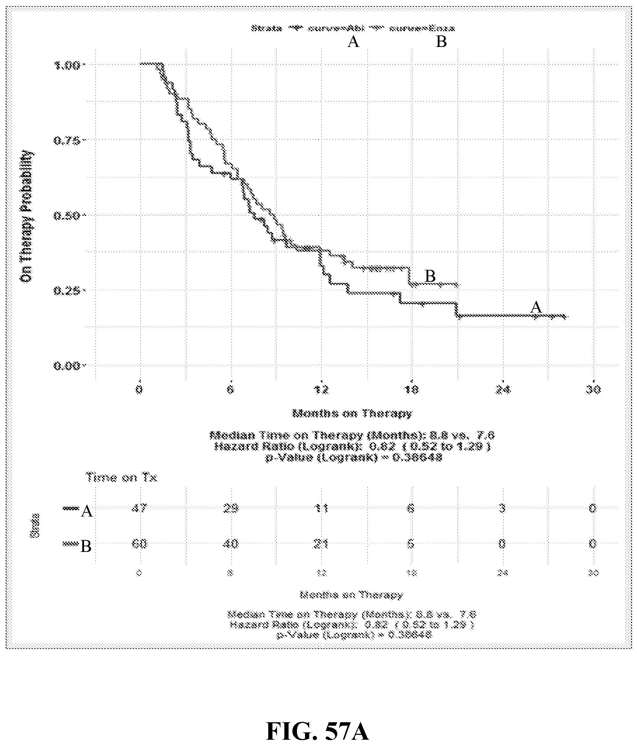

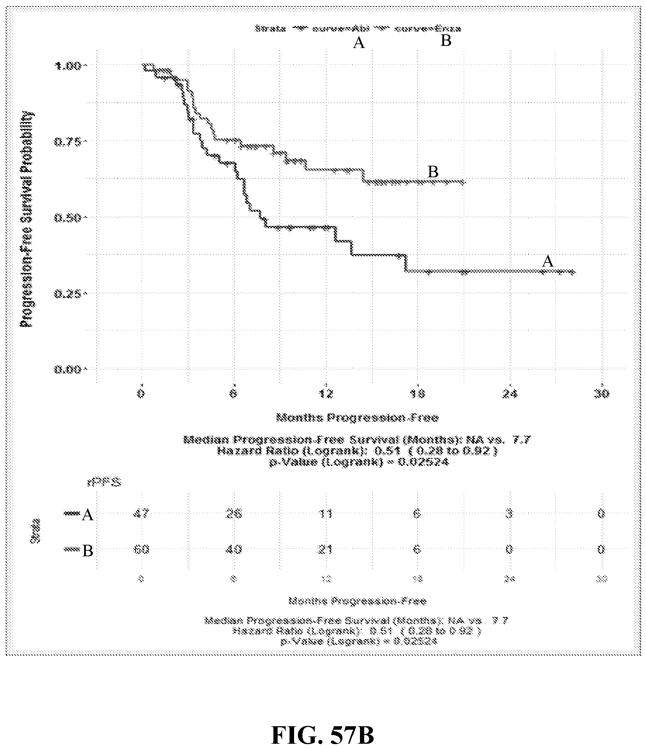

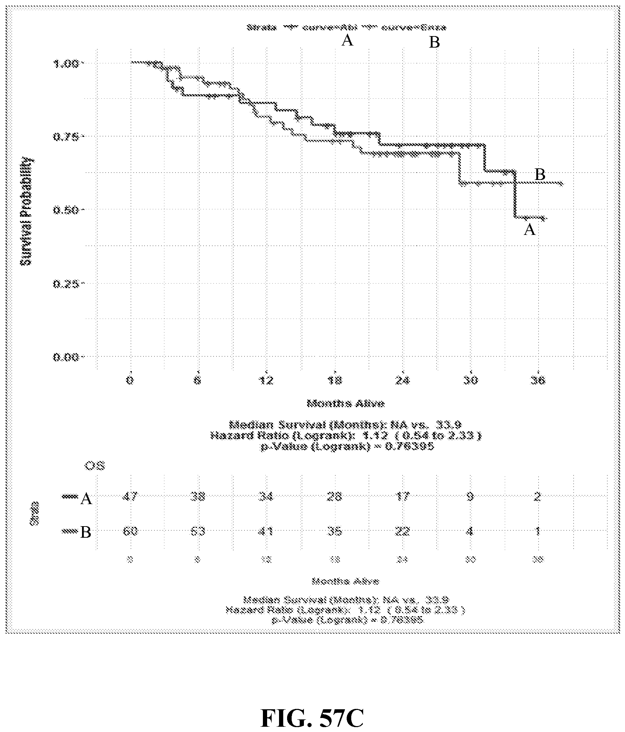

As shown in Figure 57, patients had similar overall survival (OS) and Time on therapy (Tx) when treated with abiraterone (Abi) or enzalutamide (Enza), but Enza treated patients had significantly better radiographic Progression Free Survival (rPFS) (p=0.025).

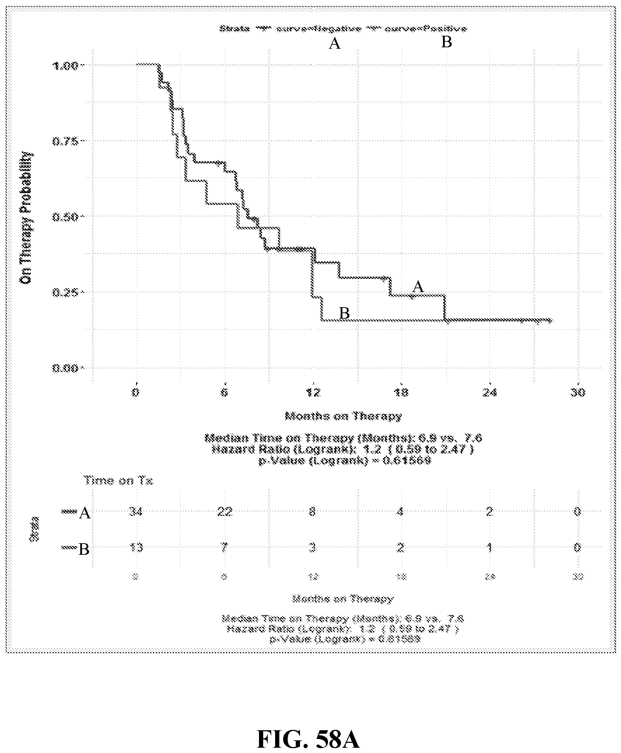

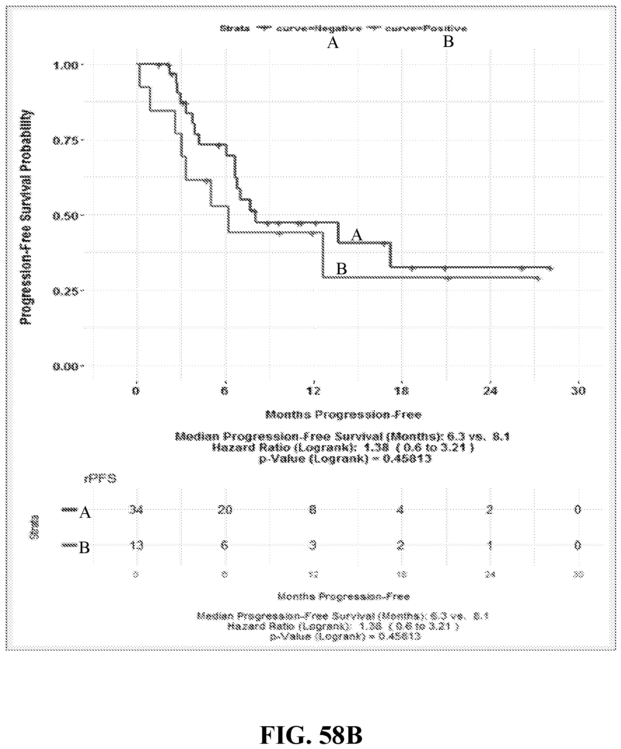

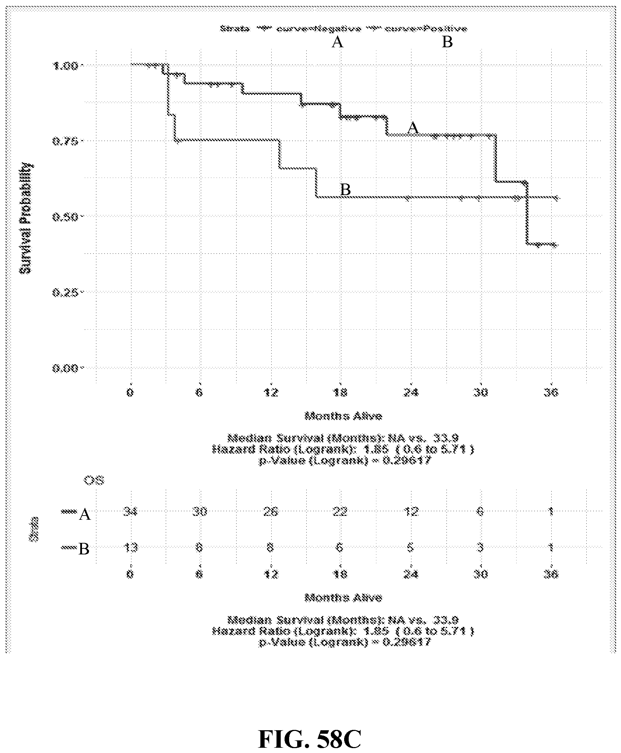

As shown in Figure 58, in Abi treated patients, patients with or without the presence of cell type K showed no differences in Time on Tx, rPFS and OS.

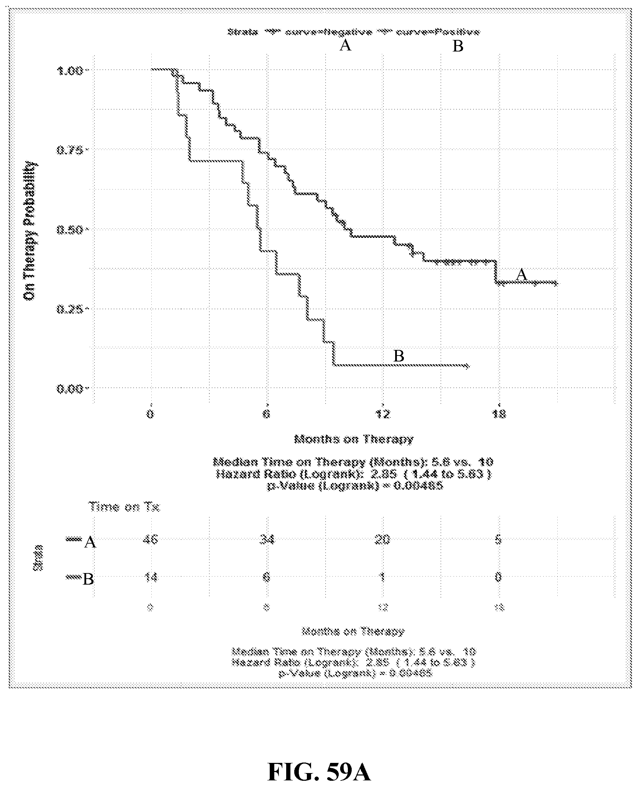

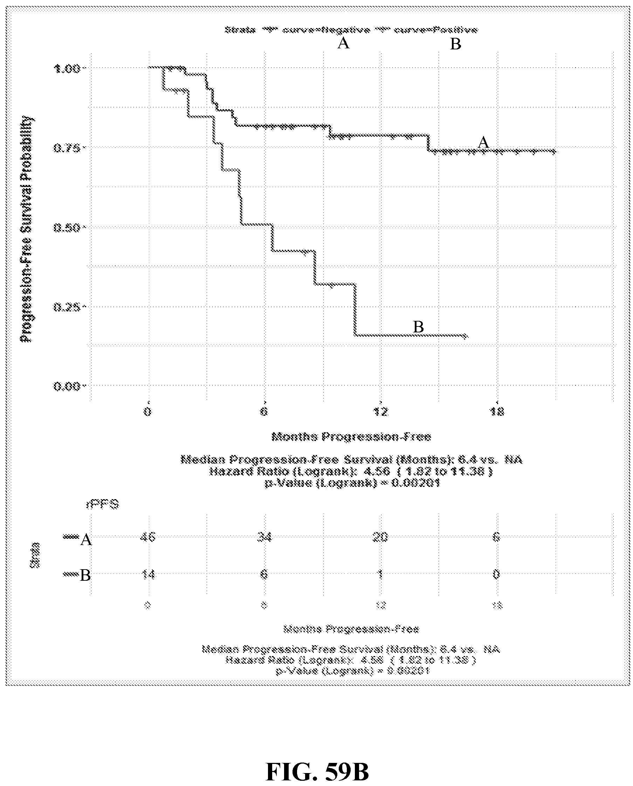

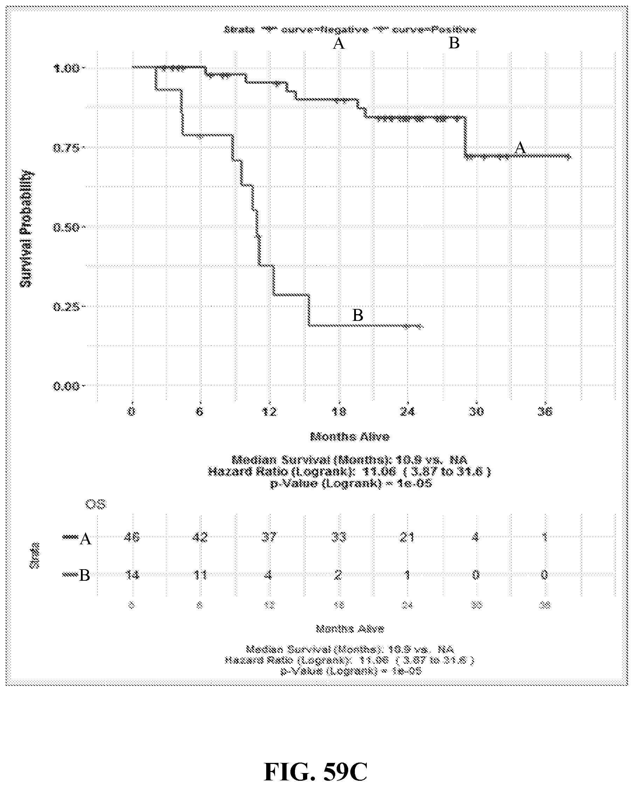

As shown in Figure 59, in Enza treated patients, patients without the presence of cell type K showed significant improvement in Time on Tx, rPFS and OS (all p<0.005).

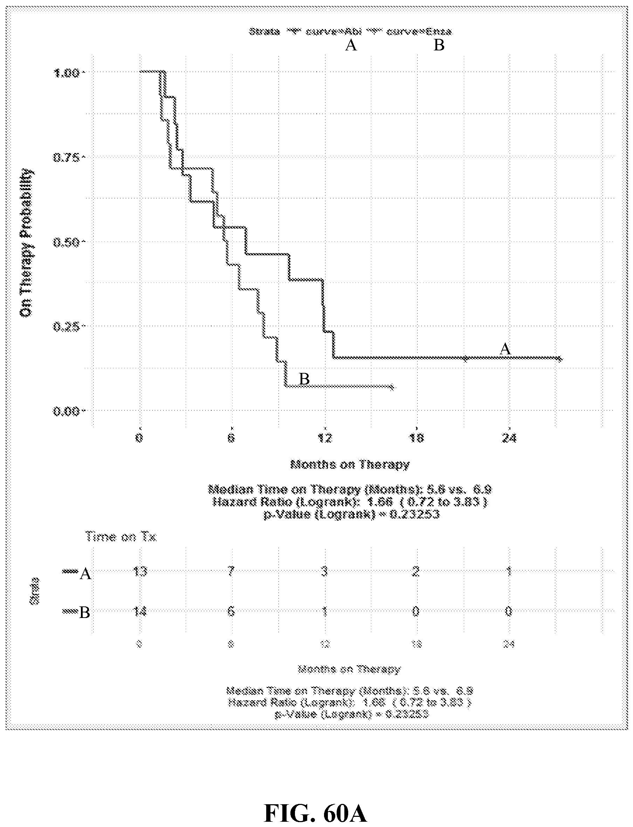

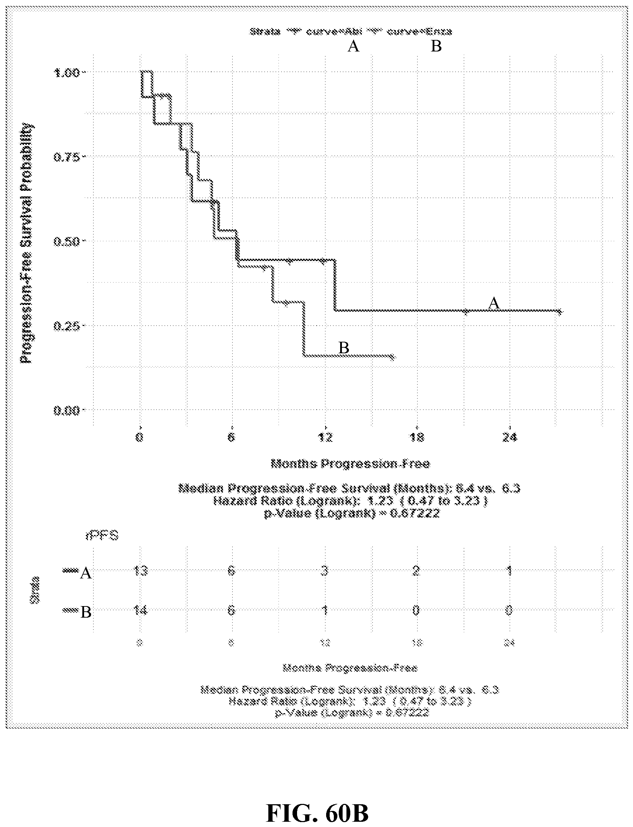

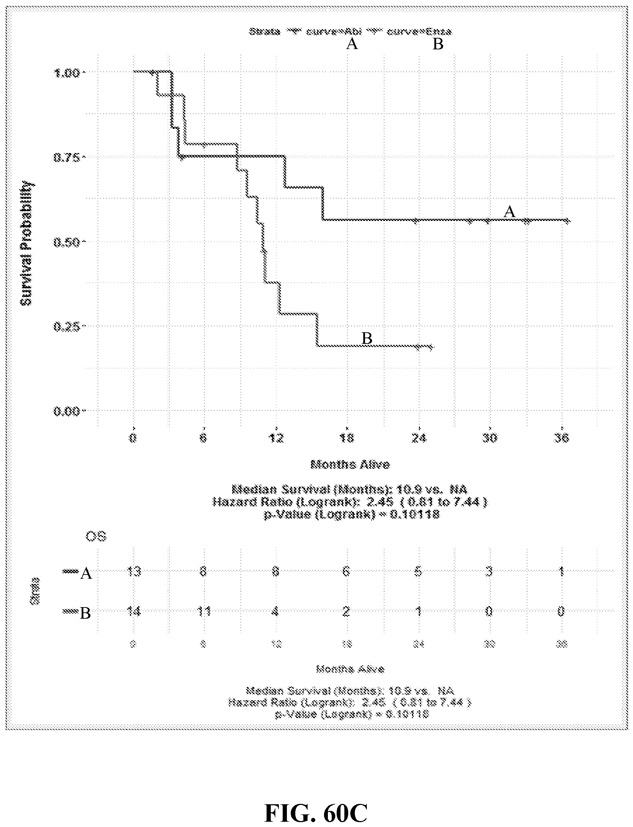

As shown in Figure 60, in patients with the presence of cell type K, patients treated with Abi showed better OS than Enza treated patients, but not statistically significant (p=0.1).

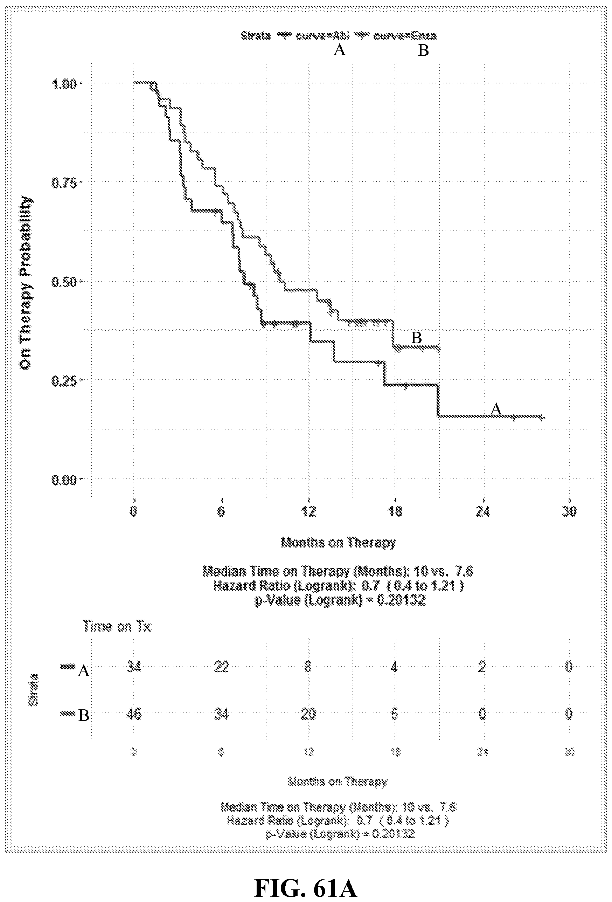

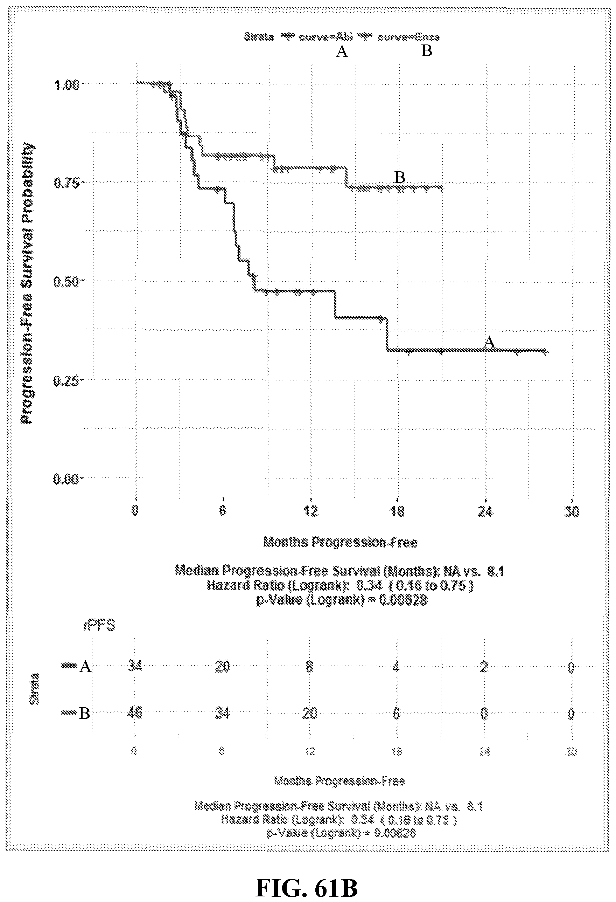

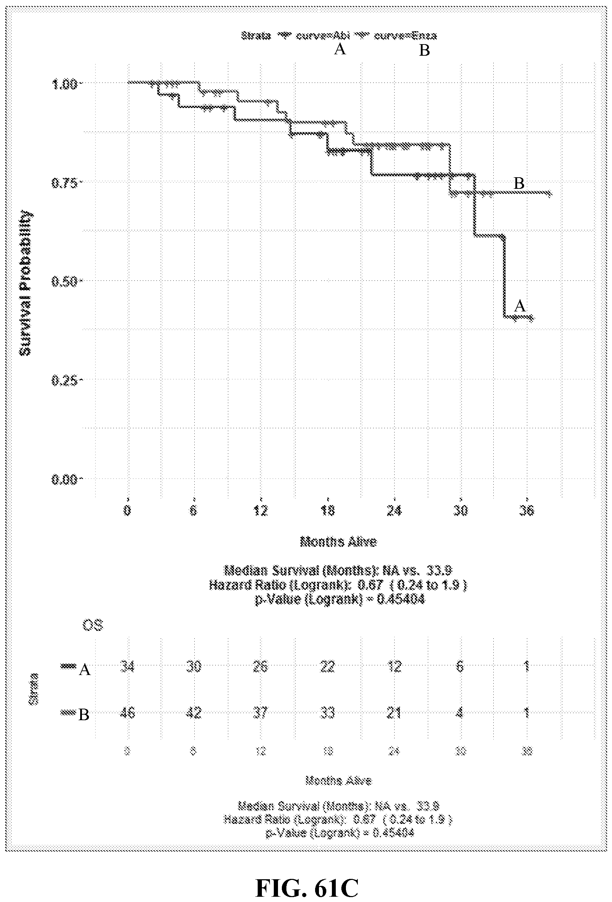

As shown in Figure 61, in patients without the presence of cell type K, patients treated with Enza showed significantly better rPFS than Abi treated patients (p=0.006), but no difference in OS.

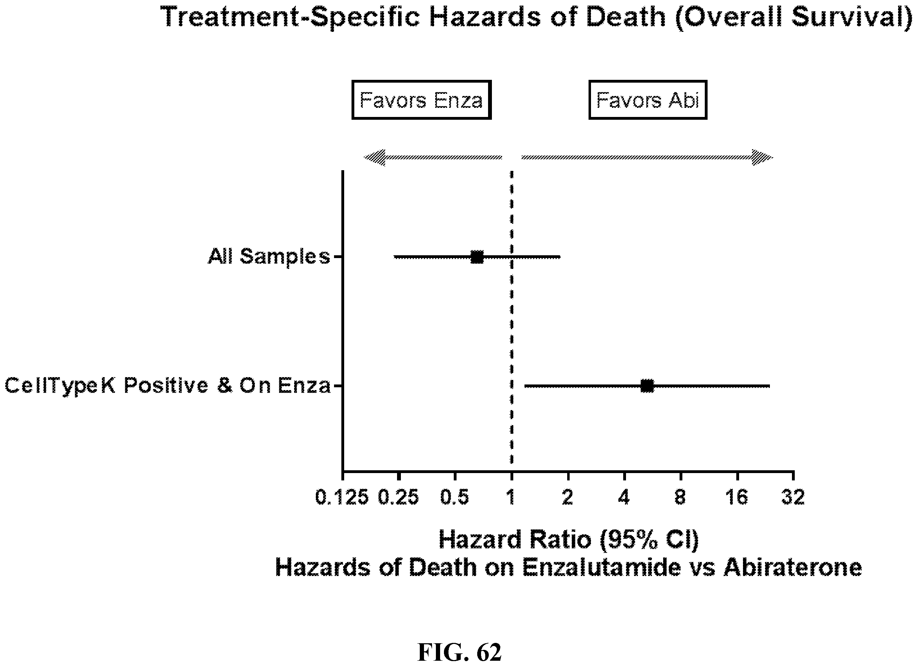

Cell type K was a predictive biomarker in mCRPC patients treated with abiraterone or enzalutamide. Cell type K incidence in relation to the hazards of death of mCRPC patients on Abi versus Enza was estimated by using multivariate cox proportional hazards models. Variables included in the multivariate models were: therapy (Abi vs. Enza), patient cell type K status (1 if patient had 1+ cell type K CTC; 0 if cell type K CTC count = 0), and the interaction term between therapy and the cell type K status. Analysis results are found in Table 4, and visualization of confidence interval is shown in Figure 62. Cell type K and therapy interactions are significant (p<0.05) in multiple variable model. Patients with the presence of cell type K were more favorable to Abi, and patients without the presence of cell type K were more favorable to Enza.

**Table 4 shows the analysis results from multiple variate cox proportional hazards model.**

| **Feature** | **Coding** | ***p* value** | **HR** | **lower .95** | **upper .95** |
|---|---|---|---|---|---|
| Therapy | =0 if Abi; =1 if Enza | 0.4167 | 0.6543 | 0.2351 | 1.821 |
| Cell type K | =0 if K-; =1 if K+ | 0.2843 | 1.8472 | 0.6008 | 5.68 |
| Cell Type K : Therapy Interaction | =0 if not K+ and Enza; =1 if K+ and Enza | **0.0306** | 5.2792 | 1.168 | 23.861 |

## Claims

1. A method of predicting responsiveness to treatment with abiraterone and enzalutamide in a hormone-refractory prostate cancer patient, comprising:
(a) performing a direct analysis comprising immunofluorescent staining and morphological characterization of nucleated cells in a blood sample obtained from the patient to identify and enumerate circulating tumor cells (CTC);
(b) individually characterizing digital pathology parameters to generate a profile for each of the CTCs, wherein the parameters comprise:
(i) size of the nucleus,
(ii) nuclear entropy, and
(iii) number of nucleoli, or size of the cytoplasm;
(c) classifying individual cells into CTC subtypes; and
(d) determining the absence or presence of a biomarker CTC in the CTC subtypes,
wherein the biomarker CTC has the characteristics of a large nucleus, high nuclear entropy, and frequent nucleoli or large cytoplasm,
wherein the presence of the biomarker CTC indicates responsiveness of the patient to abiraterone, and
wherein the absence of the biomarker CTC indicates responsiveness of the patient to enzalutamide.

2. The method of claim 1, wherein the cells are classified with a CTC subtype classifier developed from unsupervised classification.

3. The method of claim 1 or 2, further comprising the step of isolating the CTCs from said sample.

4. Abiraterone for use in a method of treatment of a hormone-refractory prostate cancer patient, wherein the method comprises the method of predicting responsiveness to treatment with abiraterone according to any one of claims 1 to 3.

5. Enzalutamide for use in a method of treatment of a hormone-refractory prostate cancer patient, wherein the method comprises the method of predicting responsiveness to treatment with enzalutamide according to any one of claims 1 to 3.

## Patentansprüche

1. Verfahren zum Vorhersagen vom Ansprechen auf Behandlung mit Abirateron und Enzalutamid bei einem Patienten mit hormonrefraktärem Prostatakrebs, umfassend:
(a) Durchführen einer direkten Analyse, umfassend immunfluoreszentes Färben und morphologische Charakterisierung von Zellkernhaltigen Zellen in einer Blutprobe, die von dem Patienten erhalten wurde, um zirkulierende Tumorzellen (CTC) zu identifizieren und durchzählen;
(b) individuelles Charakterisieren digitaler Pathologieparameter, um ein Profil für jede der CTCs zu erzeugen, wobei die Parameter Folgendes umfassen:
(i) Größe des Zellkerns,
(ii) Zellkernentropie und
(iii) Anzahl von Zellkernen, oder Größe des Zytoplasmas;
(c) Klassifizieren individueller Zellen in CTC-Subtypen; und
(d) Bestimmen des Fehlens oder Vorhandenseins einer Biomarker-CTC in den CTC-Subtypen,
wobei die Biomarker-CTC die Eigenschaften eines großen Zellkerns, höherer Zellkernentropie und häufiger Zellkerne oder eines großen Zytoplasmas aufweist,
wobei das Vorhandensein der Biomarker-CTC Ansprechen des Patienten auf Abirateron angibt und
wobei das Fehlen der Biomarker-CTC Ansprechen des Patienten auf Enzalutamid angibt.

2. Verfahren nach Anspruch 1, wobei die Zellen mit einem CTC-Subtypklassifizierer klassifiziert sind, der durch unbeaufsichtigte Klassifizierung entwickelt wurde.

3. Verfahren nach Anspruch 1 oder 2, weiter umfassend den Schritt zum Isolieren der CTCs von der Probe.

4. Abirateron zur Verwendung in einem Verfahren zur Behandlung eines Patienten mit hormonrefraktärem Prostatakrebs, wobei das Verfahren das Verfahren zum Vorhersagen vom Ansprechen auf Behandlung mit Abirateron nach einem der Ansprüche 1 bis 3 umfasst.

5. Enzalutamid zur Verwendung in einem Verfahren zur Behandlung eines Patienten mit hormonrefraktärem Prostatakrebs, wobei das Verfahren das Verfahren zum Vorhersagen vom Ansprechen auf Behandlung mit Enzalutamid nach einem der Ansprüche 1 bis 3 umfasst.

## Revendications

1. Procédé de prédiction d'une réponse à un traitement à l'abiratérone et l'enzalutamide chez un patient atteint d'un cancer de la prostate réfractaire aux hormones, comprenant :
(a) la réalisation d'une analyse directe comprenant une immunofluorescence et une caractérisation morphologique de cellules nucléées dans un échantillon sanguin obtenu auprès du patient pour identifier et énumérer des cellules tumorales circulantes (CTC) ;
(b) la caractérisation individuellement de paramètres pathologiques numériques pour générer un profil pour chacune des CTC, dans lequel les paramètres comprennent :
(i) la taille du noyau,
(ii) l'entropie nucléaire, et
(iii) le nombre de nucléoles, ou la taille du cytoplasme ;
(c) la classification de cellules individuelles en sous-types de CTC ; et
(d) la détermination de l'absence ou de la présence d'un CTC biomarqueur dans les sous-types de CTC,
dans lequel le CTC biomarqueur présente les caractéristiques d'un grand noyau, d'une grande entropie nucléaire, et de fréquentes nucléoles ou d'un grand cytoplasme,
dans lequel la présence du CTC biomarqueur indique une réponse du patient à l'abiratérone, et
dans lequel l'absence du CTC biomarqueur indique une réponse du patient à l'enzalutamide.

2. Procédé selon la revendication 1, dans lequel les cellules sont classifiées avec un classificateur de sous-type de CTC développé à partir d'une classification non supervisée.

3. Procédé selon la revendication 1 ou 2, comprenant en outre l'étape de l'isolation des CTC à partir dudit échantillon.

4. Abiratérone pour utilisation dans une méthode de traitement d'un patient atteint d'un cancer de la prostate réfractaire aux hormones, dans laquelle la méthode comprend le procédé de prédiction d'une réponse à un traitement à l'abiratérone selon l'une quelconque des revendications 1 à 3.

5. Enzalutamide pour utilisation dans une méthode de traitement d'un patient atteint d'un cancer de la prostate réfractaire aux hormones, dans laquelle la méthode comprend le procédé de prédiction d'une réponse à un traitement à l'enzalutamide selon l'une quelconque des revendications 1 à 3.
